# EUROPEAN PATENT APPLICATION

(11) **EP 4 023 228 A1**
(43) Date of publication of application: **06.07.2022**
(21) Application number: 21210808.8
(22) Date of filing: 05.05.2017
(51) Int. Cl.: A61K 31/712, A61K 31/7125, C12N 15/10

(54) **GENOME EDITING OLIGONUCLEOTIDE WITHOUT PROGRAMMABLE NUCLEASES**

(30) Priority: 06.05.2016 US 201662333004 P; 20.10.2016 US 201662410487 P
(62) Divisional of application: 17793489.0
(71) Applicant: Woolf, Tod M., Sudbury, MA 01776 (US)
(72) Inventor: Woolf, Tod M., Sudbury, MA 01776 (US)
(74) Representative: Hertin und Partner Rechts- und Patentanwälte PartG mbB

(57) **Abstract**

The present invention includes compositions and methods for genome editing with in isolated cells or within an organism. The editing oligonucleotides contain an oligonucleotide strand which may contain a linker that positions an editing moiety in the proper location for modifying the targeted nucleobase and crisprRNA domain and an inactivated Cas 9 domain that cause deamination of the targeted nucleobase. The editing oligonucleotides may also contain at least one nucleotide sequence change from the targeted sequence in the genome. Certain embodiments of the method include modifying a genomic sequence within a cell utilizing an editing oligonucleotide without exogenous proteins to assist in the editing process. The editing oligonucleotide may comprise backbone modifications that increase the nuclease stability of the oligonucleotide as compared to unmodified oligonucleotides.

## Description

### CROSS-REFERENCE TO RELATED APPLICATIONS

The following application claims priority to U.S. provisional patent application serial no. 62/333,004 filed 6 May 2016 and U.S. provisional application serial no. 62/410,487 filed 20 October, 2016.

### STATEMENT REGARDING FEDERALLY SPONSORED RESEARCH OR DEVELOPMENT

Not applicable

### THE NAMES OF THE PARTIES TO A JOINT RESEARCH AGREEMENT

Not applicable

### INCORPORATION-BY-REFERENCE OF MATERIAL SUBMITTED ON A COMPACT DISC

Not applicable

### TECHNICAL FIELD

The present invention relates to the use of polynucleotides, including oligonucleotides or polypeptides, including proteins, that modify the sequence of a genome or RNA for applications in the areas of human and animal therapeutics (including *in vivo* and *ex vivo* therapeutic applications), cosmetic procedures, pre-clinical development, basic research, and for agriculture to improve food stocks, animal husbandry for modifying animal breeds (farm and other domesticated animals) to impart desirable features, and energy production.

### BACKGROUND OF THE INVENTION

Patients born with simple genetic mutations resulting in the loss of key functional proteins, such as metabolic enzymes, currently have few options for corrective treatment. For a handful of inborn errors of metabolism, exogenous protein delivery has been used successfully to provide replacement enzymes for treatment and requires lifetime treatment. Unfortunately, the number of disorders amenable to protein replacement is limited, as most genetic defects require the protein to be produced within a particular cell type in a patient, and cannot simply be treated by administration of the protein.

Gene replacement therapy ("gene therapy") has the potential to be a more generally useful method of functionally correcting genetic deficits. However, gene replacement therapy is a misnomer, as in most cases the cDNA is inserted into the cell (not the entire gene), and the defective gene is not replaced, but rather a wild-type cDNA is inserted into the cell extrachromosomally or in a different site than the endogenous gene.

Initial enthusiasm for the enormous potential of this technology has been tempered by several decades of clinical trials leading to a more realistic view of gene therapy as a pharmaceutical. What has emerged is the recognition that gene therapy has a number of limitations, including 1) the potential for adverse immune responses to viral vectors 2) the potential for integrating viral vectors to activate oncogenes, leading to cancer 3) epigenetic silencing of transgenes and 4) the potential of the expressed transgene to induce a cellular immune response. Advances in oligonucleotide chemistry and *in vivo* nucleic acid delivery technologies over the past decade have unlocked the potential for DNA and RNA modifying therapies. Positive data in numerous clinical trials and the approval of the first systemic antisense drug in the United States, Mipomersen (Ionis Pharmaceuticals, San Diego, CA) have further demonstrated the clinical utility of oligonucleotide drugs.

While the clinical benefits of using therapeutic oligonucleotides to inhibit protein expression by modulation of RNA levels have been demonstrated, the therapeutic potential of nucleic acid editing or repair approaches will likely exceed that of these inhibition approaches (Woolf, et al., PNAS 92:8298-8302, 1995, and Woolf, Nat. Biotech 16:341-344, 1998). A robust editing technology platform enables site-specific correction of mutated DNA, the creation of protective alleles or otherwise creating changes in the genome of whole organisms, cells or tissues that are desirable for research, therapeutic, cosmetic or agricultural purposes.

Such a platform will have broad utility as a therapeutic intervention and potential cure for a wide range of diseases caused by genetic point mutations, and other genetic lesions. Unlike gene therapy, genome editing has the potential to repair the actual lesion(s), leaving the edited chromosome with a wild-type sequence, without vector sequences, integrations at other sites, or random insertions or deletions. This "footprint-free" approach is highly desirable for therapeutic indications, as it precludes potential side effects due to unnatural sequences. Also, if the genome editing therapeutic inadvertently edited the germline of a patient, precise "footprint-free" editing to wild-type would not create unnatural sequences within progeny.

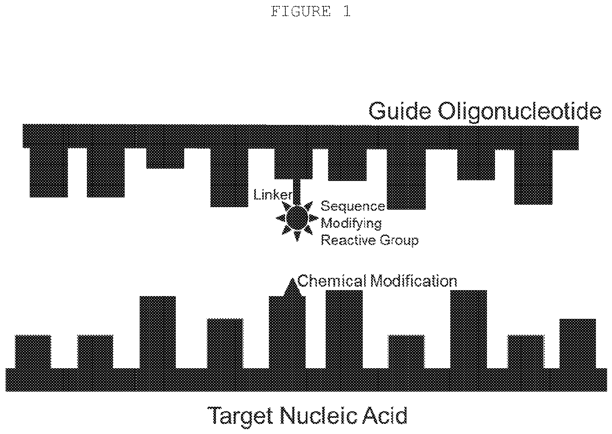

There are two general mechanisms of sequence editing with nucleic acids. These are chemical modification and incorporation of nucleic acid sequences into the target. With the chemical modification mechanism, the editing oligonucleotide causes a chemical modification of the targeted nucleobase, such that the coding of the targeted nucleobase is changed. The second general mechanism is by incorporation of one or more oligonucleotides into the target RNA or DNA sequence. In this mechanism, the oligonucleotide is often referred to as "donor" DNA. This mechanism is loosely referred to as homologous recombination (HR) or homology directed repair, but can also include mechanisms such as gene conversion, induction of mismatch repair (See Figure 1 in (PCT/US2015/65348) and trans-splicing or strand-invasion followed by priming of nucleic acid synthesis.

The explosion of information on genetic and molecular pathways, driven by Next Generation Sequencing and SNP analysis, has provided a vast array of targets for therapeutic editing to treat monogenic and polygenic diseases (see PCT/US2015/65348). The therapeutic potential of DNA editing repair has been demonstrated by promising data. Engineered zinc finger nucleases (Sangamo

Biosciences, Inc., Richmond, CA) have been used to treat HIV and mRNA has been repaired with exon skipping antisense morpholinos (Sarepta Therapeutics, Inc., Cambridge, MA) have been used to treat muscular dystrophy. CRISPR/Cas-9 and other gene editing approaches employing programmable nucleases to enhance editing efficiency have spawned a number of research products and major investments in therapeutic applications.

Therapeutic mRNA editing was first demonstrated in a vertebrate model system by Woolf et al. (PNAS 92:8298-8302, 1995). In this system, a targeted stop codon mutation in a Duchenne Muscular Dystrophy mRNA was modified by duplex formation with an editing antisense RNA that induced chemical modification of the targeted nucleobase by enzymes at the target site. The enzyme was an endogenous adenosine deaminase that modified the targeted adenosine to an inosine which is translated primarily as guanine. The work by Woolf *et al.* induced editing that was limited in specificity.

Montiel et al., (PNAS 110(45):18285-90, 2013) demonstrated a related mechanism of mRNA repair for Cystic Fibrosis wherein a 20% correction was achieved in mammalian cells. While this successfully demonstrated the principle of therapeutic editing, Montiel's methods are complicated to use clinically. The primary reason for this is that the method of Montiel *et al.* requires the introduction of a modified gene, mRNA or proteins into cells by gene therapy, mRNA therapy or other methods. Because of this all of the known disadvantages recognized with gene therapy and mRNA therapy are also relevant to Montiel's method of therapeutic editing.

In another approach, Singer, et al. (Nucleic Acids Research, 27(24):38-45, 1999) targeted DNA with an alkylating oligomer that hybridized to the target strand assisted by RecA protein. However, cross-linking of the invading oligonucleotide to the targeted DNA typically results in a variety of mutations distributed over a region of DNA and can result in inhibition of replication. Conjugation of reactive base modifying chemistries to oligonucleotides and sequence specific modification of targeted dsDNA sequences has been achieved (Nagatsugi, et al. Nucleic Acids Research, Vol. 31(6):e31 DOI: 10.1093/nar/gng031, 2003). This study demonstrated site-specific mutation of the targeted sequence with some specificity for the targeted base and a significant albeit low efficiency (0.3% with one treatment). However, this method has the same disadvantages as Singer, *et al.* because it results in cross-linking.

Sasaki et al. (J. Am. Chem. Soc., 126(29):8864-8865, 2004; see also U.S. patent no. 7,495,095) developed a method for delivery of nitric oxide (NO) to a specific cytosine site of DNA sequence followed by specific deamination of the cytosine base. This technique required non-physiological pH to allow the reaction to occur, and long incubation times, that would not necessarily be applicable to therapeutic intervention. In addition, the chemically reactive oligonucleotide strategies, even if made efficient in cells, require complex chemical synthesis, and may be reactive with non-targeted cellular components, including DNA, which is not ideal. They also require different targeted chemistries for each base change, and are mostly suitable for transitions, not transversions, which limits their general utility.

Further, this method does not repair deletions and insertions, which is a further limitation to its general application to correcting any mutation. Nevertheless, this chemical modification approach to editing has the advantage that
it does not require the addition of exogenous proteins to the cell in order to facilitate editing, and it can in principle be used with highly modified oligonucleotide backbones that can allow for nuclease resistance greater than an oligonucleotide that has one or more unmodified DNA linkages, better tissue distribution and cellular uptake.

Editing with single-stranded editing oligonucleotides led to consistent reproducible editing, but with relatively low efficiencies (∼0.1-1%). The most active single-stranded editing oligonucleotides had unmodified DNA internal regions, which resulted in rapid nuclease degradation in cells and likely resulted in Toll-like receptor activation. Editing efficiency was increased by the following approaches:
1. adding three phosphorothioate residues to each end of the editing oligonucleotides (However, the resulting editing oligonucleotides where still susceptible to rapid endonuclease digestion within the cell and the phosphorothioates increase their toxicity);
2. synchronizing the cell cycle such that the cells are treated with the editing oligonucleotides during the S-Phase. Unfortunately while this increased editing efficiency to some degree, the approach is cumbersome and not always practical for *in vivo* therapeutics;
3. treating the cell with reagents that slow the progression of the replication forks and/or induce DNA strand-cleavage in the cell, which results in increased DNA repair in the cell (However while this increased editing efficiency to some degree, the approach is also cumbersome and not always practical for *in vivo* therapeutics; and
4. adding PNA clamps or strand invading single-stranded PNAs that bind in the vicinity of the targeted edit (Bahal et al. Current Gene Therapy 14(5):331-42, 2014, Chin et al. PNAS 105(36):13514-13519, 2008, Rogers et al. PNAS 99(26):16695-16700, 2002, U.S. Patent 8,309,356.

These improvements increased editing efficiency to up to approximately ∼8% per treatment in model *in vitro* cellular systems, but each approach had limitations as cited above (Kmiec, Surgical Oncology 24:95-99, 2015).

Programmable nucleases, such as zinc finger nucleases, TALENs and endonucleases based on I-CreI homing endonuclease (such as ARCUS^{™} by Precision BioSciences, Durham, NC) have been used to enhance incorporation of donor DNA sequences into the chromosome by cutting the chromosome in the vicinity of the editing target site.

Targeted cleavage by the CRISPR-Cas9 system has also been used in recent years to enhance the efficiency of editing genomes. However, the programmable nucleases often times cause off-target modifications and require potentially dangerous and undesirable single and double-stranded breaks in the chromosome. One particularly undesirable consequence of using programmable nucleases is the generation of random insertions and deletions (indels) at the cleavage site(s) . The desired precise edit by a donor DNA competes with indel creation, leaving a mixture of
precisely edited chromosomes and chromosomes with a variety of indels. This system also strictly requires that a foreign engineered protein be expressed or delivered in functional form to cells. The engineered protein, Cas9, is immunogenic and therefore less desirable for therapeutic applications. In addition, expression or delivery of a protein to a cell is a substantial challenge for clinical development. In order to make a specific change of one sequence to another defined sequence, the CRISPR- Cas9 system requires, in addition to Cas9, a gRNA exceeding 70 nucleotides and one or two additional oligonucleotides for insertion in the genome. Thus, the CRISPR/Cas9 system of editing is highly complex, and this complexity creates a challenge for clinical development.

Consequently, there is a need in the biomedical and biotechnology industry for nucleic acid editing compounds that work more efficiently and do not strictly require: cross-linking the editing agent to the nucleobase of the targeted nucleic acid as a method of action; or the introduction of indel inducing breaks in the target nucleic acid by exogenous programmable nucleases to obtain editing. In addition, it is desirable that these editing agents are able to repair point mutations and in some cases insertions and deletions, in the case of editing oligonucleotides contain chemical modifications that enhance the pharmacokinetics and have bio-distribution and intra-cellular nuclease stability without substantially reducing the editing activity, optionally reduce the activation of Toll-like receptors, and correct the underlying genetic causes of disease by editing a targeted DNA sequence and in some embodiments RNA sequence.

### SUMMARY OF THE INVENTION

One aspect of this invention is a method of utilizing a single-stranded oligonucleotide complementary to one of the DNA strands of a genome or an RNA for sequence editing (Woolf,T.M. et al. Nature Reviews Drug Discovery 16, 296 (2017*)*). The method comprises the steps of introducing into a cell or an organism a single-stranded oligonucleotide without strictly requiring exogenous proteins to assist in editing said target sequence. In certain embodiments, the oligonucleotide is substantially complementary to the target sequence, with the exception of one or more mismatches, including inserts or deletions, relative to the target sequence. Such an oligonucleotide may be referred to herein as an oligonucleotide, an oligonucleotide of the invention, or as an editing oligonucleotide.

In certain embodiments, the target recognition domain is an editing oligonucleotide that binds to the target sequence and is substantially complementary to the target sequence, and may comprise one or more chemical modifications.

In some embodiments, the target sequence recognition domain (see Table I for examples) is non-covalently bound to, or activates a nucleobase modifying activity that reacts with, or promotes a reaction with, a nucleotide on the target sequence (e.g. Figure 1). The nucleobase modifying activity can be reactive chemicals, a catalyst or an enzyme. Examples of such reactions include alkylation, acetylation, cross-linking, amination or de-amination.

These editing oligonucleotides may comprise structures wherein each oligonucleotide is substantially complementary to said target nucleic acid and is about 10 to about 50 or 10 to about 200 nucleotides and wherein at least one of said oligonucleotides may comprise crisprRNA and a Cas 9 having inactive nuclease domains linked to a base having modifying activity that is positioned in the proximity of the targeted nucleobase, wherein said base modifying activity causes the deamination of the targeted nucleobase. The targeted nucleic acid may be RNA or DNA. When the target is RNA it is preferably mRNA.

An oligonucleotide may also be referred to herein as an oligonucleotide, an oligonucleotide of the invention, or as an editing oligonucleotide. The oligonucleotide can preferably have one or more chemical modifications. This/these chemical modification(s) modification(s) may include one or more backbone modification(s), sugar modification(s) nucleobase modification(s), linkers and/or conjugates.

The oligonucleotide is complementary to a target sequence in the genome and may have mismatches to the target sequence, as described below. Modifications may increase the efficiency of editing by increasing the nuclease stability as compared to unmodified oligonucleotides or compared to oligonucleotides having three phosphorothioates on each terminus.

In one embodiment the editing oligonucleotide sequence is the sequence desired after the editing is completed. The desired edit may be a transition or transversion, or a deletion or insertion. Without wishing to be bound by a particular theory or mechanism, the editing oligonucleotide binds to the partially or fully complementary target genomic DNA sequence when the target sequence is separated from the opposite genomic strand during cellular processes such as transcription or replication. In some cases, the hybridization of the editing oligonucleotide to a double-stranded genomic DNA target can occur during "breathing" or transient melting of the target DNA. In some embodiments the invasion of the editing oligonucleotide into the duplex genome DNA is optionally promoted by a protein or proteins, such as Cas-9 (or Cas-9 homologs) or RecA and single-stranded DNA binding protein,
or other proteins that enhance strand invasion, such as those listed in Table VIII.

In one embodiment, once the heteroduplex is formed between the editing oligonucleotide and target genomic DNA strand, the area of non-perfect complementarity is corrected by cellular DNA repair. When the editing oligonucleotide is used as the "correct" template for repair, the desired edit will be incorporated into the targeted genomic DNA strand or RNA strand. In a second mechanism that can also occur in the cell, the editing oligonucleotide is incorporated into the target nucleic acid such as into DNA by Homologous Recombination (HR), or other processes that result in the editing oligonucleotide sequence being incorporated into the target DNA or RNA.

In one embodiment, each editing oligonucleotide comprises at least one of the internucleotide linkages or sugar modifications listed in Tables II and IV, respectively. Proteins or catalytic nucleic acids may be combined with editing oligonucleotides to enhance editing efficiency (Table VIII).

Other embodiments include, a pharmaceutical composition comprising a pharmaceutical carrier or delivery vehicle and one or more of the editing oligonucleotides wherein the carrier may be water, saline or physiological buffered saline and a cell containing one or more of the editing oligonucleotides.

Another aspect of the present invention is a method of improving the health of an individual requiring treatment for a medical condition or reducing or eliminating or preventing a medical condition in an individual requiring treatment for the condition comprising administering a composition containing at least one editing oligonucleotide to the individual. Some administration methods and target indications are listed in (PCT/US2015/65348).

Other aspects of the present invention include methods of administering at least one editing oligonucleotide to an individual suspected of having a condition that may be treated by such administration, wherein that condition may be reduced, prevented or eliminated by reverting a mutated nucleotide in a target nucleic acid to the wild-type nucleotide; modifying a non-mutated nucleotide of a mutated codon in a target nucleic acid to produce a wild-type codon; converting a pre-mature stop codon in a target nucleic acid to a read through non-wild type codon; or modifying a mutated codon in a target nucleic acid to produce a non-wild type codon that results in a non-disease causing amino acid, also editing which inserts or deletes a number of nucleotides (*i*.*e*., in some cases, less than about 10, less than about 5 or less than 3) (see (PCT/US2015/65348)).

Another aspect of the present invention is a method for modifying the nucleic acid coding for a protein or a functional RNA or regulating the transcription levels of a gene to modulate said protein's or RNA's activity or modifying a mutant protein to suppress its disease causing affects comprising the steps of administering to a cell or to an individual at least one editing oligonucleotide or protein. In these methods the target nucleic acid for editing is DNA.

An editing oligonucleotide or proteins of the present invention may perform one or more of the following functions, which include: exact reversion of a mutated base to a base with the coding specificity of the wild-type DNA or RNA sequence; change a mutated codon to encode a non-wild-type codon that results in a non-disease causing amino acid; modification of a stop codon, to a read through codon of a non-wild-type, codon that still allows for the activity or partial activity of the targeted protein; change a non-mutated base of a mutated codon, that results in the wild-type codon or non-disease amino acid codon; change the nucleic acid sequence of a protein, to increase or decrease (or eliminate) the activity of a domain of that protein; change a sequence of RNA or DNA, to produce an allele that is known to be protective of a disease; change a site in the targeted mutant protein, other than the mutated or disease variant codon, that suppresses the disease causing effects of the mutated gene; change a site in a gene or RNA other than the mutated or disease variant, that suppresses the disease causing effects of the mutated gene (2^{nd} site suppressors); change a promotor, enhancer or silencing region of a gene, that modulates the expression of the disease associated gene such that the diseased state is reduced (up or down regulation or modulation of the response of the gene's expression to changes in the environment) ; methylation of sugar in DNA, to change the epigenetic state of the targeted sequence and/or change a splice-site sequence at the DNA or RNA level to obtain a splicing pattern that treats the disease state.

Self-delivering oligonucleotides refer to chemistries that efficiently enter the interior of the cell without delivery vehicles, such as Gal-NAC conjugated oligonucleotide, lipophilic group conjugated editing oligonucleotide (U.S. Patent Application 20120065243 A1), or oligonucleotides with phosphorothioate tails or otherwise having about 8 or more phosphorothioate linkages (U.S. Patent Application 20120065243 A1).

### BRIEF DESCRIPTION OF THE DRAWINGS

Figure 1: The components of an embodiment of an editing oligonucleotide that acts by chemically modifying the targeted nucleobase (large rectangles represent pyrimidines and the smaller rectangles represent purines).

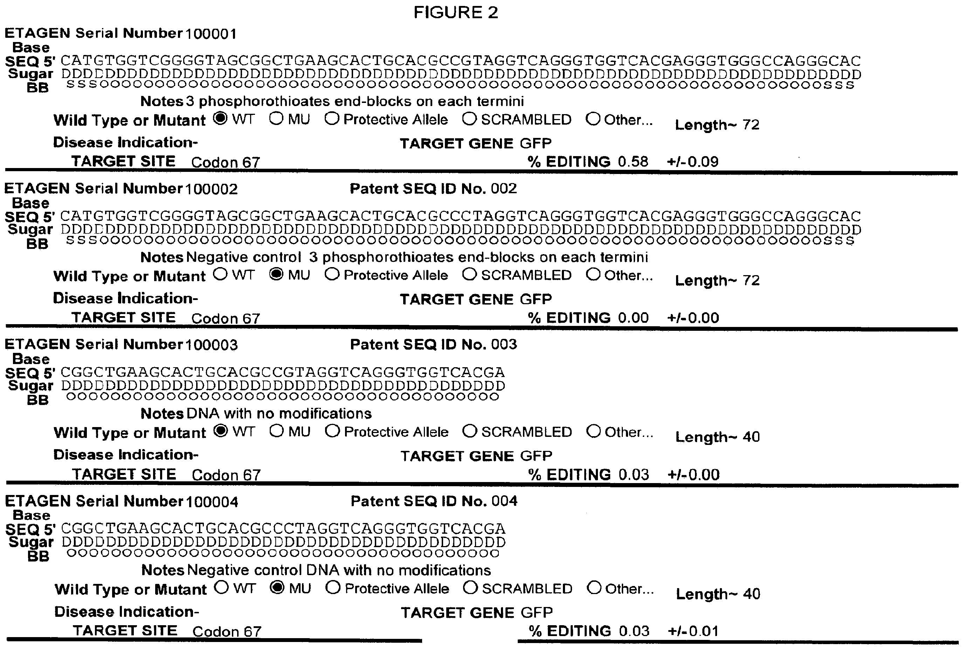

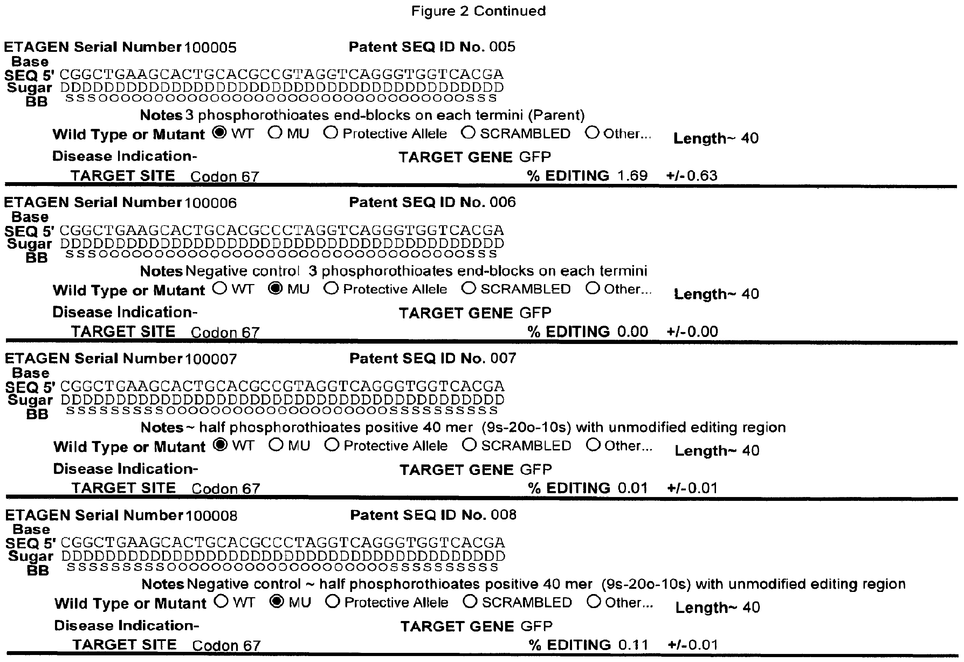

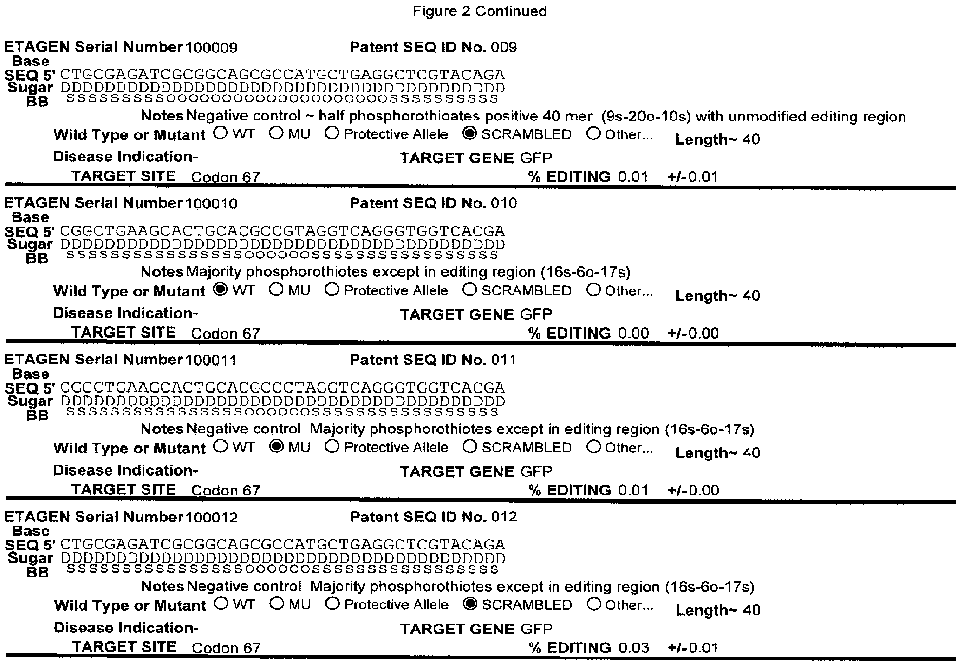

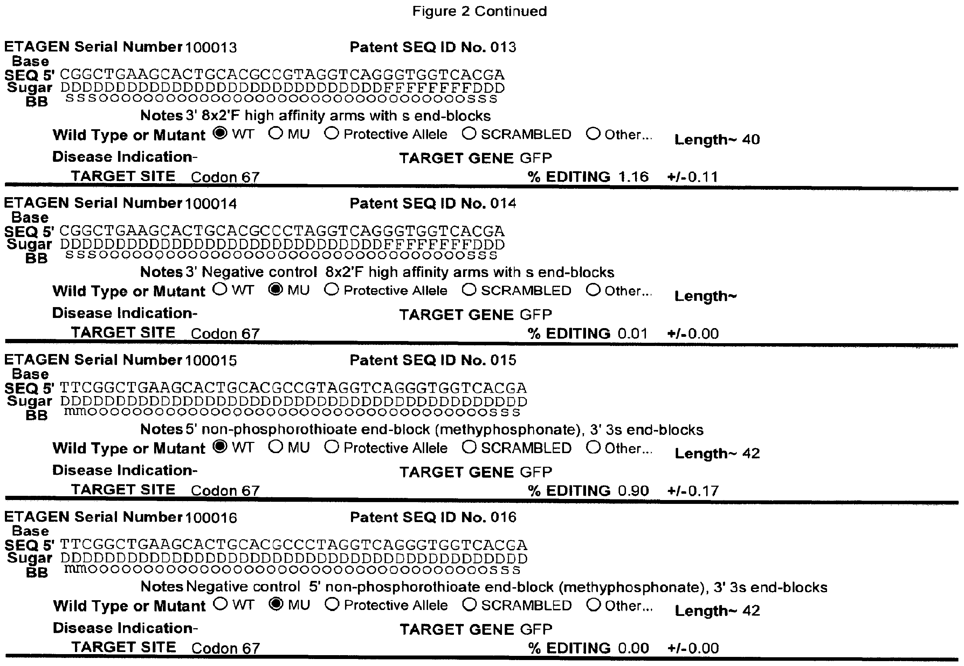

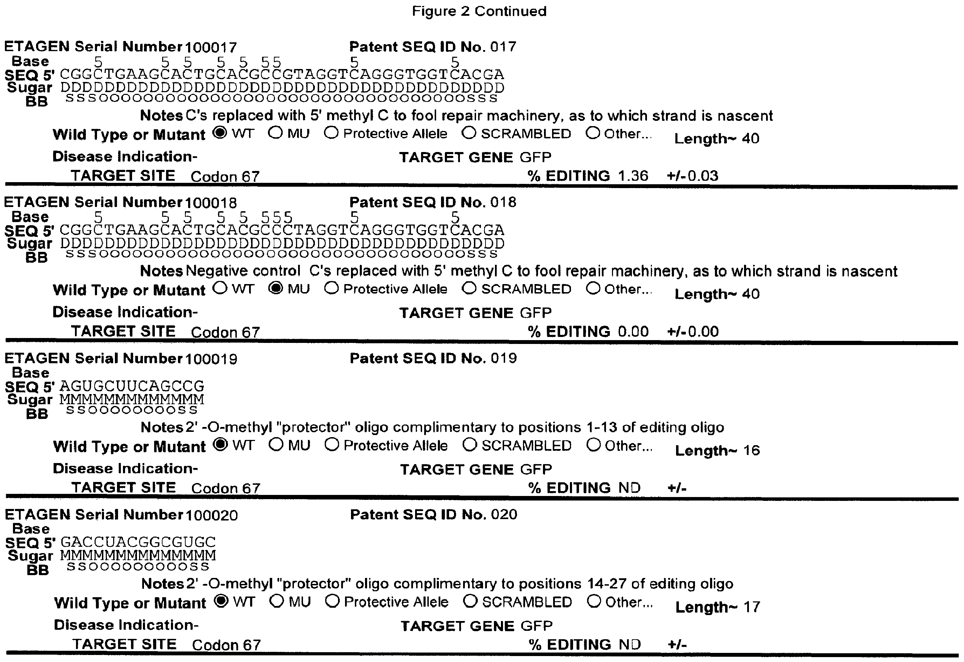

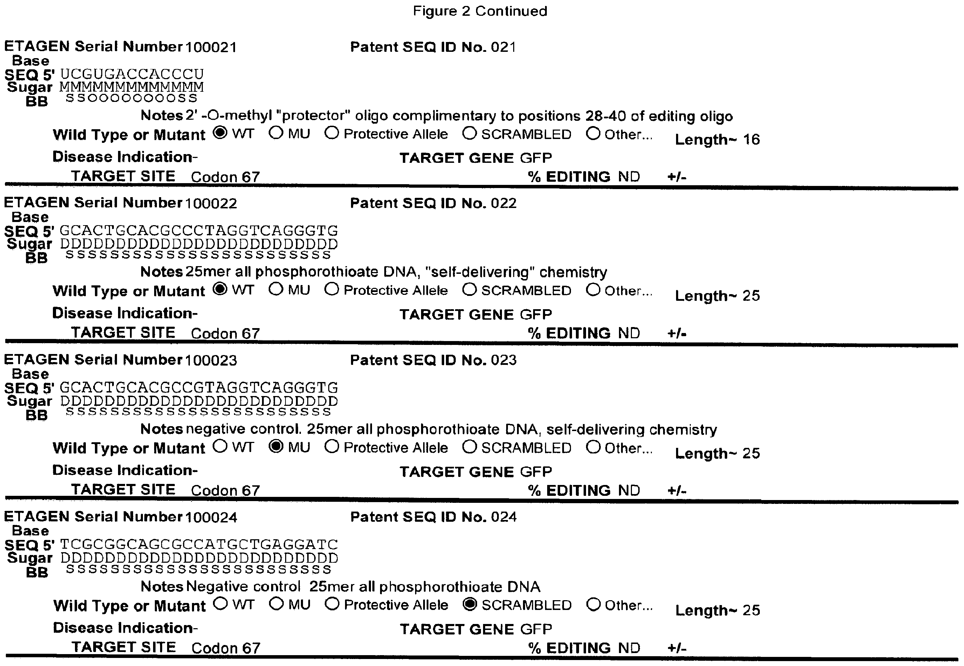

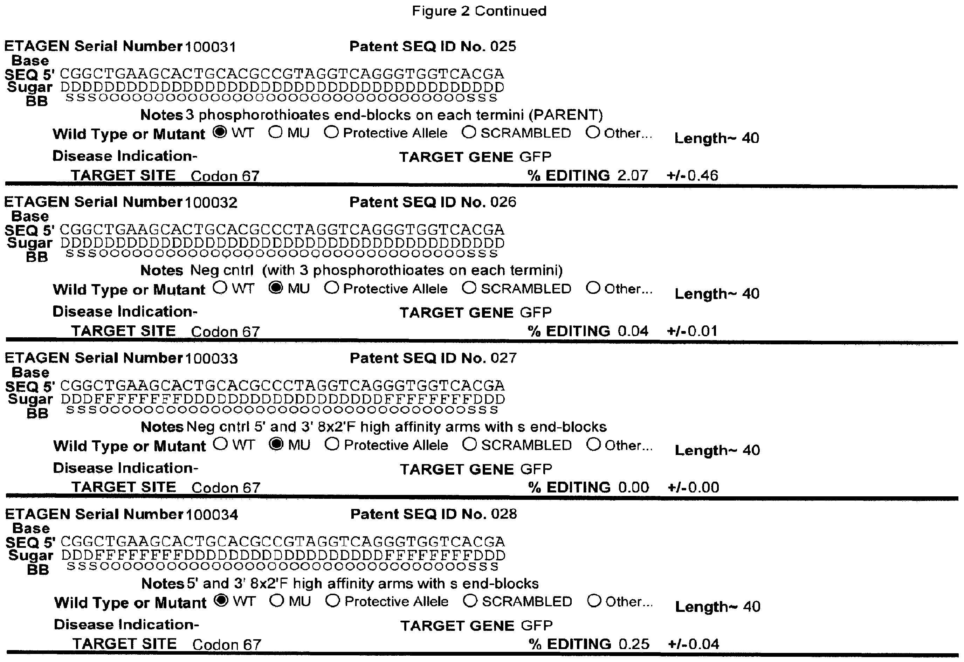

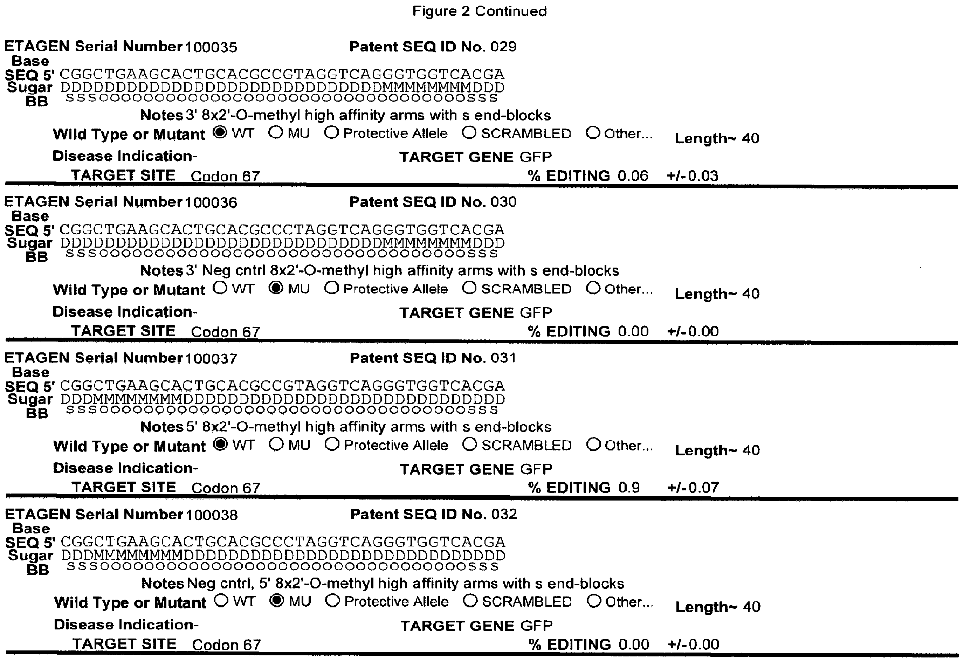

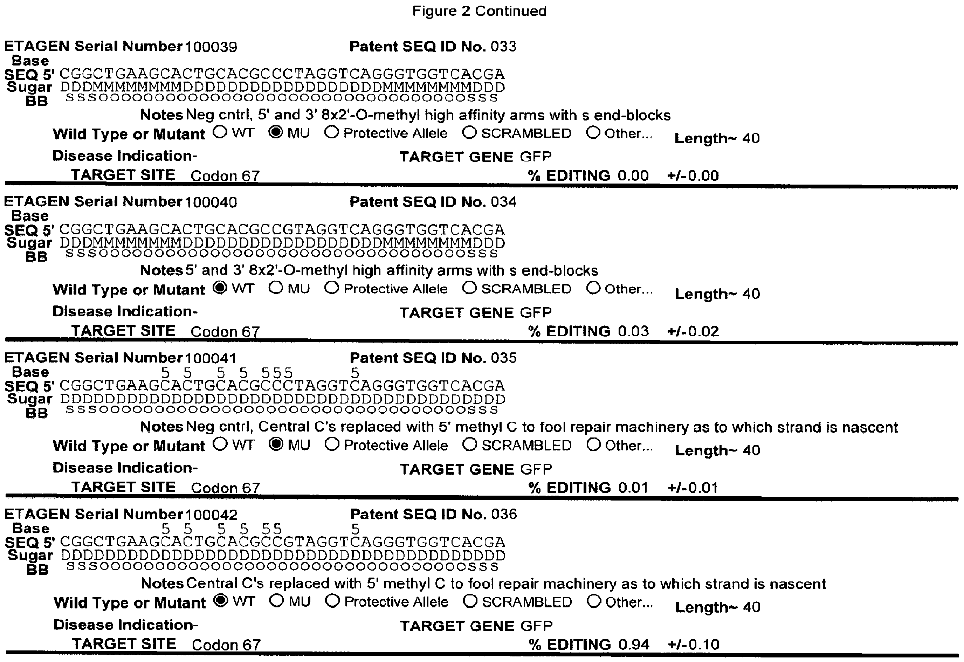

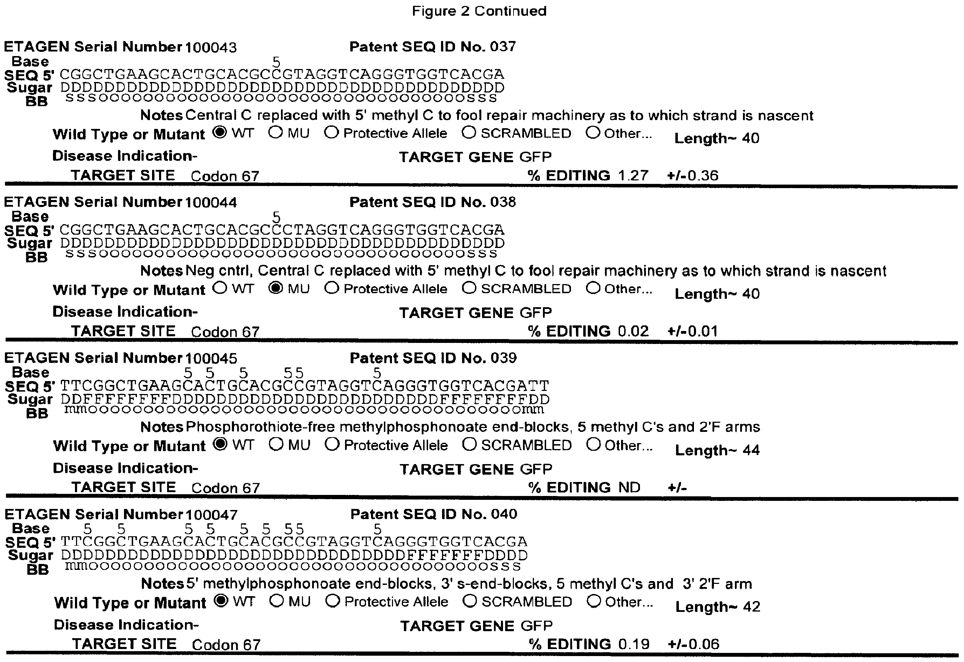

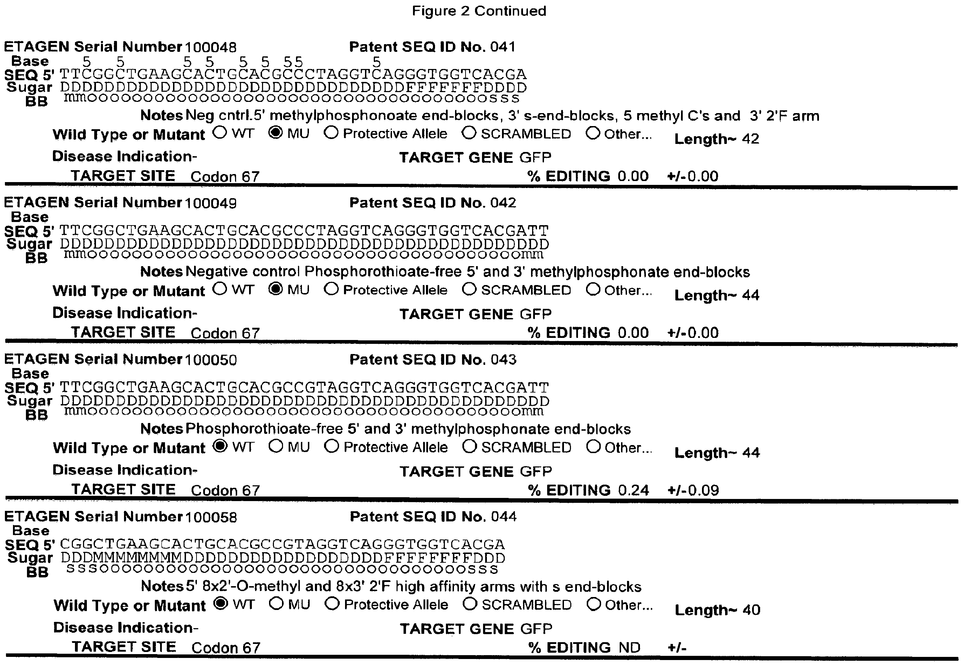

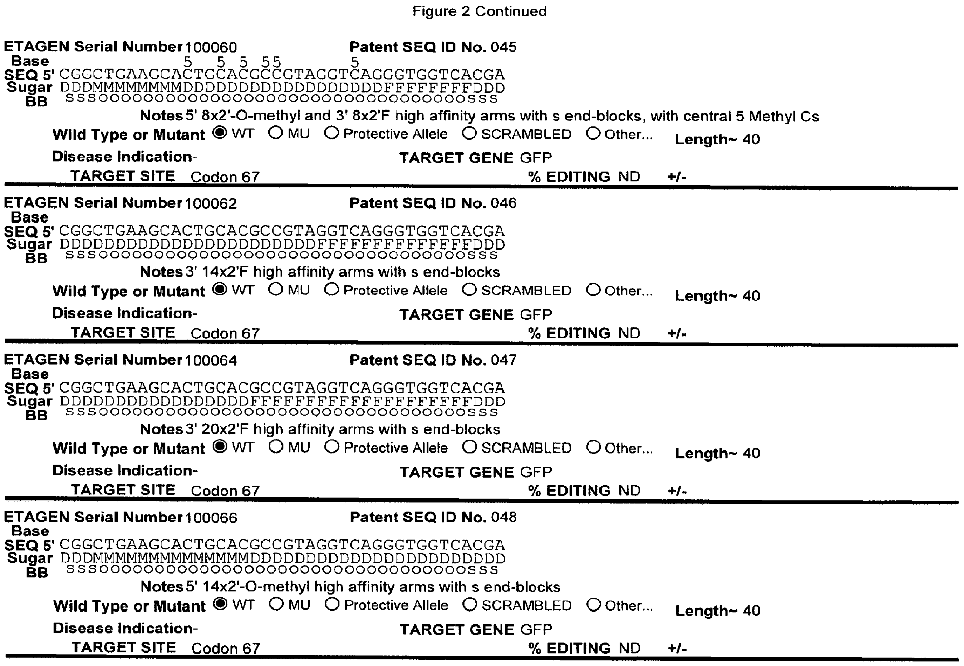

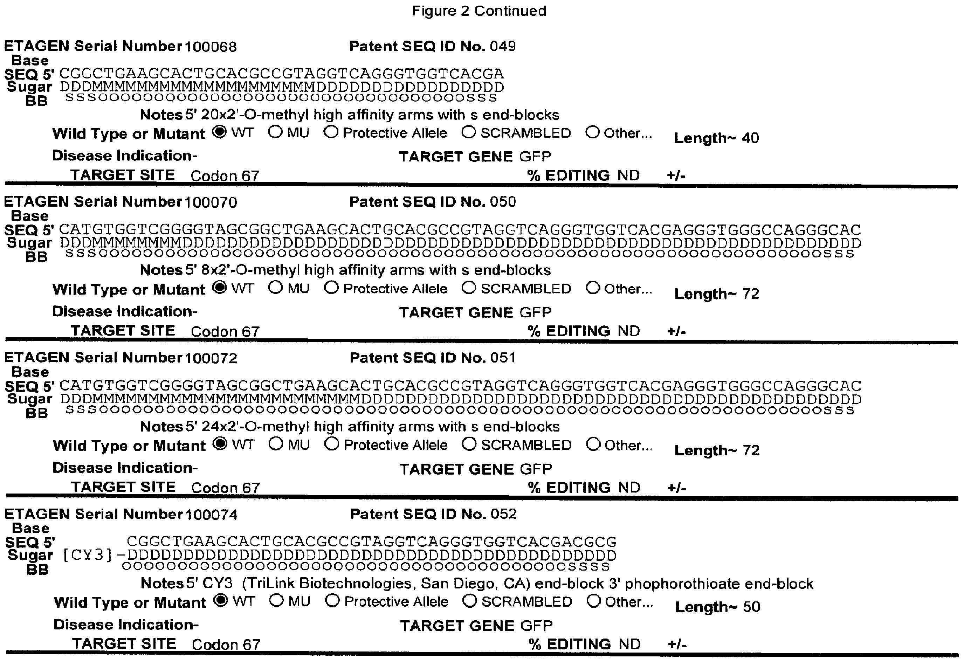

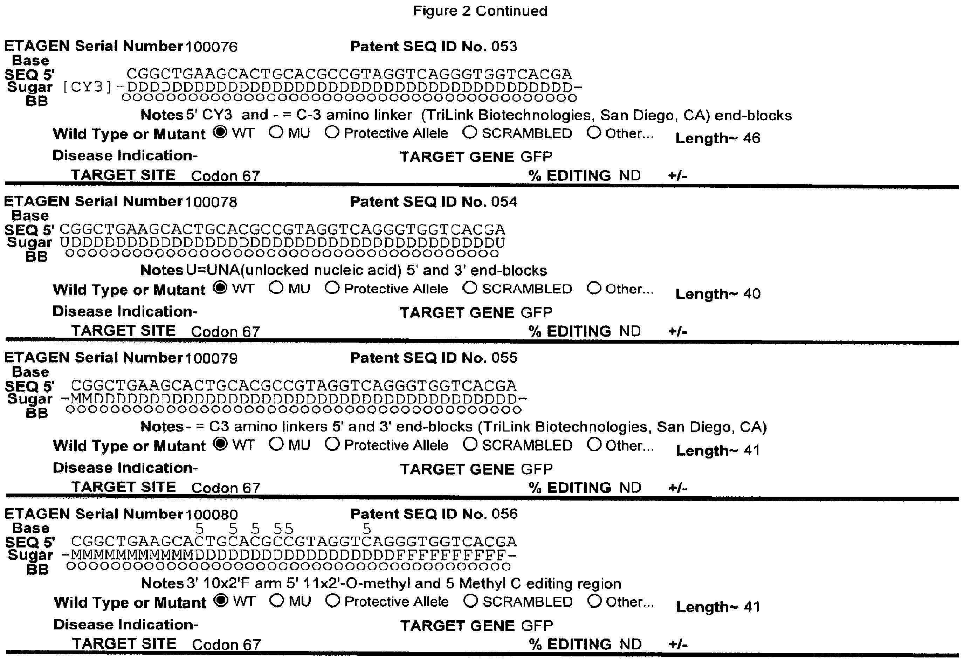

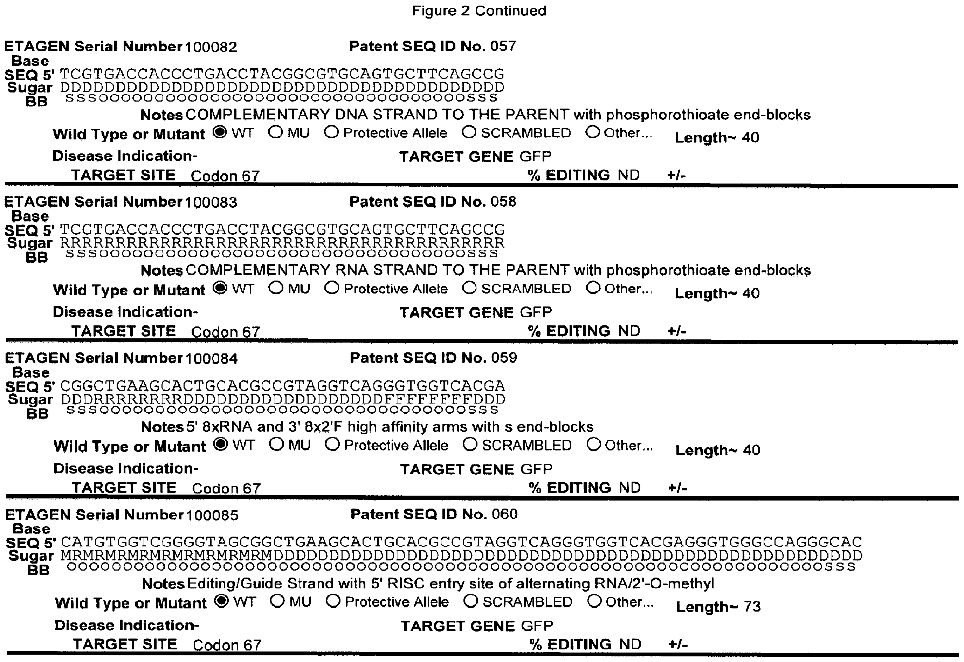

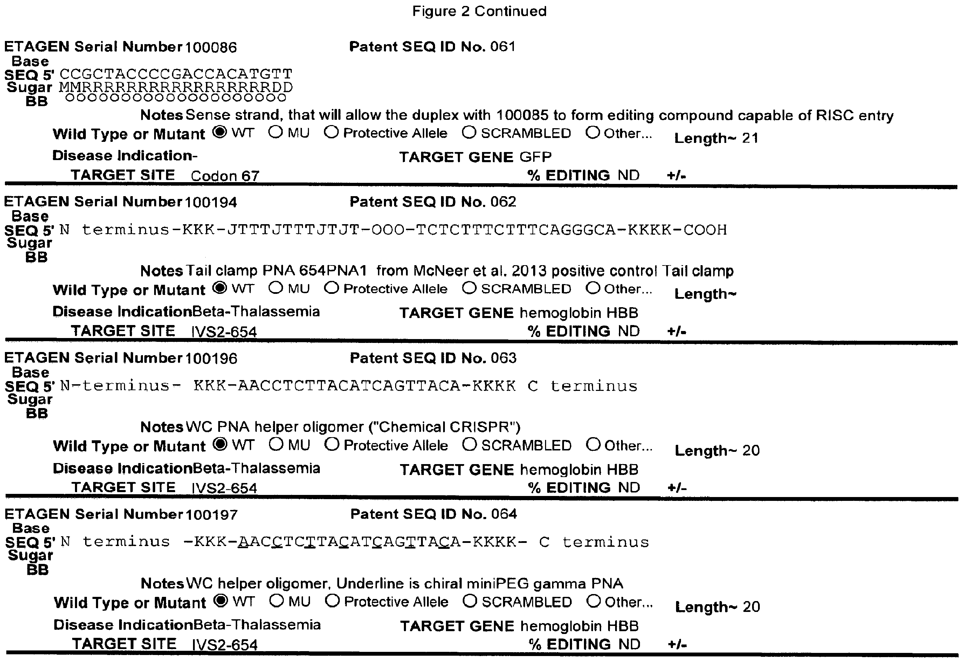

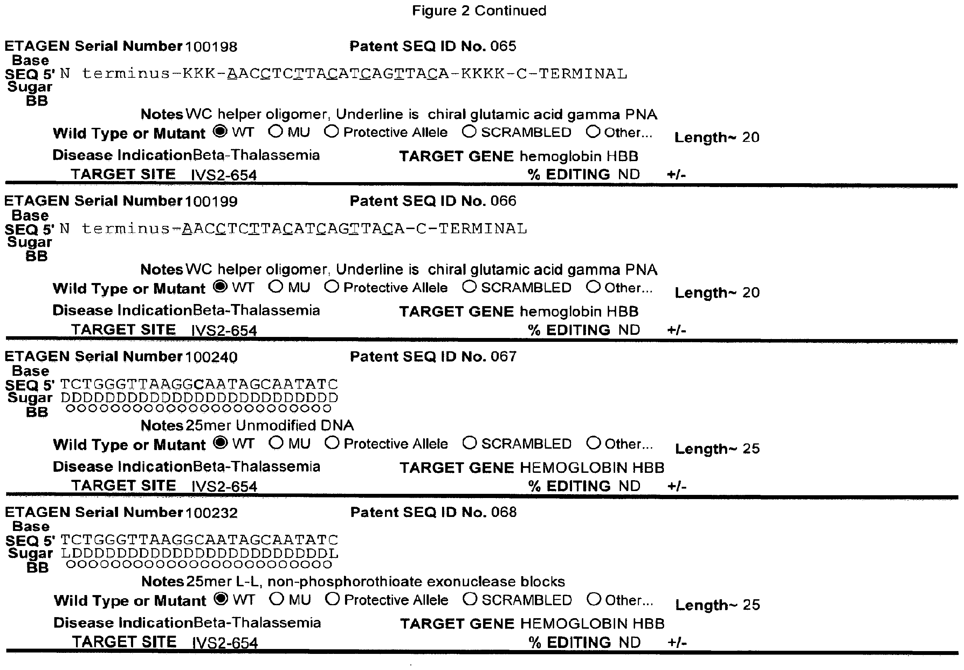

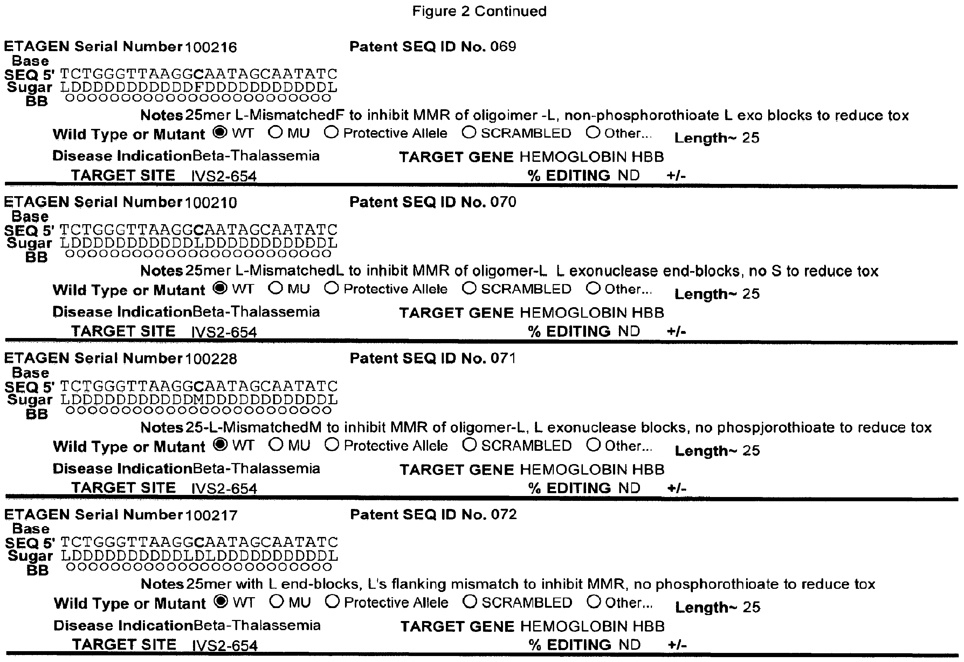

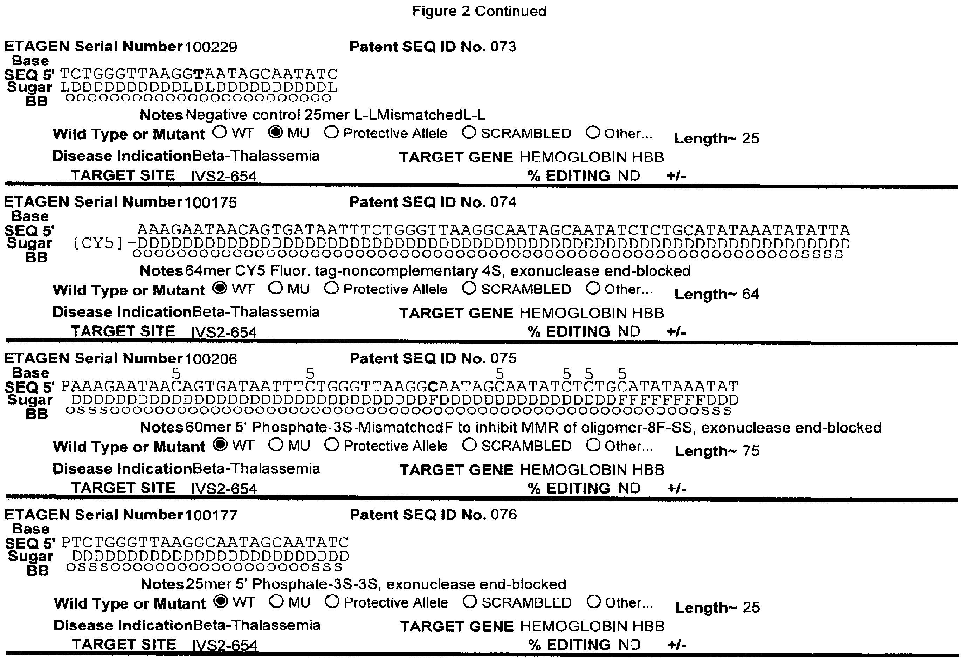

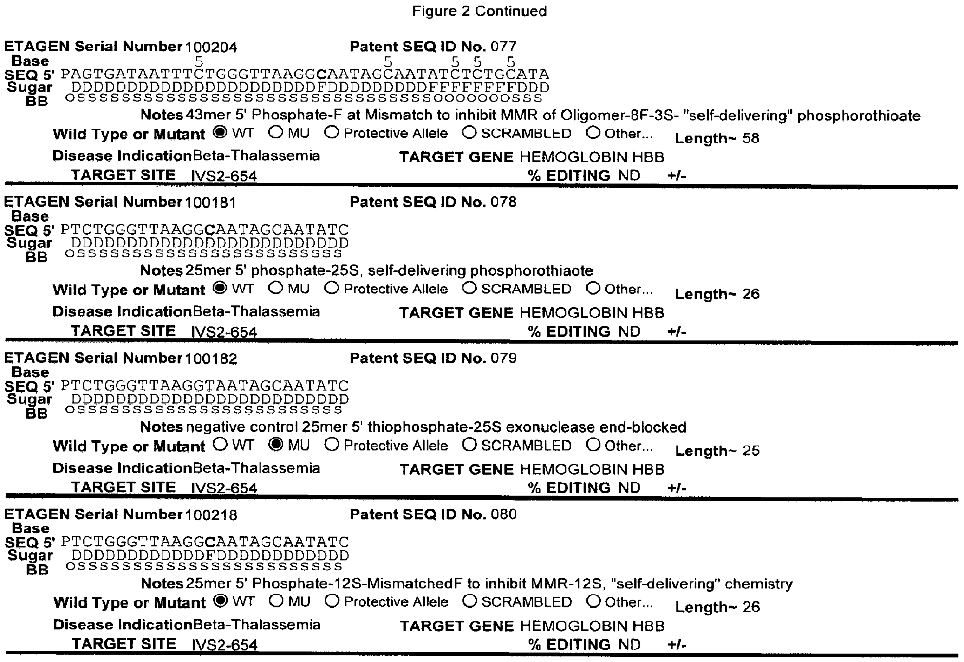

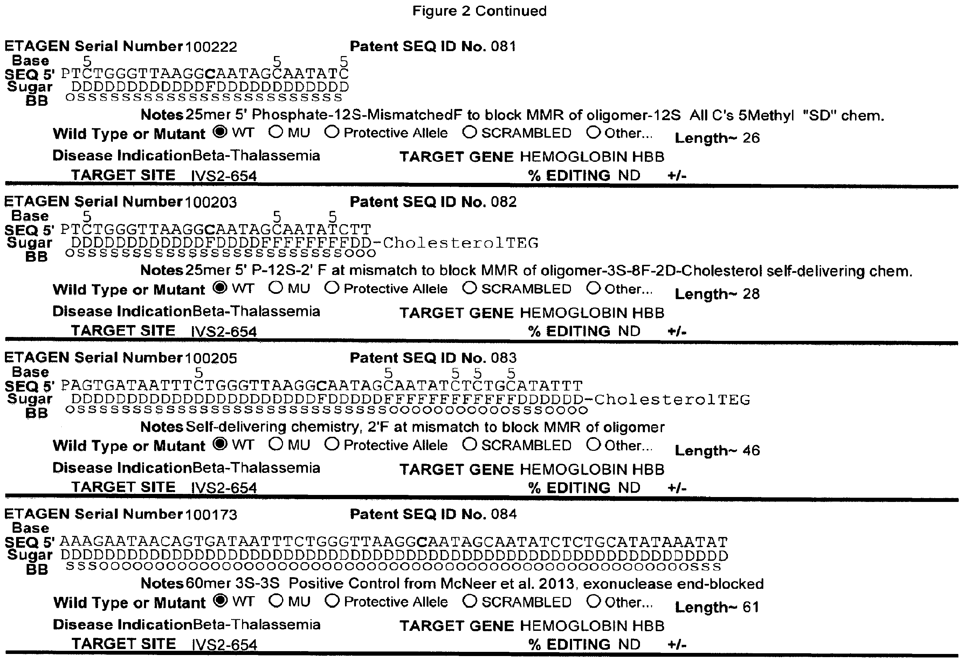

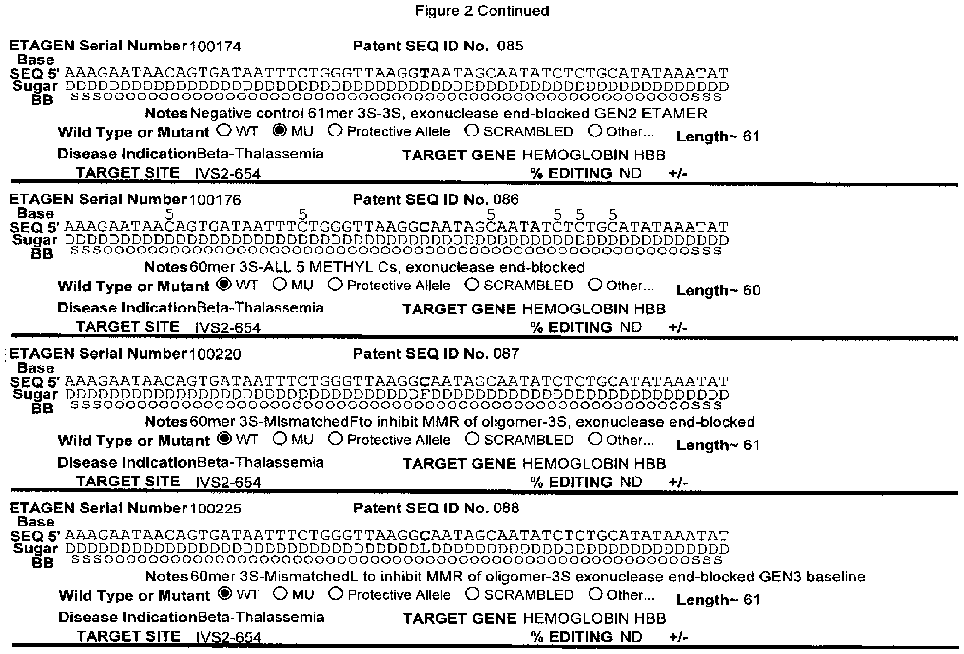

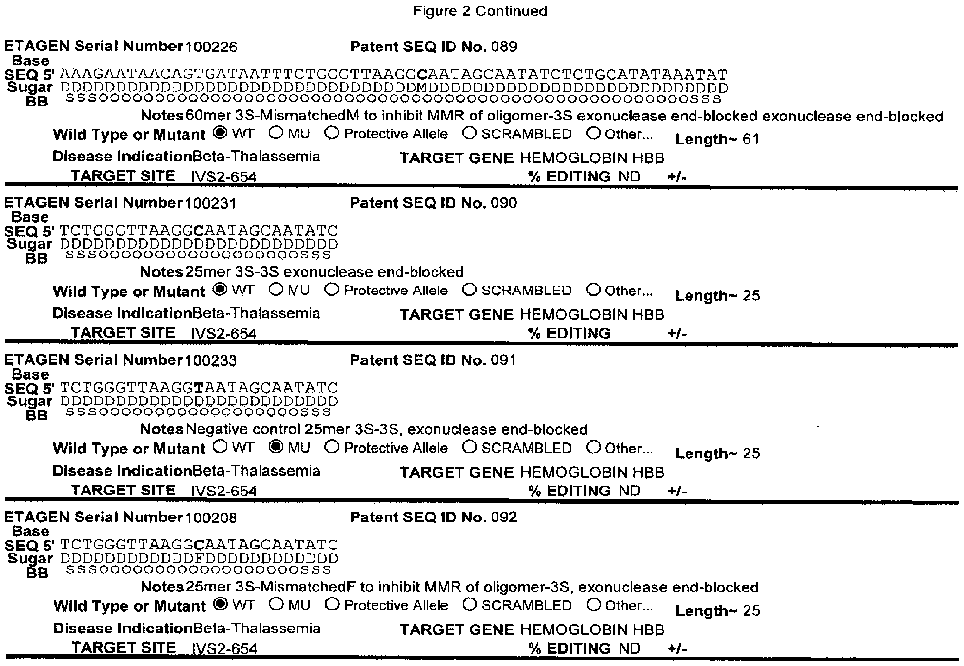

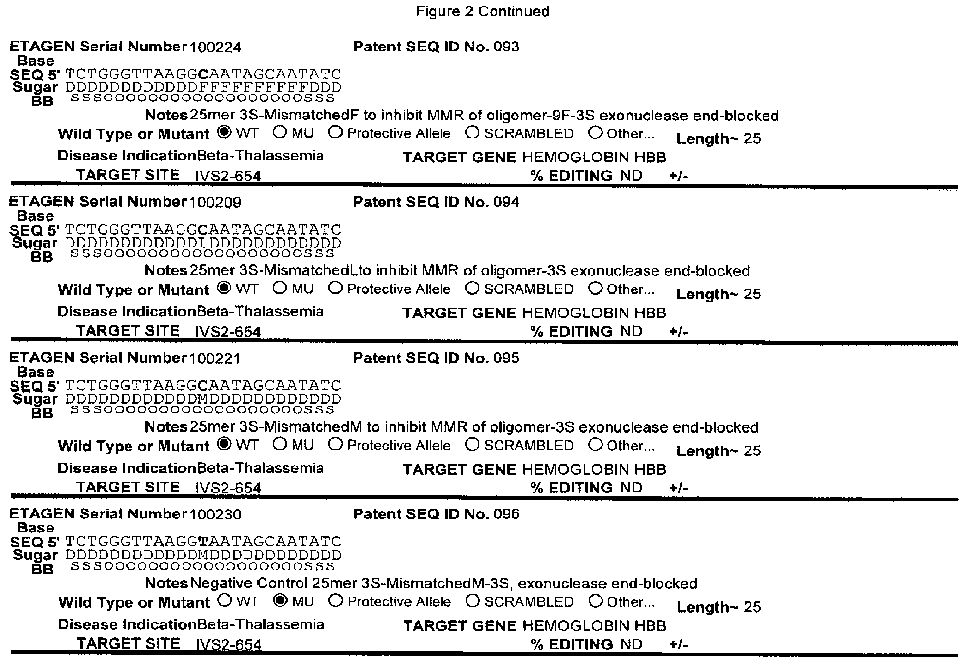

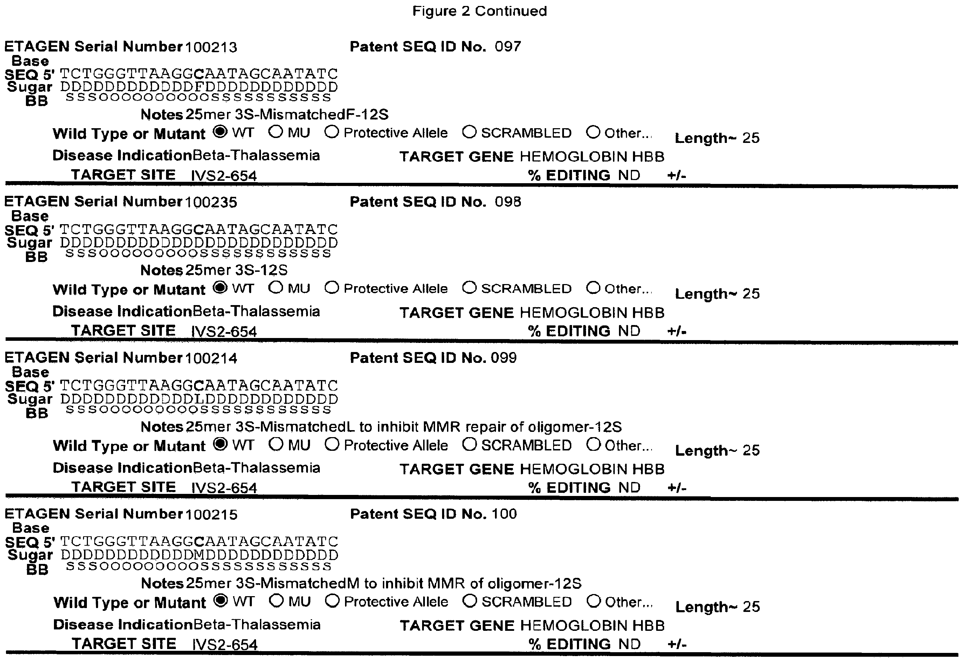

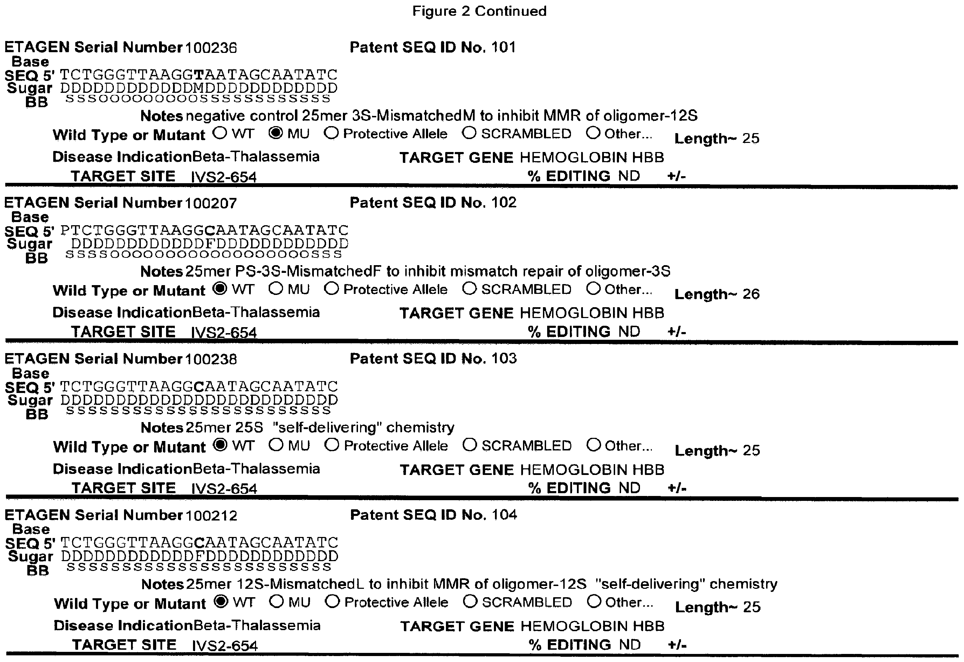

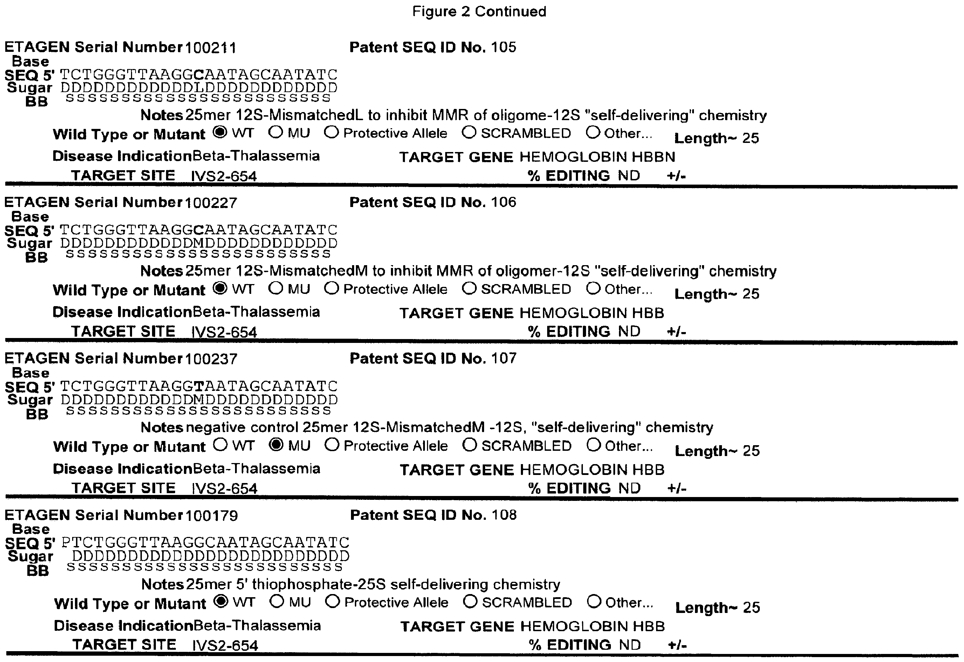

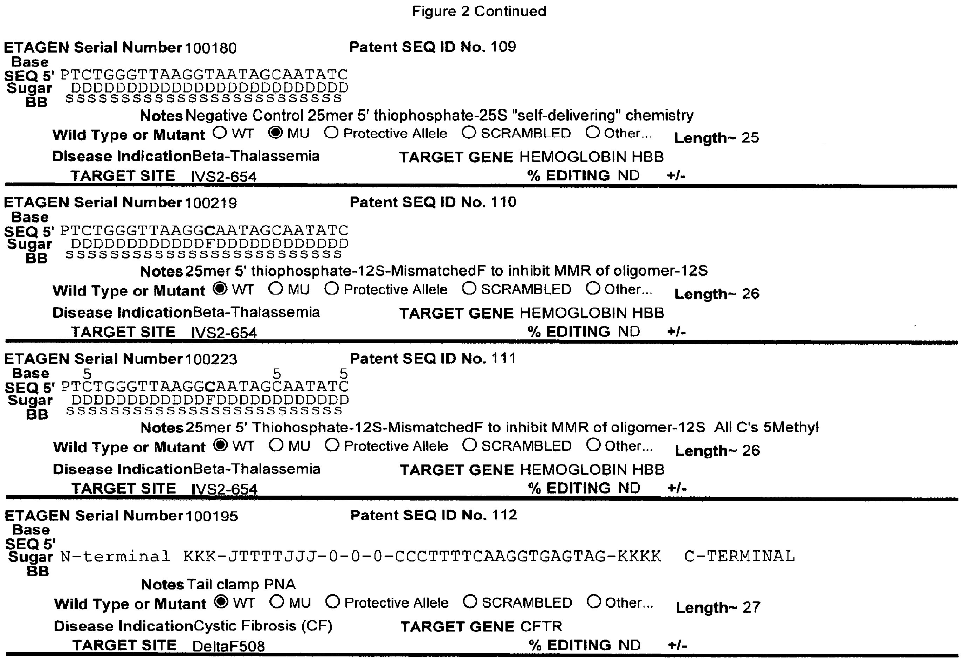

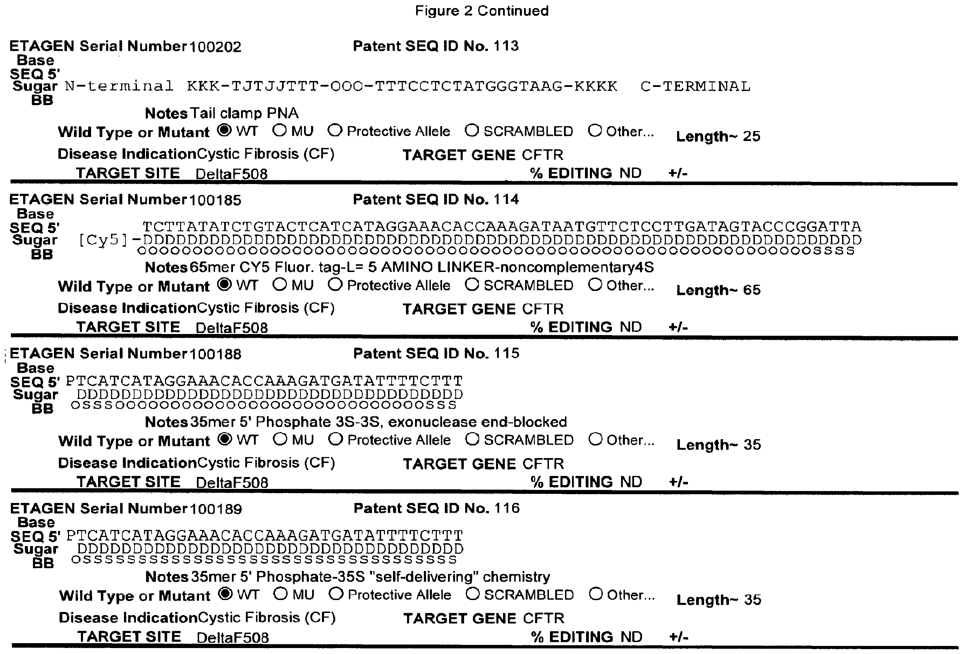

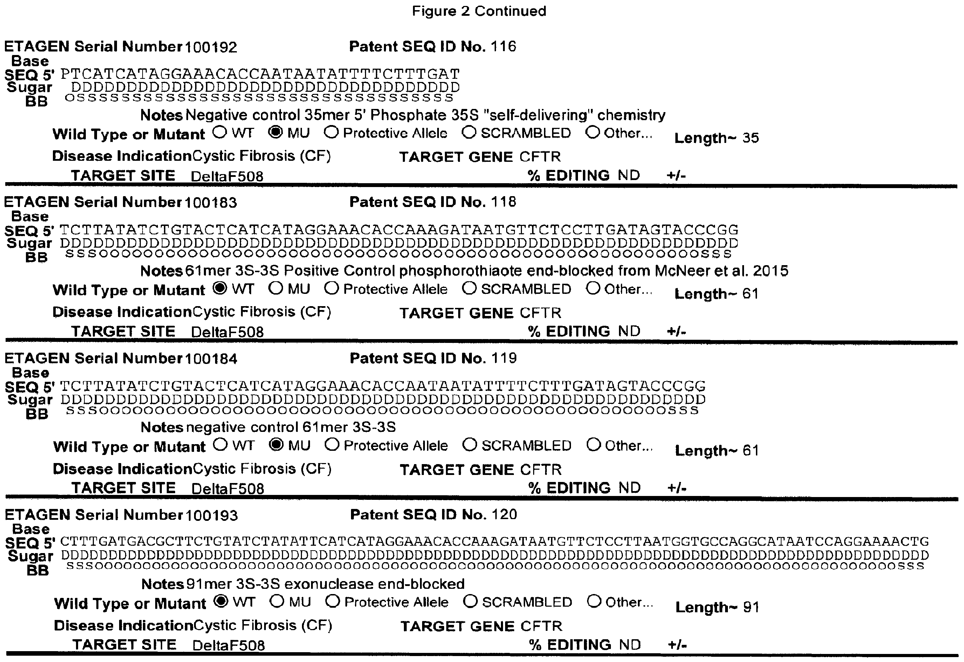

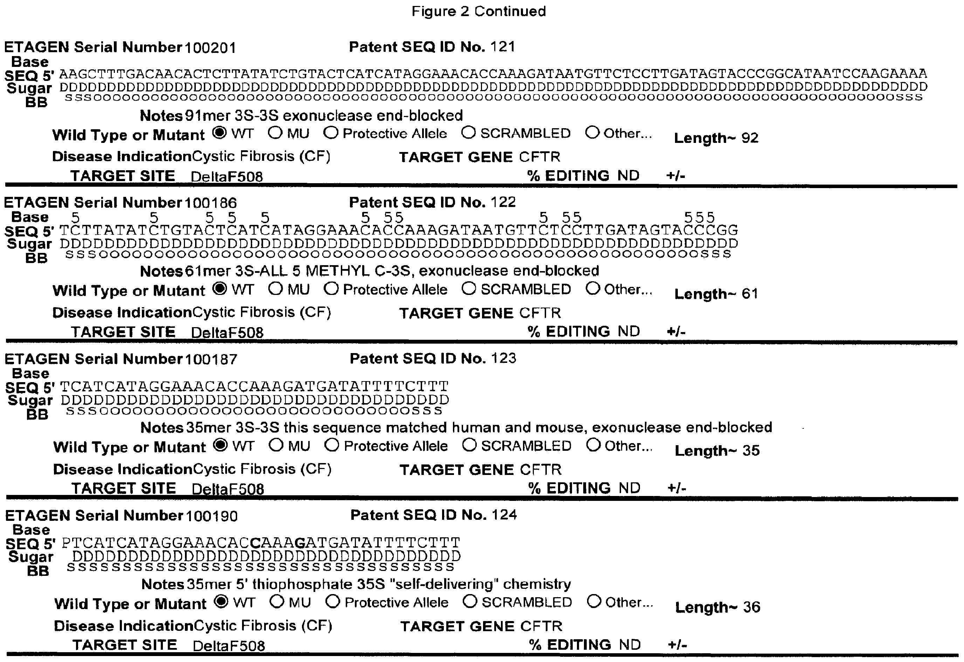

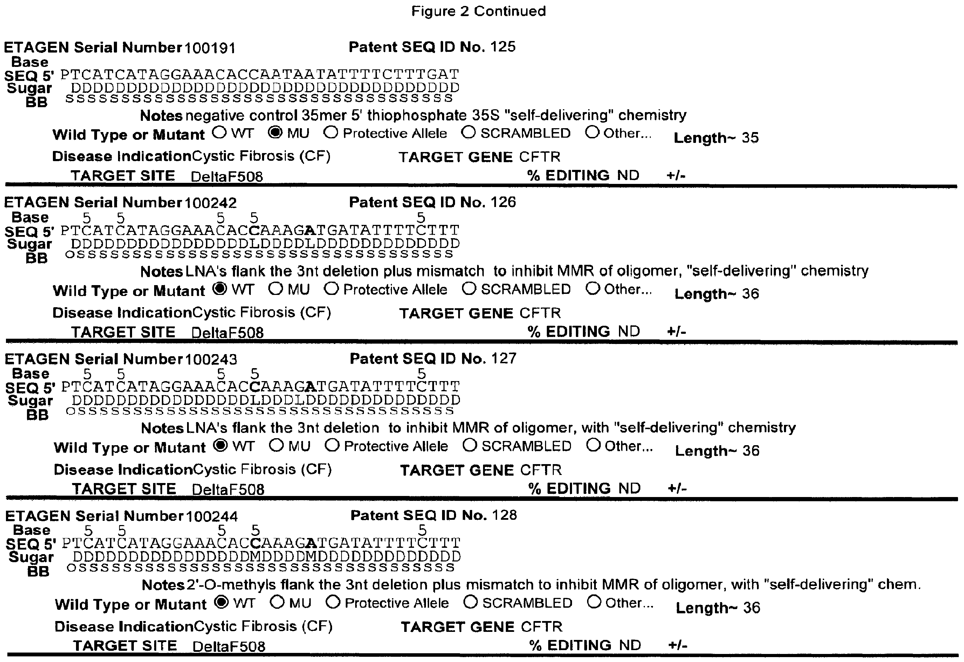

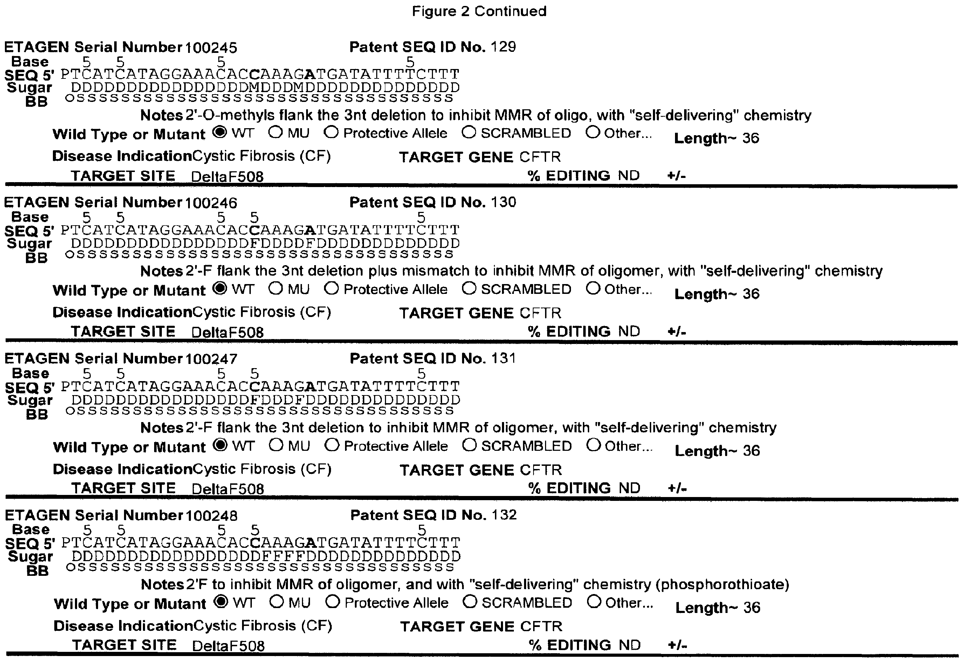

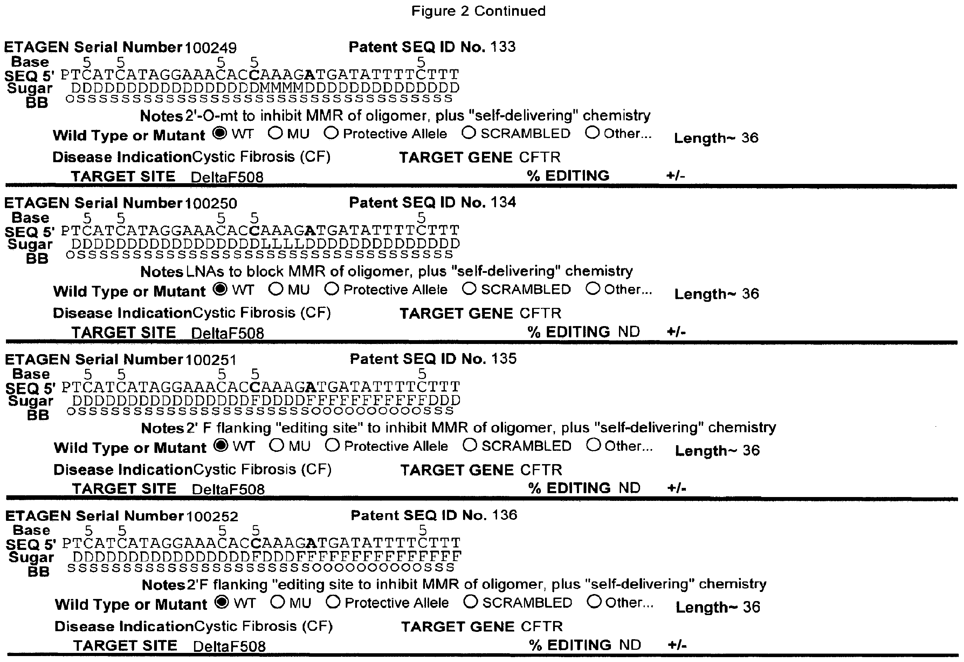

Figure 2: Exemplary list of editing and helper oligonucleotides. The 4 strings of text (base modifications (if any), sequence, sugar and backbone) form a schematic of the modified oligonucleotide. The following abbreviations are provided for the backbone moieties: o=phosphodiester; s=phosphorothioate; and m=methylphosphonate. The following abbreviations are provided for the sequences: when RNA or an RNA analogue, "T" is understood to be "U"; and P= terminal phosphate. The following abbreviations are provided for the sugar moieties: D=DNA; R=RNA; M=2' -O-methyl; F=2' F; L=LNA; and U=unlocked nucleic acid. The following abbreviation is provided for the base moiety: A=Adenine; T=Thymidine; U=Uracil;, G=Guanine; C=Cytosine; and 5=5methyl C. The following character is used indicate a linker "-"=Linker. Other abbreviations include: ND=No Data; PNAs begin in the SEQUENCE string with "N terminus"; K=lysine, O=8-amino-2,6-dioxaoctanoic acid linker; and J=stands for pseudoisocytosine. Underlined subunits are as follows: For ETAGEN serial number 100197=gamma miniPEG (PNA BIO, Thousand Oakes, California), for ETAGEN serial number 100198 and 100199=glutamic acid (PNA BIO, Thousand Oakes, California).

### DETAILED DESCRIPTION

Unless defined otherwise, all terms used herein have the same meaning as are commonly understood by one of skill in the art to which this invention belongs. All patents, patent applications, website postings and publications referred to throughout the disclosure herein are incorporated by reference in their entirety. In the event that there is a plurality of definitions for a term herein, those in this section prevail.

As used herein, the letters "G," "C," "A", "T" and "U" each generally stand for a nucleotide that contains guanine, cytosine, adenine, thymine and uracil as a base, respectively. However, it will be understood that the term "nucleotide" can also refer to a modified nucleotide, as further detailed below. In a sequence it is understood that a "T" refers to a "U" if the chemistry employed is RNA or modified RNA. Likewise, in a sequence, "U" is understood to be "T" in DNA or modified DNA. The skilled person is well aware that guanine, cytosine, adenine, thymine and uracil may be replaced by other moieties without substantially altering the base pairing properties of an oligonucleotide comprising a nucleotide bearing such replacement moiety. For example, without limitation, a nucleotide comprising inosine as its base may base pair with nucleotides containing adenine, cytosine, or uracil. Also, for example, 5-methyl C can exist in the target site DNA or in the editing oligonucleotide in place of C.

The term "oligonucleotide" as used herein refers to a polymeric form of nucleotides, either ribonucleotides (RNA), deoxyribonucleotides (DNA) or other substitutes such as peptide nucleic acids (PNA) (including gamma PNA and chiral gamma PNAs), which is a polymeric form of nucleobases, incorporating natural and non-natural nucleotides of a length ranging from at least 8, or generally about 5 to about 200 or up to 500 when made chemically, or more commonly to about 100 that can be obtained commercially from many sources, including TriLink Biotechnologies (San Diego, CA), Exiqon (Woburn, MA) or PNA BIO (Thousand Oakes, California) and made with methods known in the art (Oligonucleotide Synthesis:

Methods and Applications, In Methods in Molecular Biology Volume 288 (2005) Piet Herdewijn (Editor) ISBN: 1588292339 Springer- Verlag New York, LLC), or for longer oligonucleotides, Integrated DNA Technologies (Coralville, Iowa). In cases when specialized synthesis methods are employed, such as when non-chemically synthesized sources of single-stranded DNA are employed, such as single-stranded vector DNA, or reverse transcribed cDNA from *in vitro* transcribed plasmid mRNA, the single-stranded editing "oligonucleotide" or donor DNA can be up to 2,000 nucleotides. Thus, this term includes double- and single-stranded DNA and single-stranded RNA. In addition, oligonucleotides may be nuclease resistant and include but are not limited to 2'-O-methyl ribonucleotides, constrained or Locked Nucleic Acids (LNAs), 2' fluoro, phosphorothioate nucleotides (including chirally enriched phosphorothioate nucleotides), phosphorodithioate nucleotides, phosphoramidate nucleotides, and methylphosphonate nucleotides (including chirally enriched methylphosphonates). The oligonucleotides may also contain non-natural internucleosidyl linkages such as those in PNA or morpholino nucleic acids (MNA). The above definition when included in the phrase "editing oligonucleotides" refers to an oligonucleotide that may further comprise one or more chemical modifications that react with, or promote a reaction with, a nucleotide on the target sequence (*e*.*g*., a nitrosamine).

The term "5'" and "3'", in references to PNAs shall be understood to mean N-terminal or C-terminal respectively.

The term "nucleic acid" as used herein refers to a polynucleotide compound, which includes oligonucleotides, comprising nucleosides or nucleoside analogs that have nitrogenous heterocyclic bases or base analogs, covalently linked by standard phosphodiester bonds or other linkages. Nucleic acids include nucleic acids with 2'-modified sugars, DNA, RNA, chimeric DNA-RNA polymers or analogs thereof. In a nucleic acid, the backbone may be made up of a variety of linkages (Table II), including one or more of sugar-phosphodiester linkages, peptide-nucleic acid (PNA) linkages (PCT application no. WO 95/32305), phosphorothioate linkages or combinations thereof. Sugar moieties in a nucleic acid may be ribose, deoxyribose, or similar compounds with substitutions, *e*.*g.,* 2' methoxy and 2' halide (*e*.*g*., 2'-F), LNA (or other conformationally restrained modified oligonucleotides) and UNA (unlinked nucleic acid) substitutions (Table IV).

The term, "2'-modified sugar" as used herein regarding nucleic acids refers to 2'F, 2'amino, 2'-O-X (where X is a modification known in the art to result in a hybridization capable oligonucleotide, including, but not limited to an alkyl group (*e*.*g*., methyl, ethyl or propyl) or a substituted alkyl group such as methoxyethoxy or a group that bridges the 2'ribose to the 4'ribose position (*i*.*e*., often referred to as constrained nucleotides) including but not limited to LNAs and cET-BNAs, a bridging 3'-CH₂- or 5'-CH₂-, a bridging 3'-amide (-C(O)-NH-) or 5'-amide (-C(O)-NH-) or any combination thereof (see Table IV for additional examples).

The term, "target sequence" as used herein refers to a contiguous portion of the nucleotide sequence of a DNA sequence in a cell or RNA sequence in a cell that is to be modified by the editing oligonucleotide.

The term "crisprRNA" when used herein refers to crRNA or CRISPR RNA.

The term "complementary," when used to describe a first nucleotide sequence in relation to a second nucleotide sequence (e.g. the editing oligonucleotide and the target nucleic acid), refers to the ability of an oligonucleotide or polynucleotide comprising the first nucleotide sequence to hybridize and form a duplex (or triplex) structure under certain conditions with an oligonucleotide or polynucleotide comprising the second nucleotide sequence, as will be understood by the skilled person. A preferred hybridization condition is physiologically relevant conditions as may be encountered inside an organism, can apply. The skilled person will be able to determine the set of conditions most appropriate for a test of complementarity of two sequences in accordance with the ultimate application of the hybridized nucleotides.

Hybridization includes base-pairing of the oligonucleotide or polynucleotide comprising the first nucleotide sequence to the oligonucleotide or polynucleotide comprising the second nucleotide sequence over the entire length of the first and second nucleotide sequence. Such sequences can be referred to as "fully complementary" with respect to each other herein, but in some case with an editing oligonucleotide of this invention, one or more bases are different from the complementary base of the target sequence.

The term "substantially complementary", as used herein, refers to the relationship between an oligonucleotide of the invention and a target genomic sequence, wherein a sufficient percentage of nucleotides of the oligonucleotide are paired with nucleotides of the target sequence to promote hybridization. In some embodiments, the percentage is greater than 99, greater than 95, or greater than 90 percent. In some embodiments, the percentage is greater than 80, greater than 70, or greater than 60 percent.

The term "complementary sequences", as used herein, may also include, or be formed entirely from, non-Watson-Crick base pairs and/or base pairs formed from non-natural and modified nucleotides, in as far as the above requirements with respect to their ability to hybridize are fulfilled.

The term "hybridization," "hybridize," "anneal" or "annealing" as used herein refers to the ability, under the appropriate conditions, for nucleic acids having substantially complementary sequences to bind to one another by Watson & Crick base pairing. Nucleic acid annealing or hybridization techniques are well known in the art (*see*, *e*.*g*., Sambrook, et al., Molecular Cloning: A Laboratory Manual, Second Edition, Cold Spring Harbor Press, Plainview, N.Y. (1989); Ausubel, F.M., et al., Current Protocols in Molecular Biology, John Wiley & Sons, Secaucus, N.J. (1994), or physiological conditions within the cell).

The term "introducing into a cell", "introduction into a cell" as used herein refers to facilitating uptake or absorption into the cell, as is understood by those skilled in the art. Absorption or uptake of the editing oligonucleotide can occur through unaided diffusive or active cellular processes, or by auxiliary agents or devices. The meaning of this term is not limited to cells *in vitro*; an editing oligonucleotide may also be "introduced into a cell", wherein the cell is part of a living organism. In such instance, introduction into the cell will include administration to the organism. For example, for *in vivo* delivery, editing oligonucleotide can be injected into a tissue site or administered systemically. *In vitro* introduction into a cell includes methods known in the art such as electroporation, microinjection, nucleofection, lipofection or ballistic methods.

The term "edit" when used in reference to a target sequence, herein refers to the at least partial editing of the target gene, as manifested by a change in the sequence in the target gene. The extent of editing may be determined by isolating RNA or DNA from a first cell or group of cells in which the target gene is transcribed and which has or have been treated with an editing oligonucleotide, as compared to a second cell or group of cells substantially identical to the first cell or group of cells but which has or have not been so treated (control cells).

Alternatively, the degree of editing may be given in terms of a reduction or increase of a parameter that is functionally linked to the target gene transcription, e.g. the amount of protein encoded by the target gene which is secreted by a cell, or the number of cells displaying a certain phenotype, e.g. apoptosis. In principle, editing may be determined in any cell expressing the target by any appropriate assay.

For example, in certain instances, a target gene is edited in at least about 0.1%, 1%, 3%, 5%, 10%, 20%, 25%, 35%, or 50% of the targeted cells by administration of the editing oligonucleotide of the invention. In a particular embodiment, a target gene is edited in at least about 60%, 70%, or 80% of the targeted cells by administration of the editing oligonucleotide of the invention. The target cell often contains two copies of the target gene, and one or both of those copies can be edited. In some cases, the target cell contains only one copy of the gene targeted for editing and consequently only one desired edit per cell can occur.

The terms "treat", "treatment", and the like, refers to relief from or alleviation of a condition. In the context of the present invention insofar as it relates to any of the other conditions recited herein below, the terms "treat", "treatment", and the like mean to relieve or alleviate at least one symptom associated with such condition, or to slow or reverse the progression of such condition, or to protect against future disease formation.

Treatment may also include modifying the properties of an organism in case of agriculture and industrial applications.

As used herein, the phrases "therapeutically effective amount" and "prophylactically effective amount" refer to an amount that provides a therapeutic benefit in the treatment, prevention, or management of a condition or an overt symptom of the condition.

The specific amount that is therapeutically effective can be readily determined by an ordinary medical practitioner, and may vary depending on factors known in the art, such as, e.g. the type of disease, the patient's history and age, the stage of the disease, and the possible administration of other treatment agents.

As used herein, a "pharmaceutical composition" comprises a pharmacologically effective amount of an editing oligonucleotide and a pharmaceutically acceptable carrier. As used herein, "pharmacologically effective amount," "therapeutically effective amount" or simply "effective amount" refers to that amount of an editing oligonucleotide effective to produce the intended pharmacological, therapeutic or preventive result. For example, if a given clinical treatment is considered effective when there is at least a 25% reduction in a measurable parameter associated with a disease or disorder, a therapeutically effective amount of a drug for the treatment of that disease or disorder is the amount (including possible multiple doses) necessary to effect at least a 25% reduction in that parameter.

The term "pharmaceutically acceptable carrier" refers to a carrier for administration of a therapeutic agent. Such carriers include, but are not limited to carriers described in (PCT/US2015/65348).

In order to edit a target sequence site specifically, an editing agent must have a domain that recognizes the target nucleic acid sequence (see Table I). In certain embodiments, the target recognition domain is an editing oligonucleotide that binds to the target sequence and is substantially complementary to the target sequence, and may comprise one or more chemical modifications. In some embodiments the target nucleic acid is recognized by an engineered sequence specific nucleic acid binding protein (e.g. an engineered transcription factor).

**TABLE I**

| Target Sequence Recognition Modes | | |
|---|---|---|
| **Oligonucleotide Binding Modes:** | | |
| | 1.Watson-Crick hybridization (single stranded region of oligonucleotide binds target single strand to form a duplex. | |
| | 2.Triplex (single stranded region of the oligonucleotide binds target duplex to form a triplex). | |
| | | Clamp or tail clamp (single-strand of oligonucleotide binds single-stranded target by Watson/crick duplex, and another single-stranded region binds resulting duplex forming triplex *(e.g.* McNeer *et al.* , 2015 *supra*) |

| **Target Sequence Recognition Proteins:** | | |
|---|---|---|
| | 1.Naturally occurring or engineered sequence specific RNA binding protein. | |
| | 2. Engineered transcription factors: | |
| | | Talen (without nuclease activity) Zinc Fingers CRISPR ribonucleic protein DNEs without nuclease activity, as described by Precision BioSciences, Durham, NC |

The RNA editing mode is limited to the chemical modification of the targeted nucleobase mode or induction of exon skipping, as homologous recombination and DNA replication modes do not apply to RNA targets.

In one aspect of this invention, a single-stranded oligonucleotide complementary to one of the DNA strands of the genome is utilized for sequence editing (see Figure 1 in PCT/US2015/65348). The desired edit may be a transition or transversion, or a deletion or insertion. In this aspect of the invention, the editing oligonucleotide sequence is the sequence desired after the editing is completed. Without being bound by a particular theory or mechanism, the editing oligonucleotide binds to the partially complementary or fully complimentary target genomic DNA sequence when the target sequence is separated from the opposite genomic strand during cellular processes such as transcription or replication. When binding occurs during DNA replication, the editing oligonucleotide may prime DNA synthesis and thus be incorporated into the nascent genomic DNA strand. In some cases, the hybridization of the editing oligonucleotide to a double-stranded genomic DNA target can occur during "breathing", transient melting or unwinding of the target DNA. Once the heteroduplex is formed between the editing oligonucleotide and target genomic DNA strand, the area of non-perfect complementarity is corrected by cellular DNA repair (including homologous recombination (HR). When the editing oligonucleotide is used as the template for repair, the desired edit will be incorporated into the targeted genomic DNA strand.

Self-delivering editing oligonucleotides are particularly useful for allowing for enhanced tissue penetration compared to tissue penetration with nanoparticles (such as liposomes) that are much larger than a non-encapsulated self-delivering editing oligonucleotide. For example, nanoparticle incorporated editing oligonucleotides were able to edit the CFTR deltaF508 mutation more efficiently in accessible cell culture and nasal epithelium, but yielded much lower editing efficiency in the deep lung (McNeer et al., Nature Comm. DOI:10.1038/ncomms 7952 pgs. 1-11, 2015). In particular, a CF patient's lung has additional mucus, and mucus is difficult to penetrate with nanoparticles, but mucus is able to be penetrated with molecules in the size range of 20-70 nucleotide long editing oligonucleotides. In fact, other forms of self-delivering therapeutic oligonucleotides (*e*.*g*. uniformly phosphorothioate modified antisense oligonucleotides), have been delivered deep into the lung by inhalation.

The editing oligonucleotides of the present invention include some or all of the following segments, listed in order from 5' to 3': a 5' terminal segment; a 5' proximal segment; a 5' editing segment; an editing site; 3' editing segment; a 3' proximal segment; and a 3' terminal segment. These segments are discerned by their location and/or chemical modifications but can be contiguously linked by the nucleic acid backbone, whether modified or natural DNA or RNA. Nucleotides in each of these segments may be optionally modified to improve one or more of the following properties of the editing oligonucleotides: efficiency of editing; pharmacokinetic properties; bio-distribution; nuclease stability in serum; nuclease stability in the endosomal/lysosomal pathway; nuclease stability in the cytoplasm and nucleoplasm; toxicity (*e*.*g*. immune stimulation of toll-like receptors) and the minimal length necessary for efficient editing (*e*.*g*., shorter oligonucleotides that are generally less expensive to make and easier to deliver to a cell *in vivo*). Non-limiting examples of such modifications are provided in the tables below.

**TABLE II**

| Useful Chemistries for Oligonucleotide Backbones |
|---|
| Phosphodiester |
| Alkene containing backbones |
| Amide backbones |
| Backbones having mixed N, O, S and -CH₂ component parts |
| Bridging 3'-NH- or 5'-NH- |
| Bridging 3'-S- or 5'-S- |
| Formacetyl |
| LNA and other constrained sugars (LNA can be considered a backbone modification and sugar modification) |
| Methylene formacetyl |
| Methylenehydrazino |
| Methyleneimino |
| Methylphosphonate nucleotides (includes chirally enriched methyphonsponates) |
| Moranophosphate |
| Morpholino |
| Non-bridging dialkylphosphoramidate |
| P-Boronated |
| Phosphoramidate |
| Phosphorothioate nucleotides (includes chirally enriched phosphorothioate nucleotides) |
| Phosphotriester (alkyl, aryl, heteroalkyl, heteroaryl) |
| PNA including gamma PNAs and right-handed version of yPNA, including mini-PEG (such as di-PEG), right handed gamma PNA, and cyclopentane PNAs, and derivatives thereof (Sahu et al. The Journal of Organic Chemistry 76(14):5614-27, 2011 and Bahal et al. Current Gene Therapy 14(5):331-42, 2014). The mini-PEG, PEG units can be one, two, three or four units long. (Rogers et al. Conjugate Molecular Therapy 20(1):109-118, 2012 doi:10.1038/mt.2011.163 and Watson-Crick Curr. Gene Ther. 14(5):331-342, 2014) or gamma sulphate PNA (Concetta et al., PLoS One. 2012; 7(5):e35774.). |
| Phosphophotriester backbone modifications which are removed in the cytoplasm and or nucleoplasm by endogenous esterases |
| Siloxane |
| Sulfamate |
| Sulfide, sulfoxide |
| Sulfonamide |
| Sulfonate |
| Sulfone |
| UNA (unlocked nucleic acid) |
| Thioformacetyl |
| tricycloDNA (tcDNA) |

**TABLE III**

| Useful Nucleobase Modifications for Oligonucleotides |
|---|
| 2-aminoadenine |
| 2-propyl and other alkyl derivatives of adenine and guanine |
| 2-thiocytosine |
| 2-thiothymine |
| 2-thiouracil |
| 3-deazaadenine |
| 3-deazaguanine |
| 4-thiouracil |
| C-5 propyne |
| 5-halo particularly 5-bromo, 5-trifluoromethyl and other 5-substituted uracils and cytosines |
| 5-halouracil and cytosine |
| 5-hydroxymethyl cytosine |
| 5-methylcytosine (5-me-C) |
| 5-propynyl uracil and cytosine |
| 5-uracil (also known as pseudouracil) |
| 6-azo uracil, cytosine and thymine |
| 6-methyl and other alkyl derivatives of adenine and guanine |
| 7-deazaadenine |
| 7-deazaguanine |
| 7-methyladenine |
| 7-methylguanine |
| 8-azaadenine |
| 8-azaguanine |
| 8-halo, 8-amino, 8-thiol, 8-thioalkyl, 8-hydroxyl and other 8-substituted adenines and guanines |
| 9-(aminoethoxy)phenoxazine (G-clamp) |
| Biotinylated bases |
| Fluorescent labeled bases |
| pseudoisocytosine (preferred for triplex forming oligonucleotides) |
| Hypoxanthine |
| Pseudo complementary bases |
| Universal bases (bind all for complementary bases (A, T(or U), C and G) |
| Xanthine |

**TABLE IV**

| Useful Sugars for Oligonucleotides |
|---|
| Unmodified DNA sugar(deoxyribose) |
| Unmodified RNA sugar (ribose) |
| Examples of Sugar Modifications: |
| 2'-amino |
| 2'-fluoro |
| 2'-O-MCE |
| 2'-methoxyethoxy |
| 2'-O-substuted alklyl |
| 2'-O-X (where X is a modification known in the art to result in a hybridization capable oligonucleotide |
| 2'ribose bridged to the 4'ribose position (*i*.*e*., often referred to as constrained or locked nucleotides) including but not limited to LNAs and cET-BNAs, a bridging 3'-CH₂- or 5'-CH₂-, a bridging 3'-amide (-C(O)-NH-) or 5'-amide (-C(O)-NH-) or any combination thereof. |
| 2'-O-allyl |
| 2'-O-aminoalkyl |
| 2'-O-aminoalkyl, 2'-O-allyl |
| 2'-O-ethyl |
| 2'-O-methyl |
| 2'-O-propyl |
| Alpha anomers |
| Beta anomers D-arabinonucleic acids (ANA) 2'-deoxy-2'-fluoro-beta-D-arabinonucleic acid (FANA) |

**TABLE V**

| Useful Linkers for Editing and Helper Oligonucleotides* |
|---|
| 8-amino-2,6-dioxaoctanoic acid linker |
| 3' C3 amino linker |
| 3' C7 amino linker |
| 5' & 3' C6 amino linker |
| 5' C12 amino linker |
| 5' photo-cleavable amino linker |
| 3' C3 disulfide linker |
| 5' & 3' C6, disulfide linker |
| dithiol linker |
| 4-formylbenzamide aldehyde |
| C8-alkyne-thymidine |
| carboxy-dT linker |
| DADE linker (5' carboxyl linker) |
| 3' glyceryl |
| 5' hexynyl |
| thymidine-5-C2 |
| C6 amino linker |
| 2'-deoxyadenosine |
| 8-C6 amino linker |
| 2'-deoxycytidine-5-C6 amino linker |
| 2'-deoxyguanosine-8-C6 amino linker |
| Internal amino linker |
| * The linker lengths may range from one carbon to about twenty carbons or equivalent length of other chemistries, but preferably below ten carbons or ten carbon equivalent length. |

**TABLE VI**

| Useful Conjugates for Editing and Helper Oligonucleotides (*see* also Table VII, Sequence Modifying Moieties for Nucleobase Chemical Modification and PCT/US2015/65348) |
|---|
| Ligands to receptors (i.e. gal-nac or glp-1) |
| Lipophilic conjugates (that enhance cell penetration), such as in Khvorova et al. U.S. patent application 2014/0364482 and Alnylam U.S. patent no. 8,106,022 and 8,106,022 for lipophilic modification and delivery ligand conjugates. |
| Polymers to extend circulation (*e*.*g*. PEG) |
| For Lung uptake and penetration: |
| Bradykinin Receptor- 9aa ligand |
| Specific Lectins/antibodies |
| Hemaggluttin/Neuraminidase |
| Negatively charged moieties: sulphate, fucose, sialic acid |
| Endothelin |
| Monoclonal antibodies (for example of nucleic acid delivery by monoclonal antibody *see* www.aviditybiosciences.com/Avidity Biosciences, La Jolla, California). |
| ENaC- 18aa peptide ligand (S18) targets and internalizes |

The editing oligonucleotide may comprise a subset or all of the seven segments listed above, and will include an editing site and at least one segment, 5' and 3' to the editing site. Each of these segments may optionally contain the same or different chemical modifications to enhance the properties of the editing oligonucleotide, and the modifications can in some cases be uniform throughout the segment, and in other cases only occur on a portion of nucleotides in the segment.

Other embodiments include editing oligonucleotides with reversible charge-neutralizing phosphotriester backbone modifications, as described in PCT/US2015/65348.

### I. Editing Oligonucleotides

In certain embodiments, the editing oligonucleotides of the invention have the structure according to Formula I:

T₅-P₅-E₅-S_{E}-E₃-P₃-T₃ (I)

### A. The 5' terminal segment (T₅)

The 5' terminal segment is more amenable to a multitude of different types of modifications than the 3' terminus because the 3' terminus functions in priming DNA extension, which may limit the 3'-terminal segment to a modification having a generally free 3' hydroxyl in certain contexts. This priming function is not usually necessary at the 5' terminus. An optional 5' terminal segment, which may be from zero to five nucleotides in length, functions to block 5' exonucleases that may otherwise readily degrade the editing oligonucleotide in bodily fluids (*e*.*g*., blood or interstitial fluids), culture media, the endocytic pathway, or the cytoplasm or nucleoplasm. This segment may comprise a non-nucleotide end-blocking group and/or modified nucleotide(s) that are more nuclease resistant than the 5' proximal segment (*e*.*g*., inverted bases, such as inverted T). A 5' phosphate can increase editing efficiency, and is contained in certain embodiments without (Radecke et al. The Journal of Gene Medicine 8(2) :217-28, 2006) or with a thiophosphate modification (to enhance stability against cellular phosphatases). Another nuclease stable 5' phosphate analog which can be used at the 5' terminus of an editing oligonucleotide is 5'-(E)-vinylphosphonate. The 5'-(E)-vinylphosphonate modification is particularly useful for editing oligonucleotides that load into Argonaut (R. Parmar, J. L. S. Willoughby, et al. ChemBioChem 17:85. 2016). If the 5' terminal segment is not employed, then the 5' proximal segment is simply the most 5' portion of the editing oligonucleotide. Non-nucleotide end-blocking groups may include any linker known to those skilled in the art for use in performing this task (*see* Table V for a list of these and other useful linkers for this position). The linker lengths may range from one carbon to about twenty carbons or equivalent length of other chemistries, but preferably below ten carbons or ten carbon equivalent length. The linker can be used to attach a delivery moiety, such as cholesterol or 1-3 Gal-Nac moieties.

The 5' terminal nucleotide exonuclease resistant segment may comprise one, two, three or four phosphorothioate modifications. In addition to these one or more phosphorothioate modifications, or in place of them, the 5' terminal exonuclease resistant nucleotides may comprise 2' sugar modifications, which are known to enhance exonuclease stability. Additionally, neutral nucleotide analogues such as methylphosphonates, morpholinos or PNAs are highly resistant to exonucleases, and one, two, three, four or five of such modifications at the 5' terminus may be utilized as exonuclease end-blocking groups. In a particularly useful embodiment the 5' terminus is two methylphosphonates (see Figure 2 in PCT/US2015/65348). These terminal groups don't necessarily have to be complementary to the target.

### B. The 5' proximal segment (P₅)

The 5' proximal segment is more amenable to certain types of modifications than the 3' terminus for some of the same reasons as previously stated for the 5' terminal segment above. It may be from one to 150 nucleotides in length, or up to 1500 nucleotides in length and preferably from about five to about twenty nucleotides in length. The main function of the 5' proximal segment is to enhance the affinity and ability of the editing oligonucleotide to hybridize to the target sequence. Therefore, this segment can optionally be more substantially modified than the editing segment. The 5' proximal segment may contain any of the oligonucleotide modifications referenced herein. This segment may be comprised of DNA or RNA (optionally 2' modified RNA defined broadly to include LNAs and other constrained backbones). While alternate phosphorothioates (*e*.*g*., diphosphorothioates and phosphorothioates with enhanced chiral purity) in this region are not strictly required for nuclease stability, these alternate phosphorothioates will be useful to enhance nuclease stability of RNA and DNA linkages. Also, even when the phosphorothioates in this segment are not necessary for nuclease stability, they may add to the overall phosphorothioate content, which due to the chemically "sticky" nature of phosphorothioates, increases serum protein binding and cell binding that leads to increased serum half-life in animals and humans and enhances cytoplasmic uptake.

For these reasons, in a particularly useful embodiment, the total phosphorothioate content of the editing oligonucleotide may be greater than five, ten, fifteen or twenty. A content of about twenty phosphorothioates often provides for excellent serum protein binding and cell binding/uptake. However, because large numbers (*e*.*g*., more than 6) of phosphorothioate linkages in the complementary region of editing oligonucleotides can inhibit editing efficiency, a phosphorothioate tail may be added to the 5' or 3' terminus of the region complementary to the target DNA. This tail may be from 1 to about 4, about 5 to about 9 nucleotides or about 10 to about 25 nucleotides in length and may preferably be non-complementary to the target. Other modifications described herein, can be incorporated in the 5' proximal segment to enhance nuclease stability, reduce immune stimulation and/or increase target DNA binding affinity, such as ANA or FANA modified nucleotides. When using an editing oligonucleotide in "self-delivering" mode (not encapsulated), the oligonucleotide must survive transit through the endolysosomal pathway that has high endonuclease activity. Therefore in the "self-delivery" mode, an editing oligonucleotide with endonuclease resistant modifications at most or all nucleotides in the editing oligonucleotide is particularly useful. This can be achieved with various modifications, but phosphorothioate and/or 2'-O-methyl modifications are particularly useful in the 5' proximal segment. One to several unmodified DNA or RNA linkages, or other linkages that are labile within a cell can optionally be placed in the 5' most region of this segment, if it is desired to have the 5' terminal segment removed within the cell to, for example, expose of free 5' OH or 5' phosphate.

### C. The 5' editing segment (E₅)

The 5' editing segment is from one to about ten nucleotides in length, or one to about 100 nucleotides, or one to about 200 nucleotides and is positioned on the 5' end of the editing site, which is sufficiently close to the editing site to affect the cellular machinery that results in editing of the opposing genomic target DNA strand. While not being bound by any theory, the DNA mismatch repair system may use the editing segment (which is the 5' editing segment, editing site and 3' editing segment) as the template strand for editing of the target strand or for subsequent chromosomal DNA replication after incorporation of the editing oligonucleotide into a replication fork. Therefore, most of the nucleotides in the 5' editing segment and 3' editing segment are preferably substantially similar to natural DNA, (*e*.*g*., Editing Oligonucleotide 100013 has about 8 unmodified nucleotides 5' of the editing site (*see* Figure 2), which did not inhibit the overall editing efficiency, compared to the parent compound) or natural DNA chemistry and may be unmodified or include one or more modifications such as phosphorothioates (Radecke et al. The Journal of Gene Medicine 8:217-28, 2006), 5' S, 2'F, 2' amino or 3' S, reversible charge-neutralizing phosphotriester and nucleobase modifications.

In one particularly useful embodiment of the present invention, one, some or all of the deoxy-cytosines in the editing oligonucleotide are modified to 5 methyl cytosine, particularly, the cytosine nucleosides within about five to about 10 bases, 5' or 3', of the editing site(s). One reason for incorporating 5 methyl cytosines into the editing oligonucleotide is that during replication followed by mismatch repair, the mismatch repair machinery recognizes unmethylated cytosines as the nascent strand, and preferentially uses the 5 methyl cytosine containing DNA strand as the template-strand for repair. In addition, if the editing oligonucleotide contains few or no 5 methyl cytosines, then the repair machinery will likely not select this strand as the template during the DNA repair reaction that leads to editing. The fact that editing oligonucleotides in the art have not contained multiple 5-methyl cytosines is one of the reasons for their relatively low efficiency in editing.

### D. The editing site (S_{E})

The editing site contains the nucleotide(s) which are not complementary to the target genomic DNA and may be from one to six nucleotides in length, but can be longer as required. In the case of a transition/transversion modification, the editing site is equal to the number of mismatched bases (*e*.*g*., one to about six nucleotides, particularly 1). In the case of editing to create a deletion, the editing site is the junction between the two 5' and 3' nucleotides which are base-paired to the target genomic DNA strand, just opposite the non-based paired nucleotides in the genomic DNA. In the case of editing to create an insertion, the editing site will be the nucleotides containing the insertion. In some cases, one editing oligonucleotide may be used to treat different mutations at nearby sites within the region of complementarity to the target DNA that occur in different patients in the population. In this case there will be different editing sites, depending on the patient's mutant genotype. In these cases, the editing site would include the 5' and 3' most mutations and the region in between those mutations. In one particularly useful embodiment, the editing site extends to an entire exon in the target gene, or the exon including the intro/exon boundary regions, which are often sites of mutations. Alternatively, when employing the chemical modification method, the base modifying activity is placed in proximity to the nucleobase targeted for editing. In this case, the base modifying activity can be conjugated covalently to the editing oligonucleotide at any position along the oligonucleotide, or can be associated with the editing oligonucleotide non-covalently (Woolf et al. PNAS USA 92(18) :8298-302, 1995, Woolf Nature biotechnology. 16(4):341-4, 1998, Montiel-Gonzalez et al. PNAS 110(45):18285-90, 2013 and Komor et al. Nature, 2016; advance online publication (Komor et al. Nature. April 20 2016;advance online publication doi: 10.1038/nature17946). Phosphorothioate modifications at the editing site are consistent with significant editing activity (Radecke et al. The Journal of Gene Medicine 8:217-28, 2006 and Papaioannou et al. The Journal of Gene Medicine 11:267-74, 2009).

In a particularly useful embodiment, the editing oligonucleotide has a 5-methyl cytosine in a CpG sequence at the editing site. In a more useful embodiment, this CpG in the editing site is mispaired to TpG in the target sequence. In this case, the cellular 5-methyl binding protein will bind to the mismatched methylated CpG and lead the cellular mismatch repair system to convert the mismatched T into a matched C. The 5' editing segment may contain no modified nucleotides or one or more modified nucleotides up to the number of nucleotides in the segment.

When an editing oligonucleotide is bound to the target DNA strand, resulting in a mismatch between the target sequence and the editing oligonucleotide, the cellular mismatch repair machinery can repair the target DNA sequence to match the editing oligonucleotide sequence ("productive repair") or the cellular mismatch repair machinery can "repair" the editing oligonucleotide ("non-productive repair"). The non-productive repair changes the editing oligonucleotide to the mutant or otherwise undesirable target DNA sequence. While not wishing to be bound by any particular theory or mechanism, one method of avoiding "non-productive repair" is to chemically modify the "editing site" of the editing oligonucleotide and/or nucleotides flanking the "editing site" in the 5' editing segment and/or 3' editing segment with modifications that inhibit "non-productive repair" (see PCT/US2015/65348). Modified nucleotides shown to inhibit "non-productive" repair by the cellular mismatch repair machinery included 2'F (Rios, X. et al. PLoS ONE 7, e36697, 2012 doi:10.1371/journal.pone.0036697) and LNA (van Ravesteyn, T. W. et al. PNAS USA 113, 4122-4127, 2016 doi:10.1073/pnas.1513315113). Other useful modifications 3' and/or 5' to the "editing site" include the oligonucleotide chemical modifications described herein, and particularly the nuclease resistant chemical modifications described herein, and particularly ANA, FANA, and more particularly 2' modifications described herein, and particularly 2'-O-alkyl modifications which are chemically "between" 2'F and LNA, and are easier to manufacture and have less toxicity than 2'F and LNAs, and more specifically 2'-O-methyl as
exemplified herein (see examples in figure 2). Constrained nucleic acids, in addition to standard LNAs, such as cET chemistry, can also be employed in this position(s).

In the case of a mismatch (es) that is being edited, the mismatched base can be modified (the "editing site") or the mismatched base(s) plus the next nucleotide(s) 3' and/or 5 of the "editing site" can be modified (PCT/US2015/65348, Rios, X. et al. PLoS ONE 7, e36697, 2012 doi:10.1371/journal.pone.0036697 and van Ravesteyn, T. W. et al., Proc Natl Acad. Sci. USA 113, 4122-4127, 2016 doi:10.1073/pnas.1513315113). In the case of an editing oligonucleotide that is inserting a nucleotide(s) (*e.g*. a repairing oligonucleotide for Cystic Fibrosis delta-F508 (see Example 4 and Cystic Fibrosis delta-F508 oligonucleotide sequences herein), the inserted sequence may be modified, or the next nucleotide(s) 3' and/or 5 of the "editing site" may be modified
(van Ravesteyn, T. W. et al. PNAS USA 113, 4122-4127, 2016 doi:10.1073/pnas.1513315113). These modification patterns (PCT/US2015/65348) may lead to pronounced enhancements of editing, particularly when a cell that is competent for mismatch repair is being targeted for editing (van Ravesteyn, T. W. et al. PNAS USA 113, 4122-4127, 2016 doi:10.1073/pnas.1513315113).

### E. The 3' editing segment (E₃)

The 3' editing segment can have the same range of features, properties, chemical modifications and parameters as the E₅ the 3' editing segment, except for its location being 3' of editing site.

### F. The 3' proximal segment (P₃)

The 3' proximal segment has the same range of features and parameters as the 5' proximal segment, except for its location relative to the other segments. In a particularly useful embodiment, the 3' proximal segment is comprised of 2' modified nucleotides, and in a more particularly useful embodiment, it is comprised of 2'F modified nucleotides. In one particularly useful embodiment, it comprises 8 2' F modified nucleotides (Figure 2 in PCT/US2015/65348 and Figure 2 herein). Other chemical modifications described herein, can be incorporated in the 3' proximal segment to enhance nuclease stability, reduce immune stimulation and/or increase target DNA binding affinity, such as ANA or FANA modified nucleotides.

### G. The 3' terminal segment (T₃)

The 3' terminal segment may comprise the same range of features and properties as the 5' terminal segment, except as elaborated below. The 3' terminal segment may serve as a primer for DNA synthesis during DNA replication and repair, thus allowing the editing oligonucleotide to become contiguously incorporated into the genomic DNA. Consequently, in one embodiment this segment will have a free 3' hydroxy and may be made of natural-like modifications or unmodified DNA or RNA.

While non-nucleotide end-blocking groups at the 3' terminus may, in some cases, reduce or eliminate editing activity, if there is a region of RNA between the editing site and the 3' terminus of the editing oligonucleotides that is cleaved by RNase H upon hybridization of the editing oligonucleotide to the target DNA, then a free 3' hydroxyl suitable as a primer for chain extension will be created. Other designs that liberate a free 3' hydroxyl include a region of unmodified DNA (*e*.*g*., 1-10 nucleotides in length) that

will be degraded by endonucleases within the nucleus,
as Monia *et al.* have clearly demonstrated exonuclease degradation of internal phosphodiester DNA bonds within oligonucleotides in cells (Monia et al. J. Biol. Chem. 271(24):14533-40, 1996) . Another method of liberating a free 3' hydroxyl is the use of heat labile linkages or end-blocking groups, that can be tuned to slowly degrade liberating a 3' hydroxyl group at various temperatures, preferably physiological temperature (Lebedev et al. Nucleic Acids Res. 36(20):e131, 2008). Also, another editing mechanism, known as mismatch repair, may not require a free 3' hydroxyl or region of the editing oligonucleotide base paired to the target at the 3' terminus (PCT/US2015/65348) .

The oligonucleotide of Formula (I) may comprise 20-2000 nucleotides or up to about 3000 nucleotides. In one embodiment, the oligonucleotide may comprise 100-250 nucleotides. In another embodiment, the oligonucleotide may comprise 250-2000 nucleotides. In a particular embodiment, the oligonucleotide comprises 20-100 nucleotides. In a particular embodiment, the oligonucleotide comprises 25-90 nucleotides. In a particularly useful embodiment, the oligonucleotide comprises 19-50 nucleotides. When employing modified chemistries that enhance the affinity of the editing oligonucleotide for the target DNA, editing oligonucleotides as short as 12 nucleotides are useful.

The current design of single-stranded editing oligonucleotides using the methods of Brachman and Kmiec employs unmodified DNA, which is less efficient for editing, or three phosphorothioates on each terminus with the rest of the editing oligonucleotide comprising unmodified DNA, which is more efficient for editing, having an optimal length of approximately seventytwo nucleotides in both cases. While Brachman and Kmiec found that synchronized cells treated in S-phase were most efficiently edited (Engstrom and Kmiec, Cell Cycle 7(10):1402-1414, 2008), we now know that editing oligonucleotide designs of Kmiec described above are highly susceptible to cellular endonucleases. In order to increase the editing efficiency, and not require cell synchronization (*e*.*g*. because a stable editing oligonucleotide will persist in each cell until the cell enters the S-phase naturally), the present invention provides embodiments of editing oligonucleotides that have enhanced nuclease resistance. Figure 2 provide examples of these editing oligonucleotides. Increased resistance to cellular endonucleases may be achieved by modifying one or more of the nucleotide linkages to phosphorothioate linkages that are positioned near the 5' and/or 3' terminus. In one embodiment, four or more phosphorothioate linkages at one or both termini are particularly useful. Resistance may also be achieved by replacing all of the nucleotide linkages with phosphorothioate linkages, except for the "editing segment". In some embodiments,
the phosphorothioate modifications may comprise from one up to seven of the nucleotide linkages surrounding the editing bases (*e.g*., the bases that are different from the target sequence). In one embodiment all the linkages of an editing oligonucleotide are phosphorothioate DNA. In other embodiments the editing oligonucleotide has the configuration of phosphorothioate patterns and lengths shown in Table 2 in (PCT/US2015/65348). The phosphorothioate linkages can also be wholly or partly alternating, with every other linkage being a phosphorothioate linkage, or every third linkage being a phosphorothioate linkage, or 2 phosphorothioate linkages alternating with one or two phosphodiester linkages, and the like. The editing oligonucleotide may comprise from about 10%, 20%, 30%, 40%, 50%, 60%, 70%, 80%, 90% or 100% phosphorothioate linkages. While the editing oligonucleotides tested in cell culture with ∼50% or more phosphorothioate DNA (ETAGEN 100007, 100010 and 100022) had low or no GFP cell counts above background in our cell culture GFP target system, these chemistries are also active antisense cleavers of target RNA. The active antisense chemistries would transiently (several days) inhibit the GFP by antisense, and thus this confounds editing efficiency analysis of these compounds in this assay. In other cell target systems that employed editing oligonucleotides in the sense orientation, the sense DNA editing oligonucleotides with about half phosphorothioates substitutions on the 3' half of the oligonucleotide had high genome editing efficiency, and even 100% phosphorothioate DNA editing oligonucleotides retained reduced but significant editing efficiency (Radecke, et al. The journal of gene medicine 8, 217- 28, 2006)). Even though 100% phosphorothioate substitution reduced editing efficiency, the molecules will distribute to tissues and are nuclease stable enough to efficiently "self- deliver" to the interior of cells, which is a major advantage when
employing naked (non-encapsulated) editing oligonucleotides in animals and humans. These phosphorothioate configurations can be combined with other modifications or natural sugars, as described herein. In particular, some of the DNA sugars may be replaced with RNA sugars. Preferably, the RNA substitutions will comprise a block of RNA linkages beginning at the 3' terminus, and extending in the 5' direction for one, two, three, four, five, six, seven, eight, nine or ten bases. This is to make the 3' end appear as a natural Okazaki fragment or DNA primer, which will lead to more natural priming of synthesis and removal by RNase H. However a stretch of 3 or more unmodified RNA nucleotides is not preferred in the "editing segment", because it is not desirable to have the editing segment removed by RNase H.

Notwithstanding the above description of an editing oligonucleotide that acts by homologous recombination or primer extension, if the editing oligonucleotide is designed to edit solely by the virtue of comprising reactive chemical groups or enzymatic activity that modifies the targeted nucleobase, then the requirements of the editing oligonucleotide modification pattern is simpler. This class of editing oligonucleotide can be made of one or two uniform chemical modifications, such as all PNA, or all phosphorothioate along with all 2' modifications (i.e. all phosphorothioate with all 2'-O-methyl, or all phosphorothioate with all LNA).

### H. Other Modifications and Modification Patterns

An approach to making the editing oligonucleotide more efficient is to increase affinity through chemical modification. The modifications described herein may be combined in the editing oligonucleotide and include 2'-O-methyl RNA, 2'F RNA and constrained nucleic acids, including LNAs. These modifications have the additional advantage that they can also reduce immune stimulation. The modifications may be grouped to form a high affinity "seed" region for hybridization. In a particularly useful embodiment, this seed region would be positioned in the 5' proximal segment, with about two to about twelve successive modifications.

In other embodiments, the seed region may be positioned in the 3' proximal segment. The modifications can be alternating or every third or fourth linkage, in the 5' segment, the editing segment and/or the 3' segment. The total proportion of chemically modified nucleobases in the 5' segment, 3' segment, or editing segment may range independently from about 20%, 30%, 50%, 60%, 70%, 80%, 90% or 100%. In one embodiment, the editing oligonucleotide comprises a stretch of 8 or more alternating 2' F and 2'-O-methyl linkages, as these are known to interact well with Argonaut proteins that can enhance the hybridization rate to the target to increase potency.

Provided herein are various embodiments of the oligonucleotide of Formula (I). For example, Formula (I) may be RNA, DNA, single-stranded, unmodified, and/or chemically-modified, which may include sugar modifications (particularly, *e*.*g*., 2'-O-methyl and 2'-fluoro). Formula (I) may comprise one or more backbone modifications and/or a linker, as described herein.

Formula (I) may further comprise a conjugated molecule *(e.g.,* gal-nac or a lipophilic modification). The foregoing modifications may be present in various combinations. For example, one, two or three backbone modifications may be present with one, two or three sugar modifications and/or a linker and/or a conjugated molecule.

Certain oligonucleotides of the invention comprise one or more chemical modifications that react with, or promote a reaction with, a nucleotide on a target sequence. Examples of such reactions include alkylation, acetylation, cross-linking, amination, de-amination, generation of a free (non-covalently bound) reactive compound. Various classes of sequence modifying moieties useful for editing oligonucleotides are described in Table VII and the structure, synthesis and conjugation of some of these moieties are detailed in PCT/US2015/65348.

**TABLE VII**

| Sequence Modifying Moieties for Nucleobase Chemical Modification |
|---|
| **Chemical-based:*** |
| -Alkyator |
| -Acetylator |
| -Cross-linker |
| -Aminator |
| -Deaminator |
| -Generating a free (non-covalently bound) reactive compound |

| **Enzyme:** |
|---|
| -Alkyator |
| -Acetylator |
| -Deaminator |

| **Catalytic nucleic acid (*e*.*g*. ribozyme or DNAzyme)** |
|---|
| *In each case above, including optionally protecting reactive groups, to avoid reactivity during synthesis, purification, storage, and prior to reaching the target within cell, the protecting groups being released by: pH, such as low pH in endosome or lysosome intracellular esterases or are sensitive to the reducing environment of the cell. |

As an alternative to employing oligonucleotides for editing, a nucleobase modifying activity (from Table VII), can be bound or conjugated, or fused to a protein only target sequence recognition domain (see example of such proteins in the table VIII), to achieve genome editing.

**TABLE VIII**

| Proteins and Catalytic Nucleic Acids that may be Combined with Editing Oligonucleotides to Enhance Editing Efficiency |
|---|
| **Programmable or designable site specific nucleases:** |
| Ribonucleoprotein with nuclease activity (i.e.) Forms of CRISPR-Cas9 variants listed in Table IX or Argonauts, such as Natronobacterium gregoryi Argonaute ((Gao F, Shen XZ, Jiang F, Wu Y, Han C. DNA-guided genome editing using the Natronobacterium gregoryi Argonaute. Nat. Biotech. 2016;advance online publication) |
| TALENS |
| Zinc Fingers |
| Meganucleases including DNEs as described by Precision BioSciences, Durham, NC. |
| **Exogenous Proteins that enhance strand invasion and/or recombination:** |
| Nuclease inactive ribonucleic proteins (*i*.*e*. CRISPR-Cas9 and variants listed in Table IX, Forms of CRISPR/Cas9 Variants. |
| RecA and single-stranded DNA binding protein |
| Lambda phage beta protein (U.S. patent no. 7,566,535) |
| RADs |
| Non-protein DNA cleavage catalysts: |
| Catalytic nucleic acid (*e*.*g*. ribozyme or DNAzyme) conjugated or integral to the editing or helper oligonucleotide (Marcel Hollenstein Molecules 2015, 20(11), 20777-20804, 2015 doi:10.3390/molecules201119730 and Edmund et al. Chemistry & Biology November T:761-770, 1995 |
| The proteins and catalytic nucleic acids listed above can be covalently linked to the editing oligonucleotide, non-covalently linked, or can be mixed with editing oligonucleotide prior to application to cells or animals or they can be administered separately from the editing oligonucleotides directly, or by means of an expression vector. |

In certain embodiments, the chemical modification method can be combined with the "HR" method of editing to bias the endogenous mismatch DNA repair machinery to repair the targeted genomic DNA strand. In this embodiment the chemical modification of the targeted DNA strand can target the nucleobase targeted for editing or a nucleobase or nucleotide in the proximity of the nucleobase to be edited (*e*.*g*., within 1-10 or 1-50 bases away), and simply by virtue of causing damage (adducts) to the targeted strand activate the endogenous repair machinery to repair the DNA damage and mismatches in the vicinity of the damage using the editing oligonucleotide as a template (productive editing).

Oligonucleotides of the invention may comprise protecting groups. Suitable protection groups are known to those skilled in the art to protect chemically-reactive groups during synthesis, purification, storage and during use (*e*.*g*., to protect the oligonucleotide from conditions including acidity, intracellular esterases or reducing conditions).

While it is convenient to use only a single oligonucleotide to achieve editing, certain enhancements to the embodiments herein include additional oligonucleotides. The use of a "helper" oligonucleotide or "helper" oligonucleotides, wherein "helper" oligonucleotide(s) refers to an oligonucleotide which would bind tandemly to the editing oligonucleotide (*e*.*g*., at the 3' end, 5' end or both ends in the case of two helper oligonucleotides or further away from the editing oligonucleotide binding site, for example within 200 nucleotides 5' or 3' of the editing site. The helper oligonucleotides will help open up the structure of the target site or otherwise improve the efficiency of binding of the editing oligonucleotide. For helper oligonucleotides that bind in whole or in part by strand invasion with Watson/Crick binding, high affinity chemistries are particularly useful. In particular,
chemistries with higher affinity to the target DNA than the affinity of the target DNA for the natural opposing DNA strand are particularly useful, such that the displacement loop formed will be energetically favored. Even higher affinities, such as can be obtained with LNAs (Geny et al. Nucl. Acids Res. (2016) doi: 10.1093/nar/gkw021First published online: February 8, 2016) or PNAs including R mini-PEG gamma PNAs can allow for higher efficiency of strand invasion (Sahu et al. Journal of Organic Chemistry 76(14):5614-27, 2011, Bahal et al. Current Gene Therapy 14(5):331-42, 2014) and Bahal, R. et al. Nat. Commun. 7, 13304 (2016). Another target of helper oligonucleotides may be the opposite strand of the DNA strand targeted by the editing sequence. In this case, the helper oligonucleotide(s) preferably binds just 5' and/or 3' of the binding site of the editing oligonucleotide, so as to not hybridize strongly to the editing oligonucleotide
itself. In other embodiments, the 5' and/or 3' helper oligonucleotides overlap with the editing oligonucleotide binding site by about 1-5, about 5-10, or about 1-15 bases. In this manner, the helper oligonucleotides would not bind too tightly to the editing oligonucleotide to negatively impact the editing oligonucleotides binding to the target. These helper oligonucleotides could optionally be linked to the editing oligonucleotide covalently by phosphodiester or modified phosphodiester linkages, or by other covalent linkers (see Table V, Useful Linkers for Editing and Helper Oligonucleotides for linker examples). In another embodiment triplex forming oligonucleotides or oligonucleotide analogs bind to the target DNA within about 200 nucleotides of the editing site resulting in increased editing efficiency (McNeer et al., Nature Comm. DOI:10.1038/ncomms 7952 pgs. 1-11, 2015), Bahal et al. Current Gene Therapy 14(5) pp331-42 (2014), Chin et al. PNAS 105(36):13514-13519 (2008), Rogers et al. PNAS 99(26):16695-16700 (2002), U.S. Patent 8,309,356 and Bahal, R. et al.Nat. Commun. 7, 13304 (2016).

Some helper oligonucleotides protect oligonucleotides that are complementary to the editing oligonucleotide and block nuclease degradation by single-strand specific nucleases (PCT/US2015/65348).

Helper oligonucleotides can be made comprising self-delivering chemistries described herein. For neutral backbone helper oligonucleotides, such as PNAs, self-delivering charged groups or strings of amino acids known in the art can be employed (Natee Jearawiriyapaisarn et al. Molecular Therapy 16:1624-1629, 2008, Sazani et al. Nature Biotechnology, 20:1228-1233, 2002). For example, see helper oligonucleotides in which lysines are placed at the termini of PNA oligonucleotides to allow for delivery of naked oligonucleotide in cell culture and *in vivo* in Figure 2.

While it is useful from a clinical safety standpoint to not cleave the targeted DNA, it is known that cleavage of the targeted DNA can enhance editing efficiency. In cases in which higher editing efficiency is desired, a chemical DNA cleaving moiety (*e*.*g*. chelator, Simon et al. Nucleic Acids Research 36(11) :3531-3538, 2008. doi:10.1093/nar/gkn231.) can be conjugated to the editing oligonucleotide or helper oligonucleotide to cleave one or both strands of the targeted DNA in order to further enhance editing activity. This approach has the advantage over programmable nuclease methods known in the art, because this method does not require delivery or expression of an immunogenic engineered protein, and because the cleavage activity is covalently attached to the editing oligonucleotide, placing the editing oligonucleotide in proximity to the cleavage site.

### I. Enhancing Editing with Exogenous and Endogenous Proteins

While it is advantageous to not strictly require exogenous proteins in editing compositions, certain exogenous proteins can enhance the embodiments described above, by protecting the editing oligonucleotide from nuclease degradation and by enhancing the binding of the editing oligonucleotide to target genomic DNA. The chemically modified editing oligonucleotides described herein, can be used as the donor oligonucleotides for homologous recombination editing with programmable nucleases to improve editing efficiency and accuracy (Renaud et al., 2016, Cell Reports 14, 2263-2272 March 8, 2016). One or more of the following proteins or ribonucleoproteins may be added along with editing oligonucleotides (also *see* Table VIII and Table IX) programmable nucleases including zinc finger nucleases (Carroll D. Genetics 188:773e82. (2011)), TALENs, mega TALENs, other homing endonucleases, CRISPR-Cas9 (Jinek et al. Elife 2013;2:e00471), or homologous or similarly acting ribonucleoproteins (*i*.*e*. Cpf1, C2C1 or C2C3) including mutated forms thereof that have been selected to increase target specificity by reducing DNA binding affinity (eSpCas9, Slaymaker et al. Rationally Engineered Cas9 Nucleases with Improved Specificity, Science (2015)), and mutated forms that have been selected to tolerate more DNA substitutions in the crRNA region so that the "crRNA" can act as a donor DNA for editing, and mutated forms in which the Cas9 nuclease is inactivated, Argonauts (also spelled Argonautes, such as *Natronobacterium gregoryi* Argonaute ((Gao et al. Nat Biotech. 2016; advance online publication doi:10.1038/nbt.3547) and related argonauts that use a DNA guide, and mutated forms of such Argonauts that lack DNA cleaving activity ("dead Argonauts"). When employing dead Argonauts, the editing will be achieved by using a guide DNA that is an editing oligonucleotide, with desired edited sequence, or linked to a nucleobase modifying activity for editing. An advantage of using dead DNA guided Argonautes is that the DNA guide can be made corresponding to the desired edited sequence, and thus the DNA guide can accomplish editing without the need for target DNA cleavage and the need for a separate editing oligonucleotide ("donor DNA"). Alternatively, when using Argonautes with RNA or DNA guides, the guides can be editing oligonucleotides perfectly matched to the target DNA, with the editing oligonucleotide being linked to a target nucleobase modifying activity to achieve editing (*see* Table VII).

**TABLE IX**

| Forms of CRISPR/Cas9 Variants | | |
|---|---|---|
| **CRISPR/Cas9 and useful variants** | **Description** | **Benefit(s)** |
| SpCas9 | The original wild type nuclease isolated from *Streptococcus pyogenes* | The most widely used CRISPR nuclease |
| **(*Streptococcus pyogenes* Cas9)** | | |
| SaCas9 | About 25% smaller in size than SpCas9 | Due to its smaller size, it can be packed into adeno viral vectors suitable for *in vivo* delivery systems |
| **(*Staphylococcus aureus* Cas9)** | | |
| Cas9n | Mutation in one of the two nuclease domains in the wild type Cas9 (SpCa9 or SaCas 9) | Useful for making a nick on one strand of the target DNA |
| **(Cas9 nickase)** | | |
| dCas9 (dead cas9) | Mutation in both nuclease domains of Cas9 (SpCa9 or SaCas 9) | Useful for transcriptional regulation and epigenetic research applications |
| eSpCas9 (High efficiency SpCas9) | Mutations of certain amino acid residues that bind to the non-target strand of the DNA. | These mutants are shown to be having undetectable off-target cleavage while having high on target cleavage efficiency |
| **or SpCas9-HF1 (SpCas9 High Fidelity1)** | | |
| Cpf1 | A recently identified CRISPR nuclease that possess distinct properties compared to Cas9 | 'T' rich PAM |
| **(CRISPR from *Prevotella* and *Francisella* 1)** | | |
| | | PAM is upstream of the target sequence |
| | | Single RNA guided (only crRNA is sufficient; tracer RNA is not needed) |
| | | Creates a staggered DNA double- stranded |
| | | break with a 4 or 5-nt 5' overhang. |
| Re-engineered Cas-9 to accept DNA EDITING OLIGONUCLEOTIDES as a crRNA | | |
| CRISPR or CRISPR variant with the crRNA extended with DNA nucleotides that act as the donor DNA (editing oligonucleotide), with the extension being contiguously complementary to target, or complementary to a nearby target edit. | | |

The proteins the promote genome editing can be manufactured separately from the editing oligonucleotide, purified then pre-complexed with the editing oligonucleotide(s) (Kim et al. Genome Res. 24:1012e9 (2014)), or the proteins may be expressed in the target cells/tissues. Expression of the exogenous proteins in cells or tissues can be done with methods known in the art, including gene therapy vectors, naked DNA transfection, or mRNA transfection. When employing Cas9, a particularly useful embodiment employs a Cas9 with both nuclease domains inactivated by mutations known in the art (known as dead Ca9 or dCas9). The crRNA is preferably separate from the tracRNA and has the desired edited sequence. The crRNA also may have one and up to about fifteen DNA linkages substituted in and optionally around the editing site, as defined herein (the following reference shows DNA substitutions can be made in the guide region of tracrRNA: Zachary Kartje et al. Abstract 12th Annual Meeting of the Oligonucleotide Therapeutics Society. September, 2016, https://custom.cvent.com/F89D960A94384DDB8049882DD4DFBD4E/files/ cdb733770e9e4c14ac0a51b7386a9462.pdf). In this way, the specificity and non-chromosomal cutting advantages of the Brachman Kmiec-type genome editing or the editing employing oligonucleotides containing chemically reactive groups that modify the target nucleobase to change its coding as described herein will be enhanced in efficacy by the enhanced target hybrid formation driven by nuclease inactivated Cas9 (dCas9). In another
embodiment, the about 18 nucleotide guide RNA portion of the crRNA or tracRNA is extended to the 5' or 3' direction, with an editing oligonucleotide. The editing oligonucleotide may be unmodified or have various modifications described herein. The editing oligonucleotide would be attached to the crRNA or tracRNA guide covalently by a phosphodiester (or phosphodiester analog) bond, or chemical linker, or non-covalently through base-pairing to a portion of the CRISPR guide RNA, or an extension of the CRISPR guide RNA. In a particularly useful embodiment, the editing oligonucleotide portion would hybridize contiguously to the sequence complimentary to the guide portion of the tracRNA or crRNA, extending the duplex with the target DNA into the region of the targeted mutation. This approach would be more efficient than oligonucleotide-directed genome approaches that do not use CRISPR-Cas9, because Cas-9/CRISPR enhances the efficiency of strand-invasion. This approach would be more selective and simple than common Cas-9/CRISPR approaches which cleave the targeted chromosome, and require a separate donor oligo. A critical distinction between our editing approaches described above, and the common methods of using CRISPR/Cas9 to obtain precise edits, is that the common methods of using CRISPR/Cas9 comprise a guide portion (strand invading portion) of the gRNA or crRNA that is completely complimentary to the target DNA strand, while our approach herein comprise a guide portion of the crRNA or gRNA that has the desired edited sequence (*e*.*g*. the wildtype sequence, when the desired edit is a change from a point mutation to wildtype).

The current editing approach also has a region in the crRNA or gRNA guide segment that binds to the mutation and contains DNA substitutions in and optionally around the editing site that can act as the donor DNA (by acting as a template for repair or by HR) for the editing.

In another embodiment, the crRNA (separate from the tracrRNA, or as a segment of the gRNA) is associated with a nucleobase modifying moiety, and thus the crRNA serves as an editing oligonucleotide of the current invention and of our previous filing PCT/US15/65348 and recent work demonstrating this invention (Komor et al. Nature, 2016 Apr 20. doi: 10.1038/nature17946, Epub ahead of print).

### J. Small Molecules that Enhance Editing

Small molecules can also enhance editing frequencies. Addition of small molecules is much less cumbersome than addition of programmable nucleases (see Table X Non-Catalytic Agents that may be combined with the Editing Oligonucleotides to Enhance Editing Efficiency). In each case, editing efficiency can be optionally enhanced by treatment of the targeted cell or organism
with drugs that synchronize cells in S-phase (such as aphidicolin) during or prior to the exposure to the editing oligonucleotide, slow the replication forks (Erin E. Brachman and Eric B. Kmiec DNA Repair 4:445-457, 2005), or otherwise increase the expression and/or activity of the homologous DNA repair machinery, such as hydroxyl urea, HDAC inhibitors or Camptothecin (Ferrara and Kmiec Nucleic Acids Research, 32(17):5239-5248, 2004) (see Table X Non-Catalytic Agents that may be Combined with the Editing Oligonucleotides to Enhance Editing Efficiency).

**TABLE X**

| Non-Catalytic Agents that may be Combined with the Editing Oligonucleotides to Enhance Editing Efficiency | |
|---|---|
| Agents that can optionally be combined (non-covalently linked or covalently linked) with the oligonucleotides of the present invention to enhance editing efficiency, or can be administered with the editing oligonucleotides or can be separately administered at the time or near the time of editing oligonucleotide administration (*e*.*g*. within 24 hours) | |
| Small Molecule enhancers of editing | |
| A. | Agents, such as aphidicolin, that block cell division, that when removed lead to a burst of cell division, followed by treatment with editing oligonucleotides and optional helper oligonucleotides when the synchronized cells reach S Phase (*e*.*g*. Engstrom and Kmiec Cell Cycle (2008) 7:10, 1402-141) |
| B. | Agents that slow replication forks, allowing the editing oligonucleotide more time to hybridize to the exposed single stranded target genomic DNA (*e*.*g*. ddC (2',3'-dideoxycytidine or thymidine ((*e*.*g*. Rios, X. et al. Stable Gene Targeting in Human Cells Using Single-Strand Oligonucleotides with Modified Bases. PLoS ONE 7, e36697, doi:10.1371/journal.pone.0036697 (2012)). |
| C. | Agents that open up chromosomal structure making the target DNA more accessible to the editing oligonucleotide, such as HDAC inhibitors (*e*.*g*. US Publication number US20070072815 A1 Application number US 11/120,810) |
| Pretreatment or combination therapy with agents that stimulate proliferation of target cells. | |
| | A.Growth factors (*e*.*g*. Erythropoietin, EGF or hepatocyte growth factor) |
| B. | Cytokines |

Another method for enhancing the efficiency of homologous recombination of a chemically modified "donor" editing oligonucleotide is the addition of a PNA-clamp near the target mutation (Schleifman et al., Chem. Biol. 18(9):1189-1198, 2011). While Glazer has employed this technique with 2^{nd} generation editing chemistries (*e*.*g*. three phosphorothioate modification on one of both ends of the donor DNA oligonucleotide), the PNA-clamps have not been employed with the 3^{rd} generation more heavily modified donor DNA described and referenced herein. (Bahal et al. Current Gene Therapy 14(5):331-42, 2014, Chin et al. PNAS 105 (36):13514-13519, 2008, Rogers et al. PNAS 99 (26):16695-16700, 2002 and U.S. Patent 8,309,356). Another embodiment of the present invention, combines internally modified editing oligonucleotides (not just modifications at or near to the termini) described herein with PNA helper oligonucleotides (including PNA clamps, tail clamps and strand invading PNAs).

### K. Synthesis

Teachings regarding the synthesis of particular oligonucleotides to be utilized as editing oligonucleotides of the present invention may be found in art, including in PCT/US2015/65348 and the citations within (PCT/US2015/65348).

Phosphodiester or phosphodiester analogue editing oligonucleotides can be conjugated to PNAs (*e*.*g*., PNA helper oligonucleotides, or PNAs that will form segments of the editing oligonucleotide) by methods known in the art (*e.g*., Rogers et al. PNAS 99(26):16695-700, 2002).

### L. Internucleosidyl Linkages

Particularly useful modified internucleoside linkages or backbones are described herein (*see* Table II and (PCT/US2015/65348) . Various salts, mixed salts and free-acid forms are also included.

### M. Nucleoside Mimetics

In other particularly useful oligonucleotide mimetics, both the sugar and the internucleoside linkage, *i.e.,* the backbone, of the nucleoside units are replaced with novel groups. The nucleobase units are maintained for hybridization with an appropriate nucleic acid target compound. One such oligonucleotide, an oligonucleotide mimetic, that has been shown to have excellent hybridization properties, is referred to as a peptide nucleic acid (PNA). In PNA compounds, the sugar-backbone of an oligonucleotide is replaced with an amide-containing backbone, in particular an aminoethylglycine backbone. The nucleobases are retained and are bound directly or indirectly to atoms of the amide portion of the backbone. Representative United States patents that teach the preparation of PNA compounds include, but are not limited to, U.S. patent nos. 5,539,082, 5,714,331 and 5,719,262. Further teaching of PNA compounds can be found in Nielsen et al., Science, 254:1497, 1991.

Some particularly useful embodiments of the invention employ oligonucleotides with phosphorothioate linkages and oligonucleosides with heteroatom backbones, and in particular - CH₂-NH-0-CH₂-, -CH₂-N(CH₃)-O-CH₂- (known as a methylene (methylimino) or MMI backbone), -CH₂-O-N(CH₃)-CH₂-, -CH₂-N(CH₃)-N(CH₃)-CH₂-, and -O-N(CH₃)-CH₂-CH₂- (wherein the native phosphodiester backbone is represented as -O-P-O-CH₂-) of the above referenced U.S. Patent no. 5,489,677 and the amide backbones of the above referenced U.S. patent no. 5,602,240. Also particularly useful are oligonucleotides having morpholino backbone structures of the above-referenced U.S. patent no. 5,034,506.

### N. Nucleobase Modifications

The oligonucleotides employed in the editing oligonucleotides of the invention may additionally or alternatively comprise nucleobase modifications or substitutions. As used herein, "unmodified" or "natural" nucleobases include the purine bases adenine (A) and guanine (G), and the pyrimidine bases thymine (T), cytosine (C), and uracil (U). Modified nucleobases include other synthetic and natural nucleobases (see PCT/US2015/65348 for nucleobase modifications and synthesis) (*see* also Table III, Useful Nucleobases Modifications for Oligonucleotides).

### O. Complementarity

An editing oligonucleotide and a target nucleic acid are complementary to each other when a sufficient number of nucleobases of the oligonucleotide can hydrogen bond with the corresponding nucleobases of the target nucleic acid, such that a desired effect will occur (*e*.*g*., permitting the desired base modification to occur following hybridization).

Non-complementary nucleobases between an editing oligonucleotide and a target nucleic acid may be tolerated provided that the editing oligonucleotide remains able to specifically hybridize to the target nucleic acid. In certain embodiments, the oligonucleotides provided herein, or a specified portion thereof, are, or are at least, 70%, 80%, 85%, 86%, 87%, 88%, 89%, 90%, 91%, 92%, 93%, 94%, 95%, 96%, 97%, 98%, 99%, or 100% complementary to a target nucleic acid, a target region, target segment, or specified portion thereof (*see* Table III, Useful Nucleobases Modifications for Oligonucleotides, for a list of possible nucleobase modifications. In certain embodiments, the editing oligonucleotide provided herein, or a specified portion thereof, are, or are at least, 70%, 80%, 85%, 86%, 87%, 88%, 89%, 90%, 91%, 92%, 93%, 94%, 95%, 96%, 97%, 98%, or 99% complementary to a target nucleic acid or specified portion thereof. Percent complementarity of an editing oligonucleotide with a target nucleic acid can be determined using routine methods. For example, an editing oligonucleotide in which 16 of 20 nucleobases are complementary to a target nucleic acid, and would therefore specifically hybridize, would represent 80% complementarity. In this example, the remaining noncomplementary nucleobases may be clustered or interspersed with complementary nucleobases and need not be
contiguous to each other or to complementary nucleobases. They may be at the 5' end, 3' end or at an internal position of the editing oligonucleotide. In another example, an editing oligonucleotide which is 18 nucleobases in length having 1 (one) non-complementary nucleobase, which is flanked by two oligonucleotides of complete complementarity with the target nucleic acid would have 94.4% overall complementarity with the target nucleic acid and would thus fall within the scope of the present invention.

Percent complementarity of an editing oligonucleotide with a region of a target nucleic acid can be determined routinely using BLAST programs (basic local alignment search tools) and PowerBLAST programs known in the art (Altschul et al., J. Mol. Biol., 215:403 410, 1990; Zhang and Madden, Genome Res., 7:649-656, 1997). The Gap program (Wisconsin Sequence Analysis Package, Version 8 for Unix, Genetics Computer Group, University Research Park, Madison WI), using default settings, utilizing the algorithm of Smith and Waterman (Adv. Appl. Math., 2:482-489, 1981) and the like may also be used.

### II. Modes of Action

### A. Hybridization

Hybridization between an editing oligonucleotide and a target nucleic acid may occur under varying stringent conditions, are sequence-dependent and are determined by the nature and composition of the nucleic acid molecules to be hybridized. The
most common mechanism of hybridization involves hydrogen bonding (*e.g.,* Watson-Crick, Hoogsteen or reversed Hoogsteen hydrogen bonding) between complementary nucleobases of the nucleic acid molecules.

Methods of determining whether a sequence is specifically hybridizable to a target nucleic acid are well known in the art. In certain embodiments, the editing oligonucleotides provided herein are specifically hybridizable with a target nucleic acid.

### B. Target Binding

The editing oligonucleotides of the present invention are designed to target DNA or RNA. The editing nucleotide(s) may be flanked on one or both sides with oligonucleotides that are completely complementary or substantially complementary to the target nucleic acid.

A particularly useful method of binding is by strand displacement resulting in hybridization to either the Watson or Crick strand of the DNA, or when RNA is the target by hybridization of the antisense editing oligonucleotide to the sense RNA strand.

### C. Editing oligonucleotide

The editing oligonucleotide may optionally contain a "linker" that covalently attaches a delivery moiety to the oligonucleotide. The linker attachment may be to any nucleobase in the editing oligonucleotide, to the 5' terminus, to the 3' terminus, to a sugar residue, or to the backbone. The linker may be any linker known to those skilled in the art for use in performing this task. (*see* Table V, Useful Linkers for Editing and Helper Oligonucleotides for these and additional linkers). The linker lengths may range from 1 carbon to about 20 carbons or equivalent length of other chemistries, but preferably below 10 carbons or 10 carbon equivalent length.

### D. Editing by Chemical Modification of the Targeted Nucleobase

Figure 1 shows a mechanism of editing utilizing the editing oligonucleotide of the present invention for the chemical modification mode of editing. The editing oligonucleotide can be various lengths as described herein.

The editing oligonucleotide used in the chemical modification method of editing comprises at least three components that include the "guide oligonucleotide", the "linker" or non-covalent connection that attaches the "sequence modifying moiety" to the guide oligonucleotide. Methods of synthesis, methods of use, examples and compositions of editing oligonucleotides that act by the nucleobase chemical modification mode are described in PCT/US2015/65348. In Figure 1, a linker is shown attached to a nucleobase of the editing oligonucleotide. However, the attachment may be to any nucleobase in the editing oligonucleotide, to the 5' terminus, to the 3' terminus, to a sugar residue, or to the backbone. The linker may be any linker known to those skilled in the art for use in performing this task. The linker as described herein can include a non-covalent linkage to the "sequence modifying moiety" (Montiel et al., PNAS 110(45):18285-90, 2013. Woolf, et al., PNAS 92:8298-8302, 1995.) Alternatively, a linker may be utilized and tested to determine its performance in the editing oligonucleotide. For example, linkers that may be utilized
with the present invention include those in Table V. The linker lengths may range from 1 carbon to about 20 carbons or equivalent length of other chemistries, but preferably below 10 carbons or 10 carbon equivalent length). As an alternative to a chemical covalent linker, a non-covalent connection between the editing oligonucleotide and the sequence modifying moiety can be made (for examples, see (Montiel et al., PNAS 110(45):18285-90, 2013, Woolf, et al., PNAS 92:8298-8302, 1995, and Woolf, Nat. Biotech 16:341-344, 1998).

Successful treatment with the editing oligonucleotide in the chemical modification mode results in some proportion of the "target nucleic acid" becoming modified. In Figure 1 the "chemical modification" (triangle) represents an addition of a chemical moiety (*e*.*g*. a methyl group), but the modification as described herein can be one of a variety of additions or removals of chemical groups from the targeted nucleobase of the target nucleic acid sequence (*e*.*g*. deamination).

See Table VII, Sequence Modifying Moieties for Nucleobase Chemical Modification and PCT/US2015/65348 for chemical modifications that can result in an edit.

### E. Chemistries

*See* Table VII and PCT/US2015/65348 for chemical reactions that can lead to an edit by nucleobase modification.

### F. Editing Action

The present invention provides editing oligonucleotides that can reduce or eliminate the effects resulting from a variety of mutations. The editing can be reversed, if desired, by administering an editing oligonucleotide that changes the edit back to the original sequence using the methods and compositions herein. The potential for ready reversal of edits is an important option that enhances the safety of genome editing for therapeutic applications.

In one embodiment of the present invention a common mutated sequence causing Cystic Fibrosis in Western populations, deltaF508 may be corrected. The repair of a deletion mutation like deltaF508 could be achieved by inserting back the deleted 3 nucleotides with the editing oligonucleotide. McNeer et al., (Nature Comm. DOI:10.1038/ncomms 7952 pgs. 1-11, 2015) provides an example with editing oligonucleotides with three phosphorothioate modifications on each end. Oligonucleotides with the improved chemical modification patterns and configurations of editing oligonucleotides of the present invention targeting the same region can be substituted for the editing oligonucleotides with three phosphorothioate modifications on each end similar to that used by McNeer *et al.* However, single base transitions or transversions may be more efficiently achieved with editing,
compared to insertions, therefore a change from R 553 to M (R553M) in the CF protein coding sequence which suppresses the deleterious effects of the deltaF508 mutation is an alternative approach to correcting the phenotypic effect of this mutation (Liu et al. Biochemistry 51(25):5113-5124, 2012. doi:10.1021/bi300018e.

Another change in the CF protein coding sequence, from R 555 to K (R555K), suppresses the deleterious effects of the deltaF508 mutation (Liu *et al. supra*)*.*

Other non-limiting examples of common CFTR mutations that can be corrected by the methods and compositions herein, include: M470V, W1282X, G542X, Y122X and 3849+10Kb C->T.

Another aspect of the present invention includes administering an editing oligonucleotide to an individual in order to create an allele sequence in their DNA or RNA that is protective for one or more diseases (see and PCT/US2015/65348 for examples).

### III. Treatments

### A. Diseases

See figure 22 of (PCT/US2015/65348) for some target diseases, indications and edit classes for treating such diseases and indications by the compositions and methods of this invention.

To the extent not listed in figure 22 of (PCT/US2015/65348), target indications, genes and editing oligonucleotide sequences comprising editing oligonucleotide sequences described in U.S. patents 7,258,854, 7,226,785 and U.S. Patent applications 20150118311 and 20150232881

Non-limiting examples of editing oligonucleotides which target genes associated with representative diseases and disorders are in Figure.

Exemplary (non-limiting) listing of editing oligonucleotides targeting genes associated with representative diseases and disorders are shown in Figure 24 of (PCT/US2015/65348).

### B. Pharmaceutical Compositions

The pharmaceutical compositions of this invention are administered in dosages sufficient to effect the expression of the target gene. In general, a suitable dose of editing oligonucleotide will be in the range of 0.01 to 5.0 milligrams per kilogram body weight of the recipient per day, or up to 50 milligrams per kilogram if necessary, preferably in the range of 0.1 to 200 micrograms per kilogram body weight per day, more preferably in the range of 0.1 to 100 micrograms per kilogram body weight per day, even more preferably in the range of 1.0 to 50 micrograms per kilogram body weight per day, and most preferably in the range of 1.0 to 25 micrograms per kilogram body weight per day. The pharmaceutical composition may be administered once daily, or the editing oligonucleotide may be administered as two, three, four, five, six or more sub-doses at appropriate intervals throughout the day or even using continuous infusion. In that case, the editing oligonucleotide contained in each sub-dose must be correspondingly smaller in order to achieve the total daily dosage. The dosage unit can also be compounded for delivery over several days, *e.g.,* using a conventional sustained release formulation which provides sustained release of the editing oligonucleotide over a several day period. Sustained release formulations are well known in the art. In this embodiment, the dosage unit contains a corresponding multiple of the daily dose.

### i. Dosages

Certain factors may influence the dosage and timing required to effectively treat a subject, including but not limited to the severity of the disease or disorder, previous treatments, the general health and/or age of the subject, and other diseases present. Moreover, treatment of a subject with a therapeutically effective amount of a composition can include a single treatment or a series of treatments. Editing with programmable nucleases
has heretofore been designed with one or a few treatments, because of immune responses to programmable nucleases and vector proteins, and because cleavage by programmable nucleases destroys a large percentage of target sequences by causing random insertions and deletions. The reduced immunogenicity of editing oligonucleotides described herein and the precision of editing (low or no random insertions and deletions) allows for multiple dosing, of up to 3, up to 20, up to 50 or up to 100 doses or more. Multiple dosing has safety advantages, as a patient can be monitored as the editing progresses over time. Also, replicating cells are more amenable to genome editing, so multiple doses allows for longer treatment spans to edit cells when the cells are dividing. Estimates of effective dosages and *in vivo* half-lives for the individual editing oligonucleotide encompassed by the invention can be made using conventional methodologies or on the basis of *in vivo* testing using an appropriate animal model, as described elsewhere herein.

### ii. Routes of Administration

The pharmaceutical compositions encompassed by the invention may be administered by any means known in the art (see and PCT/US2015/65348 for non-limiting examples)

The pharmaceutical compositions useful according to the invention also include encapsulated formulations to protect the editing oligonucleotide against rapid elimination from the body, such as a controlled release formulation, including implants and microencapsulated delivery systems (see PCT/US2015/65348 for non-limiting examples). These can be prepared according to methods known to those skilled in the art, for example, as described in U.S. patent no. 4,522,811; PCT application no. WO 91/06309; and European patent publication EP-A-43075 or obtained commercially from Northern Lipids (Burnaby, British Columbia), Avanti Polar Lipids (Alabaster, Alabama) or Arbutus BioPharma (Burnaby, British Columbia). Nanoparticle delivery may also be used and an example is described in Zhou et al. Pharmaceuticals, 6:85-107, 2013; doi:10.3390/ph6010085, McNeer et al., Gene Ther. 20(6):658-669, 2013; doi:10.1038/gt.2012.82, McNeer et al., Nature Comm. DOI:10.1038/ncomms 7952 pgs. 1-11, 2015 and Yuen Y.C. et al. Pharmaceuticals, 5:498-507, 2013; doi:10.3390/pharmaceutics5030498. Oligonucleotides of the present invention may also be encapsulated in Invivofectamine 3.0 as described by the manufacturer (Thermo Fisher, Waltham, MA) or in LUNAR nanoparticles, as described by the manufacturer (Arcturus, San Diego, California, US Patent Application number 20150141678). For cell culture use, Lipofectamine 2000 is particularly useful for delivering editing oligonucleotides, as it works in the presence of serum and allows oligonucleotides to be delivered over a period of many hours or days while the cells are replicating (Thermo Fisher, Waltham, MA).

### iii. Toxicity and Efficacy

Toxicity and therapeutic efficacy of such compounds can be determined by standard pharmaceutical procedures in cell cultures or experimental animals (see and PCT/US2015/65348.

### iv. Compositions and Methods

The oligonucleotides of the invention are provided in the following compositions and methods.

In one aspect, provided herein is a method of modifying a nucleic acid sequence within an isolated cell or cells within an organism comprising the step of introducing the oligonucleotide into the cells such that a modification or modifications of the complementary cellular nucleic acid results, wherein the modification creates an allele that protects against disease, repairs a mutation, or inactivates a gene.

In another embodiment, the allele that protects against disease does not inactivate the function of the targeted gene, but does modulate the function of the targeted gene.

In yet another embodiment, the method results in the modulation of the function of the targeted gene. The modulation of the function of the targeted gene may increase the activity or expression the gene product. The modulation of the function of the targeted gene may partially decrease the activity or expression of the gene product.

The modulation of the function of the targeted gene may partially decrease the activity or expression of the gene product by not more than 50 percent in a modified cell. The modulation of the function of the targeted gene may partially decrease the activity or expression of the gene product by not more than 75 percent in a modified cell. The modulation of the function of the targeted gene may partially decrease the activity or expression of the gene product by not more than 90 percent in a modified cell.

In certain embodiments of the method, the targeted gene product is a protein post-translationally modified by protease cleavage. In a particular embodiment, the targeted gene protein is APP and the modification of the gene changes the sequence of APP to make it less susceptible to cleavage by the beta-secretase. The sequence encoding position 673 in APP may be changed from Alanine to Threonine.

Another aspect provided herein, are compositions comprising oligonucleotides of the invention. The compositions may be used in the methods described herein. In some embodiments, the oligonucleotide is present in a formulation (*e*.*g*., an editing oligonucleotide formulation). In one embodiment, the editing oligonucleotide formulation comprises an exogenous protein or ribonucleoprotein (or nucleic acids that express said protein or ribonucleoprotein) that increase the editing efficiency. The exogenous protein or ribonucleoprotein that increases the editing efficiency may be a programmable nuclease. The exogenous protein or ribonucleoprotein that increases the editing efficiency may be a CRISPR-Cas9, Zinc Finger, or Talen programmable nuclease. The editing oligonucleotide may be a single-stranded unmodified DNA.

The editing oligonucleotide may be single-stranded and contain at least 10 deoxyribose sugars. The editing oligonucleotide may be chemically modified. In one embodiment, the editing oligonucleotide is bound (non-covalently or covalently) by methods known in the art to the programmable nuclease (*e.g.,* a Talen or Cas-9) in order to increase the local concentration of editing oligonucleotide in proximity to the cleavage site, and thus increase the frequency of HR, compared to the frequency of indels.

Decreasing the rate of indels is useful when HR and precise editing is the desired outcome, as indels often destroy the function of the gene targeted for repair.

The chemical modifications of the editing oligonucleotide may include phosphorothioates. The chemical modifications of the editing oligonucleotide may include 3 phosphorothioate internucleotide linkages at each terminus. The chemical modifications of the editing oligonucleotide may include a total of 1-5 phosphorothioate internucleotide linkages at the termini.

The chemical modifications of the editing oligonucleotide may include a total of 7 or more phosphorothioate internucleotide linkages. In one embodiment, the chemical modifications of the editing oligonucleotide include a total of 7 or more phosphorothioate internucleotide linkages, but there remains at
least 10 internucleotide linkages that are not phosphorothioate modified. In one embodiment, the modifications do not contain any phosphorothioate modifications to reduce toxicity, which is particularly useful when using encapsulated editing oligonucleotides, because encapsulation protects the editing oligonucleotide from serum and endolysosomal nucleases. In one embodiment, the modifications include exonucleases end-blocking groups that are not phosphorothioates.

In certain embodiments, the compositions comprise oligonucleotides having chemically modified nucleobases. The chemically modified nucleobase(s) can be 5 methyl deoxycytidine. In some embodiments, there is 1 to about 500 5 methyl deoxycytidines. In other embodiments, there is 1 to about 50 5 methyl deoxycytidines. In other embodiments, there is 1 to about 10 5 methyl deoxycytidines. In other embodiments, there is 1 to about 5 5 methyl deoxycytidines. In other embodiments, there is 1 to about 5 5 methyl deoxycytidines. In other embodiments, there is one 5 methyl deoxycytidine. In other embodiments, one of the 5 methyl deoxycytidines is in a 5'CpG sequence hybridized to a mismatched 5'TG, and this modification directs the editing to the targeted strand. In a particular embodiment, this 5'TG target site is the TG of a methionine start codon, and the edit reduces or eliminates production of functional target protein. In other embodiments, modifications across from the targeted nucleotide can direct editing to the targeted strand with little or no sequence restrictions, said chemistries being in one embodiment 2'F, 2'-O-alkyl or LNA. In one more specific embodiment, the modifications at or near the editing site of the editing oligonucleotide that direct the editing to the targeted strand are on editing oligonucleotides without any phosphorothioates in order to reduce toxicity to a minimum (see figure 2 for examples). In other
embodiments, modified chemistries which direct editing to the targeted strand are combined with phosphorothioate linkages to further enhance nuclease stability and increase biodistribution in vivo (see figure 2 for examples). In some embodiments the editing oligonucleotide comprises >15 phosphorhothioates, and modifications to direct editing to the targeted strand at or near the editing site (see figure 2 for examples). In some embodiments the editing oligonucleotide is designed to repair a deletion, and chemical modifications that direct editing to the targeted strand are placed in the nucleotide directly 5' and 3' of the editing oligonucleotide sequence that is being inserted to correct the deletion (See ETAGEN Serial Numbers 100243-100245 in figure 2 for examples of this modification pattern which use LNA, 2'-O-methyl and 2'F respectively, which in this case targets the Cystic Fibrosis deltaF508 mutation). In some embodiments, the editing oligonucleotide with chemical modifications across from targeted nucleobase that direct editing to the targeted strand, are combined 5' phosphate modifications that also increase the editing efficiency.

In some embodiments, when low toxicity and high nuclease stability is desired, the editing oligonucleotide has a majority of linkages modified with 2' modifications, has no phosphorothioates, and has chemical modifications such as 2' -O-alkyl, 2'F, or LNAs across from the editing site that direct editing to the targeted strand (see figure 2 for examples). In some embodiments, the editing oligonucleotide described in the sentence above, also has self-delivering conjugates, which in one particularly useful embodiment comprises Gal-NAc.

In several particularly useful embodiments, the editing oligonucleotides in this Compositions and Methods section, are delivered to cells in vitro or in vivo in combination with a programmable nuclease from Table VIII.

In a particular embodiment of the methods and compositions described herein, the editing oligonucleotides comprise 2' sugar modifications. In another particular embodiment of the methods and compositions described herein, the editing oligonucleotides comprise only 2' modifications. In a particular embodiment, the editing oligonucleotides comprise 2'F 5' of editing site, and 2'- O-mt 3' of editing site, or both. In another particular embodiment, the editing oligonucleotides comprise modified bases that increase affinity near the editing site, wherein said modified bases are not 5 methyl C.

In other particular embodiments of the methods and compositions described herein, the oligonucleotides are encapsulated in delivery vehicles (see Yin et al. Nature Reviews, Genetics. 15:541-555, 2014 for a description of delivery vehicles for nucleic acids), the oligonucleotides used for editing include the helper oligonucleotides listed in Table XIV or comprise the sequence of one of the helper oligonucleotides listed in Table XIV.

The oligonucleotides contain 4 or more of the optional segments or 5 of these optional segments, or 6 of these optional segments, or all 7 of these optional segments; and the oligonucleotide acts primarily by one of the modes described in the following Table XI.

**TABLE XI**

| Types of Editing Achieved by Editing Oligonucleotides (Target Sequence Changes) | |
|---|---|
| **Type** | **Approaches** |
| Knockout | Create premature stop codon Missense mutation that fully or partially inactivates the protein's function |
| | Changing AUG start codon to a different codon |
| | Creating a splice mutation that disrupts production of functional target protein |
| Repair/correction of mutation | Change nucleotide |
| | Insertion or deletion |
| | Type of Repair |
| | -Exact correction |
| | -Exact amino acid, different DNA |
| | -Intragenic 2nd site-suppressor |
| | -Intergenic 2nd site-suppressor |
| | -Permanent mutant exon skipping by mutating splice site |
| Inserting a protective allele | Change Nucleotide |
| | -Change Nucleotide |
| Modulating expression levels | -Insertion or deletion |

In a particular embodiment of the methods and compositions described herein, the oligonucleotides further comprise a conjugated molecule that confers enhanced cell uptake.

In a particular embodiment of the methods and compositions described herein, the methods and compositions further comprise a helper oligonucleotide or helper oligonucleotides.

In a particular embodiment of the methods and compositions described here, the oligonucleotides of the invention have one or more improvement properties and advantages listed herein and/or cited in the Table XII.

**TABLE XII**

| Improved Properties Resulting from Chemical Modifications of Editing and Helper Oligonucleotides | |
|---|---|
| **Property** | **Advantage(s)** |
| Increased hybrid affinity | Increased potency through increased ability to invade duplex DNA target strands |
| Reduced recognition by TLR and other cellular nucleic sensors | Reduced toxicity from immune stimulation |
| Nuclease resistance | Increased potency, duration of editing and compatibility with delivery by delivery conjugates without encapsulation |
| | Enhanced serum half-life |
| | Less complex, less expensive and less toxic formulation |
| "self-delivering" | Better tissue penetration |

In a particular embodiment of the methods and compositions described herein, the oligonucleotides of the invention are delivered using the delivery vehicles listed herein and/or listed in the Table XIII.

**TABLE XIII**

| Non-Limiting Examples of Delivery Vehicles for Delivering Editing Oligonucleotides to Target Cells |
|---|
| Non-viral vectors for the delivery of nucleic acids known in the art, which are useful for delivering oligonucleotides of the present invention including those described and cited in Hao et al., Nature Review, Genetics 15:514-554, 2014. |
| PLGA and poly (beta amino ester) (PBAE), including derivatized PLGA/PBAE nanoparticles with MPG via a PEGylated phospholipid linker (DSPE-PEG2000) (McNeer, et al., Gene Ther. 20(6): 658-669, 2013. doi:10.1038/gt.2012.82, McNeer, et al., Nature Comm. DOI:10.1038/ncomms 7952:1-11, 2015, Saltzman U.S. patent application nos. 2011/0268810 and 2015/0118311) |
| Delivery vehicles described in U.S. patent application No. 20130225663 |
| Oligonucleotides of the present invention can also be encapsulated in LUNAR nanoparticles, as described by the manufacturer (Arcturus, San Diego, California, US Patent Application # 20150141678). |
| Dynamic Polar conjugates (Arrowhead Research, Wooddell et al. Molecular Therapy. 26 February 2013; doi: 10.1038/mt.2013.31) |
| Lipofectin and Lipofectamine 2000 and Invivofectamine 3.0 (Thermo Fisher Scientific, Waltham, Massachusetts). |
| SNALPS (Atbutus Biopharma (formerly Tekmira), Burnaby, British Columbia) |
| Liposomal suspensions (including liposomes targeted to infected cells with monoclonal antibodies to viral antigens. (U.S. patent no. 4,522,811; PCT application no. WO 91/06309; and European patent publication EP-A-43075. Liposomes can be obtained commercially from Northern Lipids (Burnaby, British |
| Columbia), Avanti Polar Lipids (Alabaster, Alabama) or Arbutus BioPharma (Burnaby, British Columbia). |

In one aspect, provided herein is an editing oligonucleotide, wherein the editing oligonucleotide can edit a complementary target sequence within a cell, and wherein the editing oligonucleotide comprises one type of the backbone modifications selected from the modifications listed in Table II, or two types of the modifications listed in Table II or 3 or more types of the modifications listed in Table II.

In one embodiment of the editing oligonucleotide, a backbone modification is neutral. The backbone modification can comprise 1 to about 20 neutral modifications. In a particular embodiment, the backbone modification comprises 2 to about 4 neutral modifications.

In another embodiment, a backbone modification is a methylphosphonate. The backbone modification may comprise 1 to about 20 methylphosphonates. In a particular embodiment, the backbone modification comprises 2-4 methylphosphonates. In a particular embodiment, the backbone modification comprises 2 methylphosphonates. In a more particular embodiment, the backbone modification comprises 2 methylphosphonates on the 5' termini.

In another embodiment, the editing oligonucleotide may comprise 1 to about 20 backbone modifications in a single modified backbone editing oligonucleotide. In one embodiment, at least two of the modifications are in a terminal segment. In a particular embodiment, the editing oligonucleotide comprises two modifications at the 5' termini.

In another aspect, provided herein is a method of using editing oligonucleotides as described herein to edit a gene in cell or organism. In one embodiment, the cell is an isolated human cell. In one embodiment, the organism is a human. In certain embodiments, the method is used to treat an indication selected from the indications listed in Figure 23 of (PCT/US2015/65348). In a particular embodiment, the indication is selected from the indications listed in Figure 24 of (PCT/US2015/65348).

In one embodiment, the gene is a target gene listed in Figure 23 of (PCT/US2015/65348). In a particular embodiment, the editing oligonucleotide comprises at least 25 percent of a sequence listed in Figure 24 of (PCT/US2015/65348). In another particular embodiment, the editing oligonucleotide comprises at least 51 percent of a sequence from Figure 24 of (PCT/US2015/65348).

In another aspect, provided herein is an editing oligonucleotide, wherein said editing oligonucleotide can edit a complementary target sequence within a cell, and wherein said editing oligonucleotide comprises one or more nucleobase modifications listed in Table III, Useful Nucleobase Modifications for Oligonucleotides. In one embodiment, the editing oligonucleotide comprises 1 to about 100 modified nucleobases from Table III. In one embodiment, the editing oligonucleotide comprises 1 to about 30 modified nucleobases from Table III. In one embodiment, the editing oligonucleotide comprises 1 to about 10 modified nucleobases from Table III. In a particular embodiment, the editing oligonucleotide comprises one or more modified nucleobases according to a modification pattern species in Table III.

In another embodiment of the editing oligonucleotide, the modified nucleobases decrease immune stimulation by editing oligonucleotide in mammals. In a particular embodiment, the modified nucleobase comprises a 5 methyl C chemical modification. In another particular embodiment, the nucleobase modification increases the affinity of the editing oligonucleotide for its complimentary target.

In another aspect, provided herein is an editing oligonucleotide, wherein said editing oligonucleotide can edit a complementary target sequence within a cell, and wherein the editing oligonucleotide comprises one or more sugar modifications listed in Table IV, Useful Sugars for Oligonucleotides. In one embodiment, the sugar modifications are selected from 2' sugar modifications.

A 2' sugar modification can be 2' F. A 2'sugar modification may be 2' O-methyl. The 2' sugar modifications can be a combination of 2' F and 2' O-methyl modifications.

In one embodiment of the editing oligonucleotide, the majority (e.g., greater than 50%) of the 2' F modifications are 3' of the editing site. In another embodiment of the editing oligonucleotide, the majority (*e*.*g*., greater than 50%) of the 2' O-methyl modifications are 5' of the editing site.

The 2' sugar modification can increase the affinity of oligonucleotide for its target nucleic acids. In one embodiment, the editing oligonucleotide comprises 1-75 sugar modifications. In another embodiment, the editing oligonucleotide comprises 2-30 sugar modifications. In another embodiment the editing oligonucleotide comprises 2-16 sugar modifications.

In one embodiment, the editing oligonucleotide comprises about 5-100% chemically modified bases. In another embodiment, the editing oligonucleotide comprises about 25-75% chemically modified bases. In another embodiment, the editing oligonucleotide comprises about 40-60% chemically modified bases. In a particular embodiment, the editing oligonucleotide comprises 2 modifications. In one particular embodiment, the editing oligonucleotide contains 2'F and 2'O-methyl modifications.

In one embodiment, the editing oligonucleotide targets a gene listed in Figure 23 of (PCT/US2015/65348).

In another aspect, provided herein is a method of treating a human disease by genome editing comprising the step of administering to a person in need of such treatment an editing oligonucleotide as described herein.

In yet another aspect, provided herein is an editing oligonucleotide that comprises one or more delivery conjugates. In a particular embodiment, the editing oligonucleotide comprises one delivery conjugate. The delivery conjugate can promote cellular uptake of the oligonucleotide. The delivery conjugate can enhance uptake of the oligonucleotide into cells in an organism. The delivery conjugate can be a chemical moiety that is either directly or indirectly covalently bonded to the editing oligonucleotide. Direct covalent bonding involves, for example, covalent bonding of the chemical moiety to the oligonucleotide. Indirect covalent bonding involves, for example, the use of a linker that is covalently bonded to both the oligonucleotide and the chemical moiety. In one embodiment, the editing oligonucleotide is not encapsulated by a delivery vehicle that enhances uptake in cells in an organism.

In one embodiment, the delivery conjugate is a ligand for a receptor. In a particularly useful embodiment, the ligand is one to ten Gal-Nacs. In another particular embodiment, the ligand is three Gal-Nacs. In one embodiment, the delivery conjugate is a lipophilic group. The lipophilic group may have about 10 to about 50 carbons. The lipophilic group may be a form of cholesterol.

Editing oligonucleotides comprising one or more delivery conjugates are useful for the treatment of disease. In one aspect, provided herein is a method of treating or preventing a human disease by administering to a patient in need of such treatment an editing oligonucleotide comprising one or more delivery conjugates, as described herein. In one embodiment, the method targets a gene for editing, wherein the targeted gene is listed in Figure 23 of (PCT/US2015/65348). In another embodiment, the targeted gene for the treatment is listed in Figure 24 of (PCT/US2015/65348). In one embodiment of the method, the editing oligonucleotide sequence comprises one of the sequences in Figure 24 of (PCT/US2015/65348).

Compositions and methods employing encapsulated editing oligonucleotides. One embodiment is an editing oligonucleotide that has eight or fewer sequence differences compared to the genomic DNA target sequence. Each of the sequence differences being a mismatch that would result in a transition or transversion, an insertion and/or a deletion of nucleotide(s) compared to the target sequence, whereby the editing oligonucleotide edits the target genomic DNA sequence to the sequence of the editing oligonucleotide, and whereby the resulting edits have a therapeutic benefit to an organism (or *ex vivo* treated cells)

treated by editing oligonucleotide, or has a desirable change useful for researching a targeted cell or organism. This editing oligonucleotide may further: have 1-4 exonuclease blocking group(s) at its 5' terminus and/or 3' terminus and/or; have fewer than 8 phosphorothioate modifications; be encapsulated in a nanoparticle; have one or more nucleobase chemical modifications that reduce immune stimulation; and/or have chemical modifications at or adjacent to the sequence differences with the targeted genomic DNA that block the endogenous mismatch repair machinery from editing the editing oligonucleotide.

In certain embodiments the editing oligonucleotide has six or fewer sequence differences, four or fewer sequence differences, two or fewer sequence differences or has one sequence difference compared to the genomic DNA target sequence. In any of the previous embodiments, an editing oligonucleotide in which the sequence differences compared to the genomic DNA target sequence are mismatches, is an editing oligonucleotide when interacting with the DNA target sequence that results in a transition(s) and/or transversion(s). Alternatively, an editing oligonucleotide, in which the editing oligonucleotide sequence differences compared to the genomic DNA target sequence, is an editing oligonucleotide when interacting with the DNA target that results in an insertion of nucleotides(s) in the targeted genomic DNA or a deletion of nucleotides(s) in the targeted genomic DNA.

The editing oligonucleotide may have: 1-4 exonuclease blocking group(s) at its 5' terminus and no exonuclease blocking groups at its 3' terminus; three exonuclease blocking group(s) at its 5' terminus and no exonuclease blocking groups at its 3' terminus; two exonuclease blocking group(s) at its 5' terminus and no exonuclease blocking groups at its 3' terminus; one exonuclease blocking group(s) at its 5' terminus and no exonuclease blocking groups at its 3' terminus; 1-4 exonuclease blocking group(s) at its 3' terminus and no exonuclease blocking groups at its 5' terminus; three exonuclease blocking group(s) at its 3' terminus and no exonuclease blocking groups at its 5' terminus; two exonuclease blocking group(s) at its 3' terminus and no exonuclease blocking groups at its 5' terminus; or one exonuclease blocking group(s) at its 3' terminus and no exonuclease blocking groups at its 5' terminus.

Further, the exonuclease blocking group(s) is: a phosphorothioate linkage; not a phosphorothioate linkage; a non-nucleotide linker; an amino linker; a C2 through C9 amino linker; a C3 amino linker; or a constrained nucleic acid. The exonuclease blocking group(s) may have a 2' sugar modification (including constrained nucleic acids). When the nuclease blocking group is a constrained nucleic acid it is: a 2',4'-BNA; a cET; or not a LNA;: or an LNA.

Editing oligonucleotide chemical modification(s) that block the endogenous mismatch repair machinery from repairing the editing oligonucleotide are: 2' sugar modification(s), including constrained nucleic acids; 2' sugar modification(s), including constrained nucleic acids, but excluding LNA(s); 2' sugar modification(s), but excluding 2'F; is LNA; or a 2'F sugar modification.

The editing oligonucleotide that is encapsulated may be administered: to an organism intravenously; ten or more times to achieve therapeutically relevant editing; or 20 or more times to achieve therapeutically relevant editing.

The editing oligonucleotides may be used to effect a therapeutic benefit to an organism (or *ex vivo* treated cells) treated by editing oligonucleotide or a human (or *ex vivo* treated human cells) treated by said editing oligonucleotide.

The editing oligonucleotide or method of using the editing oligonucleotide additionally has 2' sugar modifications between the termini or terminal exonuclease blocking groups and the modifications adjacent to the sequence differences with the targeted genomic DNA that block the endogenous mismatch repair machinery from repairing the editing oligonucleotide. The additional 2' sugar modifications are: both 5' and 3' to the editing site; only 3' to the editing site or only 5' to the editing site. In certain embodiments, the 2' sugar modifications 3' of the editing site are 2'F or the 2' sugar modifications 5' of the
editing site are 2'-O-methyl.

The editing oligonucleotide may further: have a 5' phosphate or nuclease stable analogue thereof; be combined with an additional oligonucleotide that binds the genomic DNA within 200 nucleotides of the editing site and enhances the editing efficiency of the editing oligonucleotide; or be combined with a PNA oligonucleotide that binds the genomic DNA within 200 nucleotides of the editing site and enhances the editing efficiency of the editing oligonucleotide.

The editing efficiency of the editing oligonucleotide utilized in the methods of the present invention may have an editing efficiency that is enhanced by cleaving within 200 nucleotides of the target site with a programmable nuclease.

The editing oligonucleotide and methods of using the editing oligonucleotides of the present invention may be utilized to treat diseases including beta thalassemia, cystic fibrosis or Duchenne muscular dystrophy, Alzheimer's disease, Type 2 diabetes, sickle-cell disease and beta-thalassemia.

The editing oligonucleotide may target the sense strand of the genomic DNA or the antisense strand of the genomic DNA.

Compositions and methods employing non-encapsulated editing oligonucleotides include editing oligonucleotides that have eight or fewer sequence differences compared to the genomic DNA target sequence, each of the sequence differences being a mismatch that would result in a transition or transversion, an insertion and/or a deletion of nucleotides compared to the target sequence, whereby the editing oligonucleotide edits the target genomic DNA sequence to the sequence of the editing oligonucleotide, and whereby the resulting edits have a therapeutic benefit to an organism (or ex *vivo* treated cells) treated by editing oligonucleotide, or has a desirable change useful for researching a targeted cell or organism. Furthermore, the editing oligonucleotide has: 1-4 exonuclease blocking group(s) at its 5' terminus and/or 3' terminus; is not encapsulated in a nanoparticle or other delivery vehicle; one or more nucleobase chemical modifications that reduce immune stimulation; and chemical modifications at or adjacent to the sequence differences with the targeted genomic DNA that block the endogenous mismatch repair machinery from editing the editing oligonucleotide.

The editing oligonucleotide in embodiment above has: six or fewer sequence differences compared to the genomic DNA target sequence; four or fewer sequence differences compared to the genomic DNA target sequence; two or fewer sequence differences compared to the genomic DNA target sequence; or has one sequence difference compared to the genomic DNA target sequence. The editing oligonucleotide sequence differences compared to the genomic DNA target sequence: by having mismatches that result in a transition(s) and/or transversion(s); that would result in an insertion of nucleotide(s) in the targeted genomic DNA; would result in a deletion of nucleotide(s) in the targeted genomic DNA.

The editing oligonucleotide may have: 1-4 exonuclease blocking group(s) at its 5' terminus and no exonuclease blocking groups at its 3' terminus; three exonuclease blocking group(s) at its 5' terminus and no exonuclease blocking groups at its 3' terminus; two exonuclease blocking group(s) at its 5' terminus and no exonuclease blocking groups at its 3' terminus; one exonuclease blocking group(s) at its 5' terminus and no exonuclease blocking groups at its 3' terminus; 1-4 exonuclease blocking group(s) at its 3' terminus and no exonuclease blocking groups at its 5' terminus; three exonuclease blocking group(s) at its 3' terminus and no exonuclease blocking groups at its 5' terminus; two exonuclease blocking group(s) at its 3' terminus and no exonuclease blocking groups at its 5' terminus; or one exonuclease blocking group(s) at its 3' terminus and no exonuclease blocking groups at its 5' terminus.

The exonuclease blocking group(s) may be: a phosphorothioate linkage; a non-phosphorothioate linkage; a non-nucleotide linker; an amino linker; a C2 through C9 amino linker; or a C3 amino linker. The exonuclease blocking group(s) may have a 2' sugar modification (including constrained nucleic acids) or a constrained nucleic acid. The constrained nucleic acid may be: a
2',4'-BNA: an LNA; a cET; or a non-LNA constrained nucleic acid.

The editing oligonucleotide in which the chemical modification(s) that blocks the endogenous mismatch repair machinery from repairing the editing oligonucleotide includes 2' sugar modification(s), and may further contain constrained nucleic acids but may exclude LNA(s) and/or 2'F. Alternatively the chemical modification(s) that blocks the endogenous mismatch repair machinery from repairing the editing oligonucleotide may be LNA or a 2'F sugar modification.

The editing oligonucleotide of the present invention may be administered to an organism by subcutaneous injection, intravenous injection or infusion, intravitreous injection or inhalation. The editing oligonucleotide may be administered ten or more times to achieve therapeutically relevant editing or 20 or more times to achieve therapeutically relevant editing. The resulting edits from the editing oligonucleotide may have a therapeutic benefit to an organism (or *ex vivo* treated cells) treated by editing oligonucleotide or to a human (or *ex vivo* treated human cells) treated by said editing oligonucleotide.

The methods and compositions of the present invention include editing oligonucleotides that additionally have 2' sugar modifications between the termini or terminal exonuclease blocking groups and the modifications adjacent to the sequence differences with the targeted genomic DNA that block the endogenous mismatch repair machinery from repairing the editing oligonucleotide. The additional 2' sugar modifications may be: at both 5' and 3' to the editing site; only 3' to the editing site; or only 5' to the editing site. In other embodiments, the 2' sugar modifications 3' of the editing site are 2'F or 5' of the editing site are 2'-O-methyl. The editing oligonucleotide may contain: a 5' phosphate or nuclease stable analogue thereof; phosphorothioate modifications at every internucleotide linkage; phosphorothioate modifications at >90% of internucleotide linkages; phosphorothioate modifications at >40% of internucleotide linkages; phosphorothioate modifications at 8 or more internucleotide linkages; or other endonuclease resistant modifications at 40% or more of the non-phosphorothioate linkages. The editing oligonucleotide may have: 2' sugar modified endonuclease resistant modifications at 40% or more of the non-phosphorothioate linkages; 2' -O-methyl modified endonuclease resistant modifications at 40% or more of the non-phosphorothioate linkages; or BNA modified endonuclease resistant modifications at 20% or more of the non-phosphorothioate linkages. In one embodiment the BNA modification is LNA or cET.

In other embodiments the editing oligonucleotide may be conjugated to: a ligand that promotes delivery to the targeted cell and/or tissue; a ligand that promotes delivery to the targeted cell and/or tissue, and said ligand comprises one or more Gal-Nac residues; a ligand that promotes delivery to the targeted cell and/or tissue, and said ligand comprises a lipophilic group; or a ligand that promotes delivery to the targeted cell and/or tissue, and said ligand comprises cholesterol or a cholesterol analogue.

Further the editing oligonucleotide may be combined with an additional oligonucleotide that binds the genomic DNA within 200 nucleotides of the editing site and enhances the editing efficiency of the editing oligonucleotide or a PNA oligonucleotide that binds the genomic DNA within 200 nucleotides of the editing site and enhances the editing efficiency of the editing oligonucleotide.

The editing oligonucleotide editing efficiency may be enhanced by cleaving within 200 nucleotides of the target site with a programmable nuclease. Diseases that may be treated with the editing oligonucleotide and methods of the present invention include beta thalassemia, cystic fibrosis or Duchenne muscular dystrophy, Alzheimer's disease, Type 2 diabetes, sickle-cell disease or beta-thalassemia.

The editing oligonucleotide may target the sense strand of the genomic DNA or the antisense strand of the genomic DNA.

### V. RESULTS

The oligonucleotide constructions and modification patterns presented in Figure 2 are useful for research, therapeutic and other applications described herein, even though their activity in cell culture may be less than the parent compound, because each of these oligonucleotides contributes to projected therapeutic benefits, such as reduced immune stimulation, higher nuclease stability, higher target specificity, reduced chemical toxicity and/or higher affinity compared to unmodified DNA or DNA with three phosphorothioates on each termini.

The examples of editing oligonucleotides listed in Figure 2 have various features that improve their usefulness in genome editing. Phosphorothioate linkages allow for serum protein binding to enhance serum half-life and tissue distribution, increased nuclease stability compared to end-blocks alone and cell uptake into cytoplasm without a delivery vehicle. More phosphorothioates lead to increased nuclease stability. Uniform (all) phosphorothioate substituted oligonucleotides are stable enough to efficiently be taken up without delivery vehicles and effectively survive transit through the high nuclease endo-lysosomal compartment. However, uniform phosphorothioate substitution also decreases editing efficiency one delivered to the interior of cells, so there is a tradeoff between the number of phosphorothioate bases and phosphodiester. Extensive
phosphorothioate substitution is more permissive to editing on the 3' half of the editing oligonucleotide up to the editing site, which is the rationale for the example oligonucleotides with phosphorothioate linkages on the 3' approximately half of the editing oligonucleotide. Another design to reduce phosphorothioate linkages places other modifications that are permissive to editing at the phosphodiester linkages, as exemplified in the designs with the 2'F phosphodiester segment toward the 3 portion of editing oligonucleotide. The phosphorothioate substitutions at or adjacent to the editing site, will also inhibit non-productive mismatch repair.

Five prime (5') phosphate is designed to enhance editing efficiency, particularly with short sequences (<30mer) and stable sequences, like all phosphorothioates (Radecke *et al.* 2006). 5' thiophosphate is designed to further stabilize against removal by cellular phosphatases. Shorter oligonucleotides have enhanced free uptake into cells, and they simplify and reduced the cost of synthesis, which is balanced against the enhanced editing efficiency of some longer oligonucleotides. 5 methyl C modifications enhance affinity to the target, reduce immune stimulation and direct editing to target strand. 5' non-phosphorothioate end-block such as a linker or linker with fluorescent conjugate (*e*.*g*. 6FAM) is less toxic than phosphorothioate end-blocks, and are permissive for editing. Four 3' phosphorothioate end-blocks that are not complimentary to the target DNA will be trimmed by cellular machinery, allowing for a natural 3' OH termini. Two to five non-complimentary 3' end-blocks are particularly useful.

Cholesterol conjugates enhance cell uptake, particularly when used on a highly nuclease stable oligonucleotide that can survive the endolysosomal nucleases. For liver uptake, Gal-NAc conjugates, known in the art to function in vivo with siRNA and antisense, can replace cholesterol as a delivery conjugate. A stretch of 3' unmodified nucleobases can be inserted at the termini next to a conjugation to allow cellular nucleases to cleave off the conjugate liberating free 3' OH which is required for most efficient editing.

Two prime (2') modifications (*i.e*. 2'F, LNA, 2'-O-methyl) at or adjacent to the "editing site" inhibit non-productive mismatch repair of the editing oligonucleotide to enhance editing efficiency, and also increase hybrid affinity (enhance efficacy), increase nuclease stability and reduce immune stimulation.

Three prime (3') F segments enhance editing efficiency (increase affinity) and reduce immune stimulation. LNA terminal modification enhances nuclease stability and increase hybrid affinity leading to higher editing efficiency and reduced immune stimulation, reducing toxicity. However, more than one LNA linkage at the termini can reduce editing efficiency.

The examples of helper PNA editing oligonucleotides listed herein in Figure 2 have various features to improve their usefulness in genome editing. PNA linkages impart nuclease stability and target high affinity. High target affinity promotes strand invasion. Lysines at termini enhance solubility and enhance strand invasion, and allow for "self-delivery" to cells in culture and in vivo without delivery vehicles. Gamma miniPEG or gamma glutamic acid PNA substitutions enhance solubility and target affinity. Glutamic acid imparts a negative charge which permits efficient encapsulation when used with positively charged delivery vehicles (see Figure 2 for examples).

Editing oligonucleotide 100034 has 5' and 3' 2'F arms (5' and 3' proximal segments), and demonstrates low but significant editing. This was unexpected because 2' F is sterically more similar to DNA than 2'-O-methyl, and 2' F was highly active in editing when incorporated in the 3' arm. In the 5' arm, 2'-O-methyl modification is better tolerated than the 2'F modification, which was again unexpected. This implies constructions like editing oligonucleotide 100058 are particularly useful over constructs with the same modification in each arm.

Extensive modifications as seen with editing oligonucleotide 100047 was compatible with editing, which is useful because each of the modifications lowers the projected toxicity relative to the parent oligonucleotide often used in the art (5' and 3' phosphorothioate DNA exonuclease blocking terminal segments are commonly used in the art). It is believed that part of this reduced toxicity is due to reduced activation of Toll-Like Receptors by 2' modified linkages, compared to 2' H in DNA or 2' OH in RNA. Higher target specificity is achieved because the arms do not serve as efficient editing sites, thus there are less potential off-target edits.

While 5' methylphosphonate exonuclease blocking terminal segments were quite active, using both 5' and 3' methyl phosphonate terminal segments were useful but less active. This construction removed all phosphorothioates that are associated with blocking cell proliferation in many *in vitro* assays.

A single 5 methyl C modification near the editing site was consistent with relatively high editing efficiency, as were multiple 5 methyl C modifications.

Extending the stretch of 3' proximal segment modifications towards the 3' editing segment may be less preferred due to interference with the editing reaction, but these additional modifications are projected to further increase nuclease stability and reduce immune stimulation (*e*.*g*. editing oligonucleotide 100062). This is also the case with the 5' modifications (e.g. editing oligonucleotide 100066).

Longer editing oligonucleotides have more linkages that may be modified at locations distant from the 5' or 3' editing segment, or the editing site (*e*.*g*. editing oligonucleotide 100072).

While methylphosphonates made an excellent 5' end-block. Editing oligonucleotide 100074 has a CY3 5' end-block and 3 complimentary phosphorothioate DNAs on the 3' end, and provides another useful combination of modifications.

Locked Nucleic Acids (LNAs) may also be employed as an end-blocking group, but they can add to *in vivo* toxicity. For this reason embodiments that employ Unlocked Nucleic Acids (UNAs) (*e*.*g*. editing oligonucleotide 100078), or simple linkers on one or both termini as nuclease end-blocks are particularly useful (see Table V). While exonucleases can jump over a single modification of DNA, this may be less of a problem in combination with 2'-modified terminal residues (e.g. editing oligonucleotide 100080). End-blocking linkers have the additional advantage that they can also be used to link conjugates to the editing oligonucleotide. These conjugates (*e*.*g*., conjugation with cholesterol (U.S. patent application no. 20130131142 A1) and Gal-Nac (U.S. patent no. 8,106,022) have been shown to increase uptake of oligonucleotides into cells in culture and *in vivo* in animals. Conjugates of these moieties with editing oligonucleotides can be prepared utilizing methods known in the art and will eliminate the need for delivery vehicles that add expense and/or toxicity (*e*.*g*., liposomes).

Editing oligonucleotide 100082 contains end-blocks that are complimentary to editing oligonucleotide 100005, 100031 and others oligonucleotides of this sequence. The editing oligonucleotide may be added to cells separately from a complimentary oligonucleotide, or may be pre-hybridized with a complimentary editing oligonucleotide of any modified chemistry described herein to form a duplex. The advantages of the pre-formed duplex, is that double- stranded DNA is resistant to single-stranded nucleases. However, a perceived disadvantage of the duplex may be that the bases are
not free to hybridize with the target DNA, unless some cellular repair/recombination machinery facilitates target binding. Depending on the target gene, cell type and route of administration single or double-stranded editing oligonucleotides may be more suitable for editing.

Editing oligonucleotide 100083 is an RNA protector oligonucleotide with end-blocks that are complimentary to editing oligonucleotides 100005, 100031 and others in the series of chemically modified editing oligonucleotides targeting GFP disclosed herein. This oligonucleotide protects the complimentary editing guide oligonucleotide from nucleases in serum, the endolysosomal pathway and the cytoplasm. When in the cytoplasm or nucleoplasm, the RNA strand will eventually be degraded by endogenous RNase H, liberating the single-stranded editing oligonucleotide for hybridization to the target DNA. This is an improvement upon 2'-O-methyl protecting oligonucleotides which reduced the activity of the editing oligonucleotide presumably due to interfering with hybridization to the target DNA.

Editing oligonucleotide 100085 has been designed so that the 5' proximal region, when hybridized to protector oligonucleotide 10086 forms a duplex capable of loading into the RNA-Induced Silencing Complex (RISC). The guide strands hybridization rate to complementary target nucleic acid (both RNA and DNA targets; Saloman, et al. Cell 162:84-96, 2015) is dramatically increased as a result of being loaded into Argonaut. This enhancement of the hybridization on-rate by RISC is what makes siRNA about 10-100 times more potent than the corresponding antisense (*e*.*g*., no enhancement by RISC observed with antisense). Thus, the 5' end of the editing oligonucleotide loaded into Argonaut will hybridize more rapidly to the target chromosomal DNA, increasing the potency and/or efficiency of genome editing. This mechanism uses the endogenous cellular machinery to enhance target binding, and therefore does not require the addition of exogenous proteins like Cas9 or *Natronobacterium gregoryi* Argonaute to accelerate enhance target binding. The advantage of this embodiment of the present invention is that it avoids the challenges of delivering exogenous proteins to cells. Once the binding to target DNA is seeded by the 5' proximal region of the editing oligonucleotide complexed with Argonaut, the remaining duplex will form rapidly.

Based on the data herein with editing oligonucleotide 100037, it the 5' end region of editing oligonucleotides can be modified with 2'-O-methyl RNA while maintaining editing efficiency, and partial modification of an oligonucleotide with 2'-0-methyl modification is compatible with the requirements for RISC loading (U.S. patent application nos. 20130317080, 20150267200, 20150105545, 20110039914 and U.S. Patent application serial no. 12/824,011). The protector oligonucleotide (passenger strand) is preferably 10-50 nucleotides, more preferably 12-30 nucleotides, and most preferably 19-27 nucleotides and completely or substantially complementary to the target. In this construction the editing oligonucleotide is designed following some generally accepted design rules for preparing RNAi or microRNAs (miRNAs).

For example, a two base pair 3' overhang of the passenger strand is particularly useful, but blunt and other end structures compatible with RNAi are also useful. A free 5' hydroxyl or a phosphorylated 5' hydroxyl on the guide (editing strand) is also provided. A range of chemical modifications and structures that are compatible with RNAi may be employed in this editing strategy.

It is particularly useful that the 5' end of the editing oligonucleotide duplexed with the passenger RNA does not have more than about 4 DNA linkages bound to RNA in the passenger strand, which can activate RNase H cleavage of the passenger strand, reducing RISC loading. In a particularly useful embodiment, when employing a RISC loading double-stranded region within the editing oligonucleotide that is long enough to serve as a dicer substrate, chemical modifications or mismatches may be inserted in a manner known in the art to reduce or eliminate dicer cleavage, such as incorporating 2'-O-methyl modification(s) at the dicer cleavage site(s) (Salomon et al. Nucleic Acids Research, 38(11):3771-9 Feb. 2010). Reducing dicer cleavage is beneficial, because dicer cleavage of the editing oligonucleotide on the side of the duplex nearest the editing site would detach the RISC loaded region from the rest of the editing oligonucleotide, which would eliminate the advantage of RISC loading if this occurred prior to strand invasion into the targeted DNA duplex.

Double-stranded structures capable of loading into RISC are known in the art, and include STEALTH RNAi compounds (Life Technologies, San Diego CA and U.S. patent no. 8,815,821), Dicer substrates (U.S. patents no. 8,349,809, 8,513,207, and 8,927,705), rxRNA ori (RXi Pharmaceuticals, Marlborough, Massachusetts), RNAi triggers with shortened duplexes (U.S. patent application no. 20120065243 filed 2009), and siRNA (U.S. patent nos. 7,923,547; 7,956,176; 7,989,612; 8,202,979; 8,232,383; 8,236,944; 8,242,257; 8,268,986; 8,273,866 and U.S. patent application serial no. 13/693,478). These RNAi trigger configurations, with the various chemical modification patterns known to support RISC loading, and in some cases enhance tissue and cellular uptake, can be incorporated into the editing oligonucleotide, as has been done with siRNA in the RISC loading editing oligonucleotide described herein (ETAGEN serial number 100085 hybridized to 100086) so long as a free 5' hydroxyl or a phosphorylated 5' hydroxyl on the editing strand is maintained, or liberated within the cell. Additional examples of editing oligonucleotides oligonucleotides are listed in Figure 2 and helper oligonucleotides in Table XIV, which comprise various features described above. The chemical modification patterns, lengths and configurations of editing oligonucleotides and optional helper oligonucleotides described in Figure 2 can be applied to various editing targets, including those listed herein, using the methods described herein. The number of and positioning of each chemical modification can be varied as described herein.

### VI. Advantages

The embodiments of the present invention that employ the Kmiec method have some advantages over the chemical modification method, because the Kmiec method does not require chemically reactive groups be attached to the editing oligonucleotide, achieves editing of a base to any other natural base and allows for creating insertions or deletions. These "footprint-free" edits can be more readily reversed, if necessary, by performing additional "footprint-free" edits back to the original sequence. This is an important safety feature for genome editing therapeutics.

The embodiments of the present invention that employ the chemical modification method has some advantages over the Kmiec method, because the chemical modification method involves the addition or removal of specific groups (*i*.*e*., methylation, ethylation or deamination) to change the targeted nucleobase base-pairing specificity, and thus does not require active cellular recombination machinery.

The editing oligonucleotides of the present invention may be utilized without CRISPR or proteins such as zinc finger or engineered programmable nucleases. Methods utilizing CRISPR and/or zinc finger are using single-stranded oligonucleotides which are not the guide RNA in CRISPR, but a separate single-stranded oligonucleotide, as the donor to repair the site. However, the methods and compositions of the present invention do not strictly require these other exogenous protein components and result in similar or substantially similar efficiencies of precise editing as current methods.

The present invention is a nucleic acid repair approach that differs from approaches that strictly require CRISPR/Cas9, Zinc Finger and Talen DNA nucleases because it repairs the mutant sequence directly and accurately without the requirement of creating potentially dangerous breaks in the DNA. In addition, some embodiments of the present invention may optionally be administered without delivery particles or immunogenic proteins.

The present invention may be utilized to permanently inactivate any gene by creating a site-specific mutation, for example a stop codon at a desired location that prevents translation. One of the unique applications of the present invention is the targeting of a point mutation that modulates or corrects the function of a gene (*e*.*g*., gain-of-function mutations caused by dominant mutations) that cannot be addressed by other known silencing methods

Other approaches such as competing gene therapy and mRNA replacement strategies can replace a mutated gene product. However, certain embodiments of the present invention has the advantage of achieving completely normal gene regulation and expression levels without incorporation of vector sequences or causing chromosomal damage at vector insertion sites.

It will be understood that any of the above described methods can be used in combination with certain other methods herein, or not used in such combinations. Furthermore, any of the above described compositions can be optionally used with certain methods described herein and/or combined with other compositions herein. Furthermore, improved features of editing oligonucleotides and optional helper oligonucleotide compositions described herein (*i*.*e*. chemical modifications, structures such as hairpins and delivery vehicles) can be used in combination with other improved features of editing oligonucleotides and optional helper oligonucleotide compositions described herein.

### VII. EXAMPLES

Figure 2 describes examples of editing oligonucleotides of the present invention. Some of the editing oligonucleotide sequences in Figure 2 target a null mutation in green fluorescent protein, and correct this mutation into a functional sequence that can be readily monitored by assaying for fluorescence (Erin E. Brachman and Eric B. Kmiec *supra*). Other oligonucleotides in Figure 2 target other genes. The chemical modification patterns in Figure 2, however, can be applied to editing oligonucleotides targeting mutations, to editing oligonucleotides which create a protective allele or to editing oligonucleotides that create other desirable changes in the genome in other target genes. In each case in these examples, when not already determined by the disclosed sequence, and in other embodiments of the present invention the editing site may be in the center region of the editing oligonucleotide, or may be offset towards the 5'- or 3'- termini.

In particularly useful embodiments, the editing site is more than five nucleotides from either terminus. It is also preferable to have the editing site in a region of DNA that is unmodified or, if modified, has modifications that are still recognized as template DNA by the cellular machinery that repairs the target DNA strand and/or replication machinery (*i*.*e*. phosphorothioate, 2'F, LNA, 2'-O-methyl or 5 methyl C).

Editing oligonucleotides may be designed to be complementary to either strand of the genomic DNA. It is particularly useful that they will be designed to bind to the template strand for lagging strand synthesis, as this tends to lead to more efficient editing. However, each strand may be targeted and it can be readily determined which strand leads to more efficient editing. Useful phosphorothioate backbone modification patterns and lengths of editing oligonucleotides for the present invention may be found in PCT/US2015/65348.

**TABLE XIV EXEMPLARY HELPER OLIGONUCLEOTIDE SEQUENCES OF THE PRESENT INVENTION**

| **Primary Indication(s)** | **Target** | **Optional Helper Oligonucleotide:** |
|---|---|---|
| AIDS | CCR5 (McNeer *et al.* 2013) | N-term |
| | | KKKJTJTTJTTJT000TCTTCTTCTCATTTCKKK |
| AIDS | CCR5 (McNeer *et al.* 2013) | N-term |
| | | KKKJTJTTJTTJT000TCTTCTTCTCATTTCKKK |
| Beta- thalassemia | HBB (McNeer *et al.* 2013) | N-term KKKKKKJJTJTTJTT000TTCTTCTCC |
| | | N term |
| | | KKKJTTTJTTTJTJTOOOTCTCTTTCTTTCAGGGCAK KK or |
| Beta- thalassemia Cystic | HBB (McNeer *et al.* 21013) | N term KKK- |
| | | JJJTJJTTJTOOOTCTTCCTCCCACAGCTCC-KKK N-term |
| Fibrosis | CFTR (McNeer *et al.* 2015) | KKKTJTJJTTTOOOTTTCCTCTATGGGTAAGKKK |
| Clamp (bis-PNA) or PNA Tail Clamp:K is lysine, O is a 8-amino-2,6-dioxaoctanoic acid linker and J is pseudoisocytosine | | |

General Methods Employed for Experiments Used to Generate Editing Efficiency Data in Figure 2.

### A. Cell line and culture conditions

For GFP targets, genetically modified HCT116 cells were employed. HCT116 cells were acquired from ATCC (American Type Cell Culture, Manassas, VA). HCT116-19 was created by integrating a pEGFP-N3 vector (Clontech, Palo Alto, CA) containing a mutated eGFP gene. The mutated eGFP gene has a nonsense mutation at position 167 resulting in a nonfunctional eGFP protein. For these experiments, HCT116-19 cells were cultured in McCoy's 5A Modified medium (Thermo Scientific, Pittsburgh, PA) supplemented with 10% fetal bovine serum, 2mM L-Glutamine, and 1% Penicillin/Streptomycin. Cells were maintained at 37°C and 5% carbon dioxide.

### B. Transfection of HCT116-19 cells

For experiments utilizing synchronized cells, HCT116-19 cells were seeded at 2.5 X 10⁶ cells in a 100mm dish and synchronized with 6mM aphidicolin for 24 hours prior to targeting. Cells were released for 4 hours (or indicated time) prior to trypsinization and transfection by washing with PBS (2/2) and adding complete growth media. Synchronized and unsynchronized HCT116-19 cells were transfected at a concentration of 5 X 10⁵ cells/100ul in 4mm gap cuvette (BioExpress, Kaysville, UT), using ∼1ug of editing oligonucleotide. Single-stranded oligonucleotides were electroporated (250V, LV, 13ms pulse length, 2 pulses, 1s interval) using a Bio-Rad Gene Pulser XCell^{™} Electroporation System (Bio-Rad Laboratories, Hercules, CA). Cells were then recovered in 6-well plates with complete growth media at 37°C for the indicated time prior to analysis. Analysis of gene edited cells. Fluorescence (eGFP) was measured by a Guava EasyCyte 5 HT^{™} Flow Cytometer (Millipore, Temecula, CA). Cells were harvested by trypsinization, washed once with PBS and resuspended in buffer (0.5% BSA, 2mM EDTA, 2mg/mL Propidium Iodide (PI) in PBS). Propidium iodide was used to measure cell viability as such, viable cells stain negative for PI (uptake). Correction efficiency was calculated as the percentage of the total live eGFP positive cells over the total live cells in each sample. Error bars are produced from three sets of data points using calculations of Standard Error (see the following for methods: Bialk P, Rivera-Torres N, Strouse B, Kmiec EB. PLoS One. 2015;10(6):e0129308).

Examples of a Process for optimizing length and positioning of editing oligonucleotide can be found in and PCT/US2015/65348.

### Example 1

### Method for preparing editing oligonucleotides with optimized editing activity in cells

Editing oligonucleotides are readily be tested for editing in cells to obtain optimized editing oligonucleotide sequence using the following steps:
A. define the editing oligonucleotide 3 nucleotides 5' of the nucleobase targeted for editing (or the 3' most targeted nucleotide if more than one targets exist in this region) as the 5' terminal complementary nucleotide of an editing oligonucleotide sequence; moving in one nucleotide increments, add a single 5' nucleotide extension of the editing oligonucleotide; and reiterate the process above 30 times, or up to 50 times or up to 200 times;
B. define the 3' ends of editing oligonucleotides, perform the same process as above, except towards the 3' of the nucleobase targeted for editing (or the 5' most targeted nucleotide if multiple targets exist in this region); moving in one, two, 5 or 10 nucleotide increments, add one, two, 5 or 10 3' nucleotide extension of the editing oligonucleotide; and reiterate the process above 30 times, or up to 50 times or up to 200 times.
C. make a matrix of all the resulting 5' and 3' terminal nucleotides of editing oligonucleotides, eliminate editing oligonucleotide sequences of less than 12 nucleotides, as these are unlikely to be unique in the genome and then select all the remaining sequences that are equal or less than 30, 50 or 200 nucleotides, and test them for editing activity in cells to determine the most efficient editing oligonucleotide sequences.

### Example 3

### Design of helper oligonucleotide sequences

Designing helper oligonucleotide sequences is performed beginning with a helper oligonucleotide sequence that is at least 8 bases long. For helper oligonucleotides a particularly useful length of the W/C binding region is less than 25 nucleotides or less than 50 nucleotides. For helper oligonucleotides the sequence may be shifted 5' or 3' (N terminal or C terminal in the case of PNAs) by one nucleotide increments until the binding site is further than 100 nucleotides from the editing site, or further than 200 nucleotides, or further than 400 nucleotides. For triplex forming regions on helper oligonucleotides they will generally target all purine sites, or sites which are 75% or more purine, or all pyrimidine sites or sites that are 75% or more pyrimidine. For triplex forming regions on helper oligonucleotides, the sites can be shifted or lengthened only to the extent the site maintains the desired base composition. The triplex region can match the antisense region of the clamp (in the case of PNAs sometimes called bis-PNAs), or the antisense sequence can be longer than the triplex region, by the extension of complementarity to the target in the 5' or 3' direction (see McNeer, 2013 *supra,* McNeer 2015 *supra,* Bahal et al. Current Gene Therapy 14(5):331-42, 2014, Nielsen et al. Current Issues in Molecular Biology 1(1-2):89-104, 1999 and Gaddis et al. Oligonucleotides, 2006 Summer;16(2):196-201. http://spi.mdanderson.org/tfo/ that provides a search engine for identifying triplex binding sites and Table XIV for examples of clamps).

### Example 4

### Selecting optimized CFTR targeting editing oligonucleotide

### A. Optimizing editing oligonucleotide in cell culture

### 1. Initial cell culture screen for optimal editing oligonucleotide sequences

Twenty editing oligonucleotide sequences are selected for initial cell culture efficacy screening, along with control editing oligonucleotides of mutant sequences. The editing oligonucleotides will be designed to correct the deltaF508 mutation causing Cystic Fibrosis. An existing active editing oligonucleotide targeting CFTR is used as a positive control and reference sequence (*see* McNeer et *al.* 2015 *supra).* These screens are carried out in human cell lines. The screening begins with an editing oligonucleotide having a length of ∼40 nucleotides centered on the deltaF508 mutation site, which generally provides
a good balance between efficacy and ease of manufacturing. In most examples in the literature, the editing site is approximately in the center of the editing oligonucleotide. This results in some level of editing efficiency but it is not necessarily optimal. Therefore, the length, target strand (antisense or sense) and 5'- 3' positioning of the editing nucleotide will be varied. The editing oligonucleotides is tested for editing activity in cells by lipofection or electroporation to determine the optimal editing oligonucleotide sequence, as assayed by PCR/Next Gen sequencing (see methods below). Beginning with an editing oligonucleotide sequence of the desired editing efficiency range from the first round of optimization, the editing oligonucleotide sequence is further modified in length by adding or removing bases complementary to the target on the 5' termini, 3' termini, or both termini. Additional editing efficacy testing in cells will yield a lead and backup lead editing oligonucleotide sequences. Similarly, the helper oligonucleotide PNA clamp is varied as described herein, and tested with the best editing oligonucleotide, to identify the most active helper
oligonucleotide. Baseline editing efficiency of 2% or better at the DNA level per treatment *in vitro* as assayed by sequencing can then be achieved.

### 2. Chemistry and configuration optimization and sequence fine tuning

Despite the successful application of chemically modified end-blocked donors to editing in cells and animals, the projected half-life of end-blocked editing oligonucleotides is only 10-30 minutes in cells. Therefore, they require high doses and result in modest editing efficiencies. High DNA endonuclease activity in mammalian cells has been demonstrated with antisense gapmers, where even one or a few unmodified DNA linkages led to lower intracellular half-lives and reduced efficacy (Monia *et al.,* 1995 *supra*). In the case of antisense, endonuclease stability has been resolved by employing internal modifications throughout the oligonucleotide, in addition to optional end-blocks. There is an array of endonuclease resistant modified nucleic acid chemistries, but most are not recognized by the cellular recombination/repair machinery and therefore does not support editing when placed internally near the editing site. The present invention modifies the editing oligonucleotide at a number of positions along its length to improve the therapeutic properties of nucleic acids, which include: reducing inflammation; increasing nuclease stability; increasing target binding-enhanced efficacy; increasing free uptake (self-delivery without encapsulation) by conjugating delivery ligands; and stabilizing against nucleases during transit through the endo-lysosomal pathway.

It has been found that certain exonuclease stabilized modifications can be employed internally in the editing oligonucleotides (referred to here as third generation editing oligonucleotides). A tool box of third generation chemical modifications is utilized to optimize editing oligonucleotides for use in cells and *in vivo.* Examples of advanced chemical modification patterns and configurations that are deployed are:
1. Generation (Gen) 3A, 3B and 3C from Figure 2 in PCT/US2015/65348.
2. combining helper oligonucleotide PNAs of various configurations and chemistries that have been found to enhance editing efficiency with editing oligonucleotides (initial tests employ the PNA-Clamp already shown to have baseline activity specifically, hCFPNA-2 of McNeer *et al.,* 2015 *supra*).
3. implementing an editing oligonucleotide structure chemistry that interacts with endogenous RNAi cellular machinery shown to enhance the hybridization rate to DNA; and
4. utilizing self-delivering conjugates of editing oligonucleotide in combination with more heavily modified editing oligonucleotides that will survive transit through the endo-lysosomal pathway and allow for delivery without encapsulation.
5. Employing chemical modifications at or near the editing site, such as 2'F, LNA or 2'-O-methyl, to inhibit mismatch repair of the editing oligonucleotides (*see* CFTR deltaF508 targeting editing oligonucleotides in Figure 2.
6. Including a 5' phosphate or 5' phosphate analog as described herein and exemplified in Figure 2.

This will achieve a 2-8% editing or better at the genomic DNA level per treatment as assayed by PCR, and confirmed by target protein assays. The editing oligonucleotides are employed with multiple dosing in animals and eventual clinical trials to obtain the desired cumulative level of editing (*see* McNeer, 2015 *supra* for formulation, transfection and DNA and functional assays). The editing and helper oligonucleotides is formulated for nebulization, and patients treated by inhalation. Systemic formulations are used to target secondary tissues affected by CF.

### 3. Editing oligonucleotide synthesis

Better editing efficacy has been observed with higher quality compounds. Therefore, high quality controlled gel isolated editing oligonucleotides will be synthesized at 1 uMole scale, and employ mass spec to confirm identity and analytical HPLC to confirm purity. The high quality editing oligonucleotides is >80% pure (>1 mg quantity) in a scalable and reproducible manner so that future batches of the lead editing oligonucleotides may be prepared with similar activity and at larger scale for animal studies and eventual human trials.

### 4. Assays for editing efficiency

### i. Target nucleic acids

Assays for nucleic acid target sequence are performed utilizing genomic PCR and RT-PCR gels to assay splice skipping. For sequencing, primers for genomic DNA PCR will be designed and synthesized to assay for editing efficiency. Established protocols will be employed that control for potential PCR artifacts, including time zero reconstruction controls, and editing in silent mutations in addition to the desired correction to distinguish between true editing and potential contamination with environmental human DNA. Next Generation Sequencing are performed as has been established previously to assay for genome editing.

### ii. Assays for Target protein

Assays for protein targets are performed utilizing Western blot analysis, immunohistochemistry and functional NPD (*see* McNeer, 2015 *supra)*

### 5. Assays for off-target editing in cultured cells

Measuring specificity is an important step for therapeutic genome editing. When employing donor DNA, the most dramatic result of off-target editing is the potential insertion of the donor DNA into non-targeted sites. Full or partial insertions of the donor DNA is readily assayed by FISH, PCR, or genome-wide sequencing.

The 10 most homologous genomic sites for off-target editing are tested to obtain, less than 0.1% off target editing at each site per treatment. Genomic DNA is probed for off-target integration of the editing oligonucleotide sequence to achieve, less than one off-target integration event per 1000 cells. Confirmatory testing is then performed in primary target cells. These studies provide the groundwork for animal studies.

### 6. Optimizing editing in vivo

Animal efficacy in mice and formulation studies is performed with lead editing oligonucleotides developed in cell culture screening. Preliminary PK ADME and toxicity studies is then performed.

These studies: optimize formulation or self-delivering chemistry to maximize *in vivo* editing efficiency; identify a lead editing oligonucleotide that edits 2-5% of the target DNA per treatment *in vivo*; assess off target effects by targeted genome sequencing; and perform preliminary toxicity assessment. One consideration is that the editing oligonucleotide sequences are generally species specific, so an engineered knock-in transgenic animal is employed if one sequence of editing oligonucleotides is to be used for human cell culture and mouse animal testing.

Two delivery modes are tested. Self-delivering (also known as free or naked oligomers), will employ delivery conjugates in combination with nuclease stabilizing internal chemical modifications to obtain free uptake (self-delivery without encapsulation). Uptake through the endo-lysosomal pathway exposes the editing oligonucleotide to potent nucleases, so nuclease resistant modifications are utilized along the editing oligonucleotide, to the extent that the modification pattern is consistent with retaining interaction with the endogenous repair system. Chemistries that have been shown to achieve self-delivery with oligomers of similar size and charge as the editing oligonucleotides (*i*.*e*. Byrne et *al.* 2013 *supra* and Alterman et *al.* 2016 *supra*) will be employed. Potential lung-specific conjugates for nanoparticles or direct editing oligonucleotide conjugation include receptor ligands, attachment targets to increase retention time and/or diffusion enhancers to better penetrate glycolax.

*In vivo* delivery with nanoparticles is challenging, in that the nanoparticles must not be destabilized by serum and they must diffuse into targeting tissues. Fortunately, encapsulated nucleic acids are completely protected from nucleases en route to the target cell. Initial results from Saltzman, Glazer and Egan's group at Yale University have identified a nanoparticle formulation suitable for delivering editing oligonucleotides to the nasal and lung ciliated epithelial cells with modest editing efficiency by intranasal instillation in mice (McNeer *et al.,* 2015 *supra*).

Particular delivery vehicles useful for delivering genome editing oligonucleotides in vitro and in vivo include: PLGA and 15% (by weight) poly (beta amino ester) (PBAE). These polymers are particularly useful when using neutral helper oligonucleotides, such as PNA clamps, because neutral or positively charged helper oligonucleotides can be readily and efficiently loaded in these particles along with negatively charged editing oligonucleotides.

Derivatized PLGA/PBAE nanoparticles with MPG *via* a PEGylated phospholipid linker (DSPE-PEG2000) has also been shown to enhance uptake in vivo, particularly in the lung. (McNeer, et al., Gene Ther. 20(6): 658-669, 2013. doi:10.1038/gt.2012.82, McNeer, et al., Nature Comm. DOI:10.1038/ncomms 7952:1-11, 2015)

Initially animal models and administration protocols previously employed by McNeer *et al.,* 2015 *supra* with the editing oligonucleotides and delivery polymers as positive controls may be utilized. Variations in delivery particle composition and alternative delivery particles (including alternative ligand conjugates listed above in the self-delivery section) are assessed with the aim of increasing delivery of editing oligonucleotides to the targeted epithelia cells and epithelial cell progenitor cells, and thereby increase editing efficiency. Also see Bahal, R. et al. Nat. Commun. 7, 13304 (2016) as an example of delivering editing oligonucleotides *in vivo* and successful editing *in vivo.*

### Example 5

### Editing by targeted nucleobase chemical modification with engineered transcription factors without oligonucleotides

Another specific exemplification of our methods of genome editing by nucleobase modification is by Yang *et al.* 2016 (Yang, L. et al. bioRxiv, doi:10.1101/066597,2016, also published as Yang, L. et al. Nat. Commun. 7, 13330 (2016)doi:10.1038/ncomms13330) whereby a deaminase nucleobase modifying activity (also known as a sequence modifying or editing moiety) is fused to an engineered transcription factor (for example, Zinc Finger or TALEN) and used to obtain highly precise point edits in mammalian cells in culture. This method can be used for the classes of edits, nucleobase chemical modifications leading to changes in sequence, target indications and corresponding target genes and target edits within those genes
described herein. In the case of enzymatic changes, the relevant nucleobase modifying enzyme can be tethered or fused to the engineered transcription factor. In the case of reactive chemicals, the reactive chemicals could be conjugated to the engineered transcription factor. In addition to deamination, the other chemical modifications described herein can be employed (see Table VII).

Aspects of the invention disclosed herein concern the following:
In a preferred aspect, the present invention relates to an editing oligonucleotide comprising a crisprRNA domain and an inactivated Cas9 domain linked to a base modifying activity, wherein said crisprRNA domain and said inactivated Cas9 domain is positioned in the proximity of a targeted nucleobase in a genomic sequence, wherein said base modifying activity causes deamination of said targeted nucleobase.

In a further aspect, the invention relates to a method of site directed deamination of a target nucleobase in a genomic sequence, directed by an editing oligonucleotide comprising a crisprRNA domain and an inactivated Cas9 domain linked to a base modifying activity comprising the steps of: introducing into a cell or an organism said editing oligonucleotide according to claim 1 without additional exogenous proteins or nucleic acids to assist in editing said target nucleobase, wherein said editing oligonucleotide comprises one or more modification(s), wherein said modification(s) is one or more backbone modification(s), sugar modification(s) and/or nucleobase modification(s), wherein said editing oligonucleotide is substantially complementary to said genomic sequence containing said target nucleobase, wherein said modifications of said editing oligonucleotide increase the efficiency of editing and wherein said site directed deamination of said target nucleobase is deamination of a cytosine nucleobase to a uracil nucleobase, directed by a crisprRNA and said inactivated Cas9 of said editing oligonucleotide.

## Claims

1. A single-stranded chemically modified editing oligonucleotide for use as a medicament for modifying a genomic sequence within a cell of a subject wherein the use is not for modifying the germ line genetic identity of human beings, wherein the editing oligonucleotide comprises, in 5' to 3' order, a 5' terminal segment; a 5' proximal segment; a 5' editing segment; an editing site; a 3'editing segment; a 3' proximal segment; and a 3' terminal segment, wherein:
a) the 5' terminal segment comprises one or more backbone modifications and, optionally, a 5' phosphate with or without a thiophosphate modification and, further optionally a nucleobase modification;
b) the 5' proximal segment and/or the 5' editing segment optionally comprises one or more backbone modifications, one or more nucleobase modifications, and/or one or more sugar modifications;
c) the editing site comprises one or more of:
i) a 2' modified sugar;
ii) a locked nucleic acid (LNA); and
iii) a phosporothioate; and
d) the 5' proximal segment and/or the 5' editing segment optionally comprises one or more backbone modifications, one or more nucleobase modifications, and/or one or more sugar modifications; and
e) the 3' terminal segment comprises one or more backbone modifications, or the 3' terminus is conjugated to a delivery moiety, such as cholesterol-TEG, and wherein the 3' terminal segment optionally comprises a nucleobase modification.

2. The editing oligonucleotide of claim 1, wherein the 5' terminal segment comprises a LNA, or a phosphorothioate.

3. The editing oligonucleotide of claim 1 or 2, wherein the 5' terminal segment comprises a 5' phosphate or a 5' thiophosphate.

4. The editing oligonucleotide of any one of claims 1-3, wherein the 5' proximal segment and/or the 5' editing segment comprises one or more of a 5-methyl cytosine, a phosphorothioate, a LNA, a 2'-F sugar, and a 2'-OMe sugar.

5. The editing oligonucleotide of any one of claims 1-4, wherein the editing site comprises a 2'-F sugar, or a 2'-OMe sugar.

6. The editing oligonucleotide of any one of claims 1-4, wherein the editing site comprises a LNA.

7. The editing oligonucleotide of claim 5 or 6, wherein the editing site additionally comprises a phosphorothioate.

8. The editing oligonucleotide of any of claims 1-7, wherein the 3' proximal segment and/or the 3' editing segment comprises one or more of a 5-methyl cytosine, a phosphorothioate, and a 2'-F sugar.

9. The editing oligonucleotide of any of claims 1-8, wherein the 3' terminal segment comprises a LNA.

10. The editing oligonucleotide of any of claims 1-8, wherein the 3' terminal segment comprises a phosphorothioate.

11. The editing oligonucleotide of claim 10, wherein the 3' terminal segment additionally comprises a 5-methyl cytosine.

12. The editing oligonucleotide of any of claims 1-8, wherein the 3' terminal segment is conjugated to cholesterol-TEG.

13. The editing oligonucleotide of any of claims 1-12, wherein the editing oligonucleotide is self-delivering.


SEQUENCE LISTING

<110>  WOOLF, Tod M.
       LEBEDEV, Alexandre
       HOGREFE, Richard I.

<120>  IMPROVED METHODS FOR GENOME EDITING WITH AND WITHOUT PROGRAMABLE
       NUCLEASES

<130>  ETAGEN.002.PCT

<150>  US 62/333,044
<151>  2016-05-06

<150>  US 62/410,487
<151>  2016-10-20

<160>  139

<170>  PatentIn version 3.5

<210>  1
<211>  72
<212>  DNA
<213>  Artificial Sequence

<220>
<223>  Synthetic oligonucleotide (ETAGEN Serial Number 100001)


<220>
<221>  misc_feature
<222>  (1)..(72)
<223>  Base modification: 3 phosphorothioates end-blocks on each
       terminus

<400>  1
catgtggtcg gggtagcggc tgaagcactg cacgccgtag gtcagggtgg tcacgagggt      60

gggccagggc ac                                                          72


<210>  2
<211>  72
<212>  DNA
<213>  Artificial Sequence

<220>
<223>  Synthetic oligonucleotide (ETAGEN Serial Number 100002)


<220>
<221>  misc_feature
<222>  (1)..(72)
<223>  Base modification: 3 phosphorothioates end-blocks on each
       terminus

<400>  2
catgtggtcg gggtagcggc tgaagcactg cacgccctag gtcagggtgg tcacgagggt      60

gggccagggc ac                                                          72

<210>  3
<211>  40
<212>  DNA
<213>  Artificial Sequence

<220>
<223>  Synthetic oligonucleotide (ETAGEN Serial Number 100003)

<400>  3
cggctgaagc actgcacgcc gtaggtcagg gtggtcacga                          40


<210>  4
<211>  40
<212>  DNA
<213>  Artificial Sequence

<220>
<223>  Synthetic oligonucleotide (ETAGEN Serial Number 100004)

<400>  4
cggctgaagc actgcacgcc ctaggtcagg gtggtcacga                          40


<210>  5
<211>  40
<212>  DNA
<213>  Artificial Sequence

<220>
<223>  Synthetic oligonucleotide (ETAGEN Serial Number 100005)


<220>
<221>  misc_feature
<222>  (1)..(40)
<223>  Base modification: 3 phosphorothioates end-blocks on each
       terminus (Parent)

<400>  5
cggctgaagc actgcacgcc gtaggtcagg gtggtcacga                          40


<210>  6
<211>  40
<212>  DNA
<213>  Artificial Sequence

<220>
<223>  Synthetic oligonucleotide (ETAGEN Serial Number 100006)


<220>
<221>  misc_feature
<222>  (1)..(40)
<223>  Base modification: 3 phosphorothioates end-blocks on each
       terminus

<400>  6
cggctgaagc actgcacgcc ctaggtcagg gtggtcacga                          40


<210>  7

<211>  40
<212>  DNA
<213>  Artificial Sequence

<220>
<223>  Synthetic oligonucleotide (ETAGEN Serial Number 100007)


<220>
<221>  misc_feature
<222>  (1)..(40)
<223>  Base modification: half phosphorothioates positive 40 mer
       (9s-20o-10s) with unmodified editing region

<400>  7
cggctgaagc actgcacgcc gtaggtcagg gtggtcacga                          40


<210>  8
<211>  40
<212>  DNA
<213>  Artificial Sequence

<220>
<223>  Synthetic oligonucleotide (ETAGEN Serial Number 100008)


<220>
<221>  misc_feature
<222>  (1)..(40)
<223>  Base modification: half phosphorothioates positive 40 mer
       (9s-20o-10s) with unmodified editing region

<400>  8
cggctgaagc actgcacgcc ctaggtcagg gtggtcacga                          40


<210>  9
<211>  40
<212>  DNA
<213>  Artificial Sequence

<220>
<223>  Synthetic oligonucleotide (ETAGEN Serial Number 100009)


<220>
<221>  misc_feature
<222>  (1)..(40)
<223>  Base modification: half phosphorothioates positive 40 mer
       (9s-20o-10s) with unmodified editing region

<400>  9
ctgcgagatc gcggcagcgc catgctgagg ctcgtacaga                          40


<210>  10
<211>  40
<212>  DNA
<213>  Artificial Sequence

<220>
<223>  Synthetic oligonucleotide (ETAGEN Serial Number 100010)

<220>
<221>  misc_feature
<222>  (1)..(40)
<223>  Base modification: Majority phosphorothiotes except in editing
      region (16s-6o-17s)

<400>  10
cggctgaagc actgcacgcc gtaggtcagg gtggtcacga                              40


<210>  11
<211>  40
<212>  DNA
<213>  Artificial Sequence

<220>
<223>  Synthetic oligonucleotide (ETAGEN Serial Number 100011)


<220>
<221>  misc_feature
<222>  (1)..(40)
<223>  Base modification: Majority phosphorothiotes except in editing
      region (16s-6o-17s)

<400>  11
cggctgaagc actgcacgcc ctaggtcagg gtggtcacga                              40


<210>  12
<211>  40
<212>  DNA
<213>  Artificial Sequence

<220>
<223>  Synthetic oligonucleotide (ETAGEN Serial Number 100012)


<220>
<221>  misc_feature
<222>  (1)..(40)
<223>  Base modification: Majority phosphorothiotes except in editing
      region (16s-6o-17s)

<400>  12
ctgcgagatc gcggcagcgc catgctgagg ctcgtacaga                              40


<210>  13
<211>  40
<212>  DNA
<213>  Artificial Sequence

<220>
<223>  Synthetic oligonucleotide (ETAGEN Serial Number 100013)


<220>
<221>  misc_feature
<222>  (1)..(40)
<223>  Base modification: 3' 8x2'F high affinity arms with s end-blocks

<400>  13
cggctgaagc actgcacgcc gtaggtcagg gtggtcacga                              40


<210>  14
<211>  40
<212>  DNA
<213>  Artificial Sequence

<220>
<223>  Synthetic oligonucleotide (ETAGEN Serial Number 100014)


<220>
<221>  misc_feature
<222>  (1)..(40)
<223>  Base modification: 3′ 8x2′F high affinity arms with s end-blocks

<400>  14
cggctgaagc actgcacgcc ctaggtcagg gtggtcacga                              40


<210>  15
<211>  42
<212>  DNA
<213>  Artificial Sequence

<220>
<223>  Synthetic oligonucleotide (ETAGEN Serial Number 100015)


<220>
<221>  misc_feature
<222>  (1)..(42)
<223>  Base modification: 5′ non-phosphorothioate end-block
       (methyphosphonate), 3′ 3s end-blocks

<400>  15
ttcggctgaa gcactgcacg ccgtaggtca gggtggtcac ga                           42


<210>  16
<211>  42
<212>  DNA
<213>  Artificial Sequence

<220>
<223>  Synthetic oligonucleotide (ETAGEN Serial Number 100016)


<220>
<221>  misc_feature
<222>  (1)..(42)
<223>  Base modification: 5′ non-phosphorothioate end-block
       (methyphosphonate), 3′ 3s end-blocks

<400>  16
ttcggctgaa gcactgcacg ccctaggtca gggtggtcac ga                           42


<210>  17
<211>  40

<212>   DNA
<213>   Artificial Sequence

<220>
<223>   Synthetic oligonucleotide (ETAGEN Serial Number 100017)


<220>
<221>   misc_feature
<222>   (1)..(40)
<223>   Base modification: C′s replaced with 5′ methyl C to fool repair
        machinery, as to which strand is nascent

<400>   17
cggctgaagc actgcacgcc gtaggtcagg gtggtcacga                              40


<210>   18
<211>   40
<212>   DNA
<213>   Artificial Sequence

<220>
<223>   Synthetic oligonucleotide (ETAGEN Serial Number 100018)


<220>
<221>   misc_feature
<222>   (1)..(40)
<223>   Base modification: C′s replaced with 5′ methyl C to fool repair
        machinery, as to which strand is nascent

<400>   18
cggctgaagc actgcacgcc ctaggtcagg gtggtcacga                              40


<210>   19
<211>   13
<212>   RNA
<213>   Artificial Sequence

<220>
<223>   Synthetic oligonucleotide (ETAGEN Serial Number 100019)


<220>
<221>   misc_feature
<222>   (1)..(13)
<223>   Base modification: 2′ -O-methyl "protector" oligo complimentary
        to positions 1-13 of editing oligo

<400>   19
agugcuucag ccg                                                           13


<210>   20
<211>   14
<212>   RNA
<213>   Artificial Sequence

<220>
<223>   Synthetic oligonucleotide (ETAGEN Serial Number 100020)

<220>
<221>  misc_feature
<222>  (1)..(14)
<223>  Base modification: 2′ -O-methyl "protector" oligo complimentary
       to positions 14-27 of editing oligo

<400>  20
gaccuacggc gugc                                                          14


<210>  21
<211>  13
<212>  RNA
<213>  Artificial Sequence

<220>
<223>  Synthetic oligonucleotide (ETAGEN Serial Number 100021)


<220>
<221>  misc_feature
<222>  (1)..(13)
<223>  Base modification: 2′ -O-methyl "protector" oligo complimentary
       to positions 28-40 of editing oligo

<400>  21
ucgugaccac ccu                                                           13


<210>  22
<211>  25
<212>  DNA
<213>  Artificial Sequence

<220>
<223>  Synthetic oligonucleotide (ETAGEN Serial Number 100022)


<220>
<221>  misc_feature
<222>  (1)..(25)
<223>  Base modification: 25mer all phosphorothioate DNA

<400>  22
gcactgcacg ccctaggtca gggtg                                              25


<210>  23
<211>  25
<212>  DNA
<213>  Artificial Sequence

<220>
<223>  Synthetic oligonucleotide (ETAGEN Serial Number 100023)


<220>
<221>  misc_feature
<222>  (1)..(25)
<223>  Base modification: 25mer all phosphorothioate DNA

<400>  23

gcactgcacg ccgtaggtca gggtg                                                25

<210>  24
<211>  25
<212>  DNA
<213>  Artificial Sequence

<220>
<223>  Synthetic oligonucleotide (ETAGEN Serial Number 100024)


<220>
<221>  misc_feature
<222>  (1)..(25)
<223>  Base modification: 25mer all phosphorothioate DNA

<400>  24
tcgcggcagc gccatgctga ggatc                                                25


<210>  25
<211>  40
<212>  DNA
<213>  Artificial Sequence

<220>
<223>  Synthetic oligonucleotide (ETAGEN Serial Number 100031)


<220>
<221>  misc_feature
<222>  (1)..(40)
<223>  Base modification: 3 phosphorothioates end-blocks on each
       terminus (PARENT)

<400>  25
cggctgaagc actgcacgcc gtaggtcagg gtggtcacga                                40


<210>  26
<211>  40
<212>  DNA
<213>  Artificial Sequence

<220>
<223>  Synthetic oligonucleotide (ETAGEN Serial Number 100032)


<220>
<221>  misc_feature
<222>  (1)..(40)
<223>  Base modification: 40mers (with 3 phosphorothioates on each
       terminus)

<400>  26
cggctgaagc actgcacgcc ctaggtcagg gtggtcacga                                40


<210>  27
<211>  40
<212>  DNA
<213>  Artificial Sequence

<220>
<223>  Synthetic oligonucleotide (ETAGEN Serial Number 100033)


<220>
<221>  misc_feature
<222>  (1)..(40)
<223>  Base modification: 5′ and 3′ 8x2′F high affinity arms with s
      end-blocks

<400>  27
cggctgaagc actgcacgcc ctaggtcagg gtggtcacga                          40


<210>  28
<211>  40
<212>  DNA
<213>  Artificial Sequence

<220>
<223>  Synthetic oligonucleotide (ETAGEN Serial Number 100034)


<220>
<221>  misc_feature
<222>  (1)..(40)
<223>  Base modification: 5′ and 3′ 8x2′F high affinity arms with s
      end-blocks

<400>  28
cggctgaagc actgcacgcc gtaggtcagg gtggtcacga                          40


<210>  29
<211>  40
<212>  DNA
<213>  Artificial Sequence

<220>
<223>  Synthetic oligonucleotide (ETAGEN Serial Number 100035)


<220>
<221>  misc_feature
<222>  (1)..(40)
<223>  Base modification: 3′ 8x2′-O-methyl high affinity arms with s
      end-blocks

<400>  29
cggctgaagc actgcacgcc gtaggtcagg gtggtcacga                          40


<210>  30
<211>  40
<212>  DNA
<213>  Artificial Sequence

<220>
<223>  Synthetic oligonucleotide (ETAGEN Serial Number 100036)


<220>

<221>  misc_feature
<222>  (1)..(40)
<223>  Base modification: 3′ 8x2′-O-methyl high affinity arms with s
       end-blocks

<400>  30
cggctgaagc actgcacgcc ctaggtcagg gtggtcacga                              40


<210>  31
<211>  40
<212>  DNA
<213>  Artificial Sequence

<220>
<223>  Synthetic oligonucleotide (ETAGEN Serial Number 100037)


<220>
<221>  misc_feature
<222>  (1)..(40)
<223>  Base modification: 5′ 8x2′-O-methyl high affinity arms with s
       end-blocks

<400>  31
cggctgaagc actgcacgcc gtaggtcagg gtggtcacga                              40


<210>  32
<211>  40
<212>  DNA
<213>  Artificial Sequence

<220>
<223>  Synthetic oligonucleotide (ETAGEN Serial Number 100038)


<220>
<221>  misc_feature
<222>  (1)..(40)
<223>  Base modification: 5′ 8x2′-O-methyl high affinity arms with s
       end-blocks

<400>  32
cggctgaagc actgcacgcc ctaggtcagg gtggtcacga                              40


<210>  33
<211>  40
<212>  DNA
<213>  Artificial Sequence

<220>
<223>  Synthetic oligonucleotide (ETAGEN Serial Number 100039)


<220>
<221>  misc_feature
<222>  (1)..(40)
<223>  Base modification: 5′ and 3′ 8x2′-O-methyl high affinity arms
       with s end-blocks

<400>  33

cggctgaagc actgcacgcc ctaggtcagg gtggtcacga                    40

<210>  34
<211>  40
<212>  DNA
<213>  Artificial Sequence

<220>
<223>  Synthetic oligonucleotide (ETAGEN Serial Number 100040)

<220>
<221>  misc_feature
<222>  (1)..(40)
<223>  Base modification: 5′ and 3′ 8x2′-O-methyl high affinity arms
       with s end-blocks

<400>  34
cggctgaagc actgcacgcc gtaggtcagg gtggtcacga                    40

<210>  35
<211>  40
<212>  DNA
<213>  Artificial Sequence

<220>
<223>  Synthetic oligonucleotide (ETAGEN Serial Number 100041)

<220>
<221>  misc_feature
<222>  (1)..(40)
<223>  Base modification: Central C′s replaced with 5′ methyl C to fool
       repair machinery as to which strand is nascent

<400>  35
cggctgaagc actgcacgcc ctaggtcagg gtggtcacga                    40

<210>  36
<211>  40
<212>  DNA
<213>  Artificial Sequence

<220>
<223>  Synthetic oligonucleotide (ETAGEN Serial Number 100042)

<220>
<221>  misc_feature
<222>  (1)..(40)
<223>  Base modification: Central C′s replaced with 5′ methyl C to fool
       repair machinery as to which strand is nascent

<400>  36
cggctgaagc actgcacgcc gtaggtcagg gtggtcacga                    40

<210>  37
<211>  40
<212>  DNA

<213>  Artificial Sequence

<220>
<223>  Synthetic oligonucleotide (ETAGEN Serial Number 100043)


<220>
<221>  misc_feature
<222>  (1)..(40)
<223>  Base modification: Central C replaced with 5′ methyl C to fool
       repair machinery as to which strand is nascent

<400>  37
cggctgaagc actgcacgcc gtaggtcagg gtggtcacga                              40


<210>  38
<211>  40
<212>  DNA
<213>  Artificial Sequence

<220>
<223>  Synthetic oligonucleotide (ETAGEN Serial Number 100044)


<220>
<221>  misc_feature
<222>  (1)..(40)
<223>  Base modification: Central C replaced with 5′ methyl C to fool
       repair machinery as to which strand is nascent

<400>  38
cggctgaagc actgcacgcc ctaggtcagg gtggtcacga                              40


<210>  39
<211>  44
<212>  DNA
<213>  Artificial Sequence

<220>
<223>  Synthetic oligonucleotide (ETAGEN Serial Number 100045)


<220>
<221>  misc_feature
<222>  (1)..(44)
<223>  Base modification: Phosphorothiote-free methylphosphonoate
       end-blocks, 5 methyl C′s and 2′F arms

<400>  39
ttcggctgaa gcactgcacg ccgtaggtca gggtggtcac gatt                         44


<210>  40
<211>  42
<212>  DNA
<213>  Artificial Sequence

<220>
<223>  Synthetic oligonucleotide (ETAGEN Serial Number 100047)

<220>
<221>  misc_feature
<222>  (1)..(42)
<223>  Base modification: 5′ methylphosphonoate end-blocks, 3′
      s-end-blocks, 5 methyl C′s and 3′ 2′F arm

<400>  40
ttcggctgaa gcactgcacg ccgtaggtca gggtggtcac ga                          42


<210>  41
<211>  42
<212>  DNA
<213>  Artificial Sequence

<220>
<223>  Synthetic oligonucleotide (ETAGEN Serial Number 100048)


<220>
<221>  misc_feature
<222>  (1)..(42)
<223>  Base modification: 5′ methylphosphonoate end-blocks, 3′
      s-end-blocks, 5 methyl C′s and 3′ 2′F arm

<400>  41
ttcggctgaa gcactgcacg ccctaggtca gggtggtcac ga                          42


<210>  42
<211>  44
<212>  DNA
<213>  Artificial Sequence

<220>
<223>  Synthetic oligonucleotide (ETAGEN Serial Number 100049)


<220>
<221>  misc_feature
<222>  (1)..(44)
<223>  Base modification: Phosphorothioate-free 5′ and 3′
      methylphosphonate end-blocks

<400>  42
ttcggctgaa gcactgcacg ccctaggtca gggtggtcac gatt                        44


<210>  43
<211>  44
<212>  DNA
<213>  Artificial Sequence

<220>
<223>  Synthetic oligonucleotide (ETAGEN Serial Number 100050)


<220>
<221>  misc_feature
<222>  (1)..(44)
<223>  Base modification: Phosphorothioate-free 5′ and 3′
      methylphosphonate end-blocks

<400>  43
ttcggctgaa gcactgcacg ccgtaggtca gggtggtcac gatt                        44


<210>  44
<211>  40
<212>  DNA
<213>  Artificial Sequence

<220>
<223>  Synthetic oligonucleotide (ETAGEN Serial Number 100058)


<220>
<221>  misc_feature
<222>  (1)..(40)
<223>  Base modification: 5′ 8x2′-O-methyl and 8x3′ 2′F high affinity
       arms with s end-blocks

<400>  44
cggctgaagc actgcacgcc gtaggtcagg gtggtcacga                             40


<210>  45
<211>  40
<212>  DNA
<213>  Artificial Sequence

<220>
<223>  Synthetic oligonucleotide (ETAGEN Serial Number 100060)


<220>
<221>  misc_feature
<222>  (1)..(40)
<223>  Base modification: 5′ 8x2′-O-methyl and 3′ 8x2′F high affinity
       arms with s end-blocks, with central 5 Methyl Cs

<400>  45
cggctgaagc actgcacgcc gtaggtcagg gtggtcacga                             40


<210>  46
<211>  40
<212>  DNA
<213>  Artificial Sequence

<220>
<223>  Synthetic oligonucleotide (ETAGEN Serial Number 100062)


<220>
<221>  misc_feature
<222>  (1)..(40)
<223>  Base modification: 3′ 14x2′F high affinity arms with s end-blocks

<400>  46
cggctgaagc actgcacgcc gtaggtcagg gtggtcacga                             40


<210>  47
<211>  40
<212>  DNA

<213>  Artificial Sequence

<220>
<223>  Synthetic oligonucleotide (ETAGEN Serial Number 100064)


<220>
<221>  misc_feature
<222>  (1)..(40)
<223>  Base modification: 3′ 20x2′F high affinity arms with s end-blocks

<400>  47
cggctgaagc actgcacgcc gtaggtcagg gtggtcacga                              40


<210>  48
<211>  40
<212>  DNA
<213>  Artificial Sequence

<220>
<223>  Synthetic oligonucleotide (ETAGEN Serial Number 100066)


<220>
<221>  misc_feature
<222>  (1)..(40)
<223>  Base modification: 5′ 14x2′-O-methyl high affinity arms with s
       end-blocks

<400>  48
cggctgaagc actgcacgcc gtaggtcagg gtggtcacga                              40


<210>  49
<211>  40
<212>  DNA
<213>  Artificial Sequence

<220>
<223>  Synthetic oligonucleotide (ETAGEN Serial Number 100068)


<220>
<221>  misc_feature
<222>  (1)..(40)
<223>  Base modification: 5′ 20x2′-O-methyl high affinity arms with s
       end-blocks

<400>  49
cggctgaagc actgcacgcc gtaggtcagg gtggtcacga                              40


<210>  50
<211>  72
<212>  DNA
<213>  Artificial Sequence

<220>
<223>  Synthetic oligonucleotide (ETAGEN Serial Number 100070)


<220>

<221>  misc_feature
<222>  (1)..(72)
<223>  Base modification: 5′ 8x2′-O-methyl high affinity arms with s
       end-blocks

<400>  50
catgtggtcg gggtagcggc tgaagcactg cacgccgtag gtcagggtgg tcacgagggt      60

gggccagggc ac                                                          72


<210>  51
<211>  72
<212>  DNA
<213>  Artificial Sequence

<220>
<223>  Synthetic oligonucleotide (ETAGEN Serial Number 100072)


<220>
<221>  misc_feature
<222>  (1)..(72)
<223>  Base modification: 5′ 24x2′-O-methyl high affinity arms with s
       end-blocks

<400>  51
catgtggtcg gggtagcggc tgaagcactg cacgccgtag gtcagggtgg tcacgagggt      60

gggccagggc ac                                                          72


<210>  52
<211>  44
<212>  DNA
<213>  Artificial Sequence

<220>
<223>  Synthetic oligonucleotide (ETAGEN Serial Number 100074)


<220>
<221>  misc_feature
<222>  (1)..(44)
<223>  Base modification: end-block 3′ phosphorothioate end-block

<400>  52
cggctgaagc actgcacgcc gtaggtcagg gtggtcacga cgcg                       44


<210>  53
<211>  40
<212>  DNA
<213>  Artificial Sequence

<220>
<223>  Synthetic oligonucleotide (ETAGEN Serial Number 100076)


<220>
<221>  misc_feature
<222>  (1)..(40)
<223>  Base modification: amino linker (TriLink Biotechnologies, San

Diego, CA) end-blocks

<400> 53
cggctgaagc actgcacgcc gtaggtcagg gtggtcacga 40


<210> 54
<211> 40
<212> DNA
<213> Artificial Sequence

<220>
<223> Synthetic oligonucleotide (ETAGEN Serial Number 100078)


<220>
<221> misc_feature
<222> (1)..(40)
<223> Base modification: 5′ and 3′ end-blocks

<400> 54
cggctgaagc actgcacgcc gtaggtcagg gtggtcacga 40


<210> 55
<211> 40
<212> DNA
<213> Artificial Sequence

<220>
<223> Synthetic oligonucleotide (ETAGEN Serial Number 100079)


<220>
<221> misc_feature
<222> (1)..(40)
<223> Base modification: amino linkers 5′ and 3′ end-blocks (TriLink
      Biotechnologies, San Diego, CA)

<400> 55
cggctgaagc actgcacgcc gtaggtcagg gtggtcacga 40


<210> 56
<211> 40
<212> DNA
<213> Artificial Sequence

<220>
<223> Synthetic oligonucleotide (ETAGEN Serial Number 100080)


<220>
<221> misc_feature
<222> (1)..(40)
<223> Base modification: Amino Linker end-blocks with 3′ 10x2′F arm 5′
      11x2′-O-methyl and 5 Methyl C editing region

<400> 56
cggctgaagc actgcacgcc gtaggtcagg gtggtcacga 40


<210> 57

<211>  40
<212>  DNA
<213>  Artificial Sequence

<220>
<223>  Synthetic oligonucleotide (ETAGEN Serial Number 100082)


<220>
<221>  misc_feature
<222>  (1)..(40)
<223>  Base modification: COMPLEMENTARY DNA STRAND TO THE PARENT with
       phosphorothioate end-blocks

<400>  57
tcgtgaccac cctgacctac ggcgtgcagt gcttcagccg                              40


<210>  58
<211>  40
<212>  DNA
<213>  Artificial Sequence

<220>
<223>  Synthetic oligonucleotide (ETAGEN Serial Number 100083)


<220>
<221>  misc_feature
<222>  (1)..(40)
<223>  Base modification: COMPLEMENTARY RNA STRAND TO THE PARENT with
       phosphorothioate end-blocks

<400>  58
tcgtgaccac cctgacctac ggcgtgcagt gcttcagccg                              40


<210>  59
<211>  40
<212>  DNA
<213>  Artificial Sequence

<220>
<223>  Synthetic oligonucleotide (ETAGEN Serial Number 100084)


<220>
<221>  misc_feature
<222>  (1)..(40)
<223>  Base modification: 5′ 8xRNA and 3′ 8x2′F high affinity arms with
       s end-blocks

<400>  59
cggctgaagc actgcacgcc gtaggtcagg gtggtcacga                              40


<210>  60
<211>  72
<212>  DNA
<213>  Artificial Sequence

<220>
<223>  Synthetic oligonucleotide (ETAGEN Serial Number 100085)

<220>
<221>  misc_feature
<222>  (1)..(72)
<223>  Base modification: Editing/Guide Strand with 5′ RISC entry site
      of alternating RNA/2′-O-methyl

<400>  60
catgtggtcg gggtagcggc tgaagcactg cacgccgtag gtcagggtgg tcacgagggt      60

gggccagggc ac                                                          72


<210>  61
<211>  21
<212>  DNA
<213>  Artificial Sequence

<220>
<223>  Synthetic oligonucleotide (ETAGEN Serial Number 100086)


<220>
<221>  misc_feature
<222>  (1)..(21)
<223>  Base modification: Sense strand that will allow the duplex with
      100085 to form editing compound capable of RISC entry

<400>  61
ccgctacccc gaccacatgt t                                                21


<210>  62
<211>  30
<212>  DNA
<213>  Artificial Sequence

<220>
<223>  Synthetic (ETAGEN Serial Number 100194)


<220>
<221>  misc_feature
<223>  N terminus has KKK and C terminus has KKKK

<220>
<221>  misc_feature
<222>  (1)..(1)
<223>  n is pseudoisocytosine

<220>
<221>  misc_feature
<222>  (5)..(5)
<223>  n is pseudoisocytosine

<220>
<221>  misc_feature
<222>  (9)..(9)
<223>  n is pseudoisocytosine

<220>
<221>  misc_feature

<222>   (11)..(11)
<223>   n is pseudoisocytosine

<220>
<221>   misc_feature
<222>   (12)..(13)
<223>   site of 3 Os (8-amino-2,6-dioxaoctanoic 5 acid linker)

<400>   62
ntttntttnt nttctctttc tttcagggca                                        30


<210>   63
<211>   20
<212>   DNA
<213>   Artificial Sequence

<220>
<223>   Synthetic (ETAGEN Serial Number 100196)


<220>
<221>   misc_feature
<223>   N terminus has KKK and C terminus has KKKK

<400>   63
aacctcttac atcagttaca                                                   20


<210>   64
<211>   20
<212>   DNA
<213>   Artificial Sequence

<220>
<223>   Synthetic (ETAGEN Serial Number 100197)


<220>
<221>   misc_feature
<223>   N terminus has KKK and C terminus has KKKK

<400>   64
aacctcttac atcagttaca                                                   20


<210>   65
<211>   20
<212>   DNA
<213>   Artificial Sequence

<220>
<223>   Synthetic (ETAGEN Serial Number 100198)


<220>
<221>   misc_feature
<223>   N terminus has KKK and C terminus has KKKK

<400>   65
aacctcttac atcagttaca                                                   20

<210>  66
<211>  20
<212>  DNA
<213>  Artificial Sequence

<220>
<223>  Synthetic (ETAGEN Serial Number 100199)

<400>  66
aacctcttac atcagttaca                                                    20


<210>  67
<211>  25
<212>  DNA
<213>  Artificial Sequence

<220>
<223>  Synthetic (ETAGEN Serial Number 100240)

<400>  67
tctgggttaa ggcaatagca atatc                                              25


<210>  68
<211>  25
<212>  DNA
<213>  Artificial Sequence

<220>
<223>  Synthetic (ETAGEN Serial Number 100232)

<400>  68
tctgggttaa ggcaatagca atatc                                              25


<210>  69
<211>  25
<212>  DNA
<213>  Artificial Sequence

<220>
<223>  Synthetic (ETAGEN Serial Number 100216)

<400>  69
tctgggttaa ggcaatagca atatc                                              25


<210>  70
<211>  25
<212>  DNA
<213>  Artificial Sequence

<220>
<223>  Synthetic (ETAGEN Serial Number 100210)

<400>  70
tctgggttaa ggcaatagca atatc                                              25


<210>  71
<211>  25
<212>  DNA

<213>  Artificial Sequence

<220>
<223>  Synthetic (ETAGEN Serial Number 100228)

<400>  71
tctgggttaa ggcaatagca atatc                                          25


<210>  72
<211>  25
<212>  DNA
<213>  Artificial Sequence

<220>
<223>  Synthetic (ETAGEN Serial Number 100217)

<400>  72
tctgggttaa ggcaatagca atatc                                          25


<210>  73
<211>  25
<212>  DNA
<213>  Artificial Sequence

<220>
<223>  Synthetic (ETAGEN Serial Number 100229)

<400>  73
tctgggttaa ggtaatagca atatc                                          25


<210>  74
<211>  64
<212>  DNA
<213>  Artificial Sequence

<220>
<223>  Synthetic (ETAGEN Serial Number 100175)

<400>  74
aaagaataac agtgataatt tctgggttaa ggcaatagca atatctctgc atataaatat    60

atta                                                                 64


<210>  75
<211>  60
<212>  DNA
<213>  Artificial Sequence

<220>
<223>  Synthetic (ETAGEN Serial Number 100206)


<220>
<221>  misc_feature
<223>  N-term Phosphate

<400>  75
aaagaataac agtgataatt tctgggttaa ggcaatagca atatctctgc atataaatat    60

<210>  76
<211>  25
<212>  DNA
<213>  Artificial Sequence

<220>
<223>  Synthetic (ETAGEN Serial Number 100177)


<220>
<221>  misc_feature
<223>  N-term Phosphate

<400>  76
tctgggttaa ggcaatagca atatc                                             25


<210>  77
<211>  43
<212>  DNA
<213>  Artificial Sequence

<220>
<223>  Synthetic (ETAGEN Serial Number 100204)


<220>
<221>  misc_feature
<223>  N-term Phosphate

<400>  77
agtgataatt tctgggttaa ggcaatagca atatctctgc ata                         43


<210>  78
<211>  25
<212>  DNA
<213>  Artificial Sequence

<220>
<223>  Synthetic (ETAGEN Serial Number 100181)


<220>
<221>  misc_feature
<223>  N-term Phosphate

<400>  78
tctgggttaa ggcaatagca atatc                                             25


<210>  79
<211>  25
<212>  DNA
<213>  Artificial Sequence

<220>
<223>  Synthetic (ETAGEN Serial Number 100182)


<220>
<221>  misc_feature

<223>  N-term Phosphate

<400>  79
tctgggttaa ggtaatagca atatc                                          25


<210>  80
<211>  25
<212>  DNA
<213>  Artificial Sequence

<220>
<223>  Synthetic (ETAGEN Serial Number 100218)


<220>
<221>  misc_feature
<223>  N-term Phosphate

<400>  80
tctgggttaa ggcaatagca atatc                                          25


<210>  81
<211>  25
<212>  DNA
<213>  Artificial Sequence

<220>
<223>  Synthetic (ETAGEN Serial Number 100222)


<220>
<221>  misc_feature
<223>  N-term Phosphate

<400>  81
tctgggttaa ggcaatagca atatc                                          25


<210>  82
<211>  27
<212>  DNA
<213>  Artificial Sequence

<220>
<223>  Synthetic (ETAGEN Serial Number 100203)


<220>
<221>  misc_feature
<223>  N-term Phosphate

<400>  82
tctgggttaa ggcaatagca atatctt                                        27


<210>  83
<211>  46
<212>  DNA
<213>  Artificial Sequence

<220>

<223>  Synthetic (ETAGEN Serial Number 100205)

<220>
<221>  misc_feature
<223>  N-term Phosphate

<400>  83
agtgataatt tctgggttaa ggcaatagca atatctctgc atattt                46

<210>  84
<211>  60
<212>  DNA
<213>  Artificial Sequence

<220>
<223>  Synthetic (ETAGEN Serial Number 100173)

<400>  84
aaagaataac agtgataatt tctgggttaa ggcaatagca atatctctgc atataaatat      60

<210>  85
<211>  60
<212>  DNA
<213>  Artificial Sequence

<220>
<223>  Synthetic (ETAGEN Serial Number 100174)

<400>  85
aaagaataac agtgataatt tctgggttaa ggtaatagca atatctctgc atataaatat      60

<210>  86
<211>  60
<212>  DNA
<213>  Artificial Sequence

<220>
<223>  Synthetic (ETAGEN Serial Number 100176)

<400>  86
aaagaataac agtgataatt tctgggttaa ggcaatagca atatctctgc atataaatat      60

<210>  87
<211>  60
<212>  DNA
<213>  Artificial Sequence

<220>
<223>  Synthetic (ETAGEN Serial Number 100220)

<400>  87
aaagaataac agtgataatt tctgggttaa ggcaatagca atatctctgc atataaatat      60

<210>  88
<211>  60
<212>  DNA
<213>  Artificial Sequence

<220>
<223>  Synthetic (ETAGEN Serial Number 100225)

<400>  88
aaagaataac agtgataatt tctgggttaa ggcaatagca atatctctgc atataaatat      60


<210>  89
<211>  60
<212>  DNA
<213>  Artificial Sequence

<220>
<223>  Synthetic (ETAGEN Serial Number 100226)

<400>  89
aaagaataac agtgataatt tctgggttaa ggcaatagca atatctctgc atataaatat      60


<210>  90
<211>  25
<212>  DNA
<213>  Artificial Sequence

<220>
<223>  Synthetic (ETAGEN Serial Number 100231)

<400>  90
tctgggttaa ggcaatagca atatc                                            25


<210>  91
<211>  25
<212>  DNA
<213>  Artificial Sequence

<220>
<223>  Synthetic (ETAGEN Serial Number 100233)

<400>  91
tctgggttaa ggtaatagca atatc                                            25


<210>  92
<211>  25
<212>  DNA
<213>  Artificial Sequence

<220>
<223>  Synthetic (ETAGEN Serial Number 100208)

<400>  92
tctgggttaa ggcaatagca atatc                                            25


<210>  93
<211>  25
<212>  DNA
<213>  Artificial Sequence

<220>
<223>  Synthetic (ETAGEN Serial Number 100224)

<400>  93
tctgggttaa ggcaatagca atatc                                          25


<210>  94
<211>  25
<212>  DNA
<213>  Artificial Sequence

<220>
<223>  Synthetic (ETAGEN Serial Number 100209)

<400>  94
tctgggttaa ggcaatagca atatc                                          25


<210>  95
<211>  25
<212>  DNA
<213>  Artificial Sequence

<220>
<223>  Synthetic (ETAGEN Serial Number 100221)

<400>  95
tctgggttaa ggcaatagca atatc                                          25


<210>  96
<211>  25
<212>  DNA
<213>  Artificial Sequence

<220>
<223>  Synthetic (ETAGEN Serial Number 100230)

<400>  96
tctgggttaa ggtaatagca atatc                                          25


<210>  97
<211>  25
<212>  DNA
<213>  Artificial Sequence

<220>
<223>  Synthetic (ETAGEN Serial Number 100213)

<400>  97
tctgggttaa ggcaatagca atatc                                          25


<210>  98
<211>  25
<212>  DNA
<213>  Artificial Sequence

<220>
<223>  Synthetic (ETAGEN Serial Number 100235)

<400>  98
tctgggttaa ggcaatagca atatc                                          25

<210>  99
<211>  25
<212>  DNA
<213>  Artificial Sequence

<220>
<223>  Synthetic (ETAGEN Serial Number 100214)

<400>  99
tctgggttaa ggcaatagca atatc                                          25


<210>  100
<211>  25
<212>  DNA
<213>  Artificial Sequence

<220>
<223>  Synthetic (ETAGEN Serial Number 100215)

<400>  100
tctgggttaa ggcaatagca atatc                                          25


<210>  101
<211>  25
<212>  DNA
<213>  Artificial Sequence

<220>
<223>  Synthetic (ETAGEN Serial Number 100236)

<400>  101
tctgggttaa ggtaatagca atatc                                          25


<210>  102
<211>  25
<212>  DNA
<213>  Artificial Sequence

<220>
<223>  Synthetic (ETAGEN Serial Number 100207)


<220>
<221>  misc_feature
<223>  N-term Phosphate

<400>  102
tctgggttaa ggcaatagca atatc                                          25


<210>  103
<211>  25
<212>  DNA
<213>  Artificial Sequence

<220>
<223>  Synthetic (ETAGEN Serial Number 100238)

<400>  103
tctgggttaa ggcaatagca atatc                                          25


<210>  104
<211>  25
<212>  DNA
<213>  Artificial Sequence

<220>
<223>  Synthetic (ETAGEN Serial Number 100212)

<400>  104
tctgggttaa ggcaatagca atatc                                          25


<210>  105
<211>  25
<212>  DNA
<213>  Artificial Sequence

<220>
<223>  Synthetic (ETAGEN Serial Number 100211)

<400>  105
tctgggttaa ggcaatagca atatc                                          25


<210>  106
<211>  25
<212>  DNA
<213>  Artificial Sequence

<220>
<223>  Synthetic (ETAGEN Serial Number 100227)

<400>  106
tctgggttaa ggcaatagca atatc                                          25


<210>  107
<211>  25
<212>  DNA
<213>  Artificial Sequence

<220>
<223>  Synthetic (ETAGEN Serial Number 100237)

<400>  107
tctgggttaa ggtaatagca atatc                                          25


<210>  108
<211>  25
<212>  DNA
<213>  Artificial Sequence

<220>
<223>  Synthetic (ETAGEN Serial Number 100179)


<220>
<221>  misc_feature

<223>  N-term Phosphate

<400>  108
tctgggttaa ggcaatagca atatc                                          25


<210>  109
<211>  25
<212>  DNA
<213>  Artificial Sequence

<220>
<223>  Synthetic (ETAGEN Serial Number 100180)


<220>
<221>  misc_feature
<223>  N-term Phosphate

<400>  109
tctgggttaa ggtaatagca atatc                                          25


<210>  110
<211>  25
<212>  DNA
<213>  Artificial Sequence

<220>
<223>  Synthetic (ETAGEN Serial Number 100219)


<220>
<221>  misc_feature
<223>  N-term Phosphate

<400>  110
tctgggttaa ggcaatagca atatc                                          25


<210>  111
<211>  25
<212>  DNA
<213>  Artificial Sequence

<220>
<223>  Synthetic (ETAGEN Serial Number 100223)


<220>
<221>  misc_feature
<223>  N-term Phosphate

<400>  111
tctgggttaa ggcaatagca atatc                                          25


<210>  112
<211>  27
<212>  DNA
<213>  Artificial Sequence

<220>

<223>  Synthetic (ETAGEN Serial Number 100195)

<220>
<221>  misc_feature
<223>  N terminus has KKK and C terminus has KKKK

<220>
<221>  misc_feature
<222>  (1)..(1)
<223>  n is pseudoisocytosine

<220>
<221>  misc_feature
<222>  (6)..(8)
<223>  n is pseudoisocytosine

<220>
<221>  misc_feature
<222>  (8)..(9)
<223>  site of 3 Os (8-amino-2,6-dioxaoctanoic 5 acid linker)

<400>  112
nttttnnncc cttttcaagg tgagtag                                          27

<210>  113
<211>  25
<212>  DNA
<213>  Artificial Sequence

<220>
<223>  Synthetic PNA (ETAGEN Serial Number 100202)

<220>
<221>  misc_feature
<223>  N terminus has KKK and C terminus has KKKK

<220>
<221>  misc_feature
<222>  (2)..(2)
<223>  n is pseudoisocytosine

<220>
<221>  misc_feature
<222>  (4)..(5)
<223>  n is pseudoisocytosine

<220>
<221>  misc_feature
<222>  (8)..(9)
<223>  site of 3 Os (8-amino-2,6-dioxaoctanoic 5 acid linker)

<400>  113
   tntnnttttt tcctctatgg gtaag                                             25

<210>  114
<211>  65
<212>  DNA
<213>  Artificial Sequence

<220>
<223>  Synthetic (ETAGEN Serial Number 100185)

<400>  114
tcttatatct gtactcatca taggaaacac caaagataat gttctccttg atagtacccg      60

gatta                                                                  65


<210>  115
<211>  35
<212>  DNA
<213>  Artificial Sequence

<220>
<223>  Synthetic (ETAGEN Serial Number 100188)


<220>
<221>  misc_feature
<223>  N-term Phosphate

<400>  115
tcatcatagg aaacaccaaa gatgatattt tcttt                                 35


<210>  116
<211>  35
<212>  DNA
<213>  Artificial Sequence

<220>
<223>  Synthetic (ETAGEN Serial Number 100189)


<220>
<221>  misc_feature
<223>  N-term Phosphate

<400>  116
tcatcatagg aaacaccaaa gatgatattt tcttt                                 35


<210>  117
<211>  35
<212>  DNA
<213>  Artificial Sequence

<220>
<223>  Synthetic (ETAGEN Serial Number 100192)


<220>
<221>  misc_feature
<223>  N-term Phosphate

<400>  117
tcatcatagg aaacaccaat aatattttct ttgat                                 35


<210>  118
<211>  61
<212>  DNA

<213>  Artificial Sequence

<220>
<223>  Synthetic (ETAGEN Serial Number 100183)

<400>  118
tcttatatct gtactcatca taggaaacac caaagataat gttctccttg atagtacccg      60

g                                                                      61


<210>  119
<211>  58
<212>  DNA
<213>  Artificial Sequence

<220>
<223>  Synthetic (ETAGEN Serial Number 100184)

<400>  119
tcttatatct gtactcatca taggaaacac caataatatt ttctttgata gtacccgg        58


<210>  120
<211>  91
<212>  DNA
<213>  Artificial Sequence

<220>
<223>  Synthetic (ETAGEN Serial Number 100193)

<400>  120
ctttgatgac gcttctgtat ctatattcat cataggaaac accaaagata atgttctcct      60

taatggtgcc aggcataatc caggaaaact g                                     91


<210>  121
<211>  91
<212>  DNA
<213>  Artificial Sequence

<220>
<223>  Synthetic (ETAGEN Serial Number 100201)

<400>  121
aagctttgac aacactctta tatctgtact catcatagga aacaccaaag ataatgttct      60

ccttgatagt acccggcata atccaagaaa a                                     91


<210>  122
<211>  61
<212>  DNA
<213>  Artificial Sequence

<220>
<223>  Synthetic (ETAGEN Serial Number 100186)

<400>  122
tcttatatct gtactcatca taggaaacac caaagataat gttctccttg atagtacccg      60

g                                                                      61

<210>  123
<211>  35
<212>  DNA
<213>  Artificial Sequence

<220>
<223>  Synthetic (ETAGEN Serial Number 100187)

<400>  123
tcatcatagg aaacaccaaa gatgatattt tcttt                                35


<210>  124
<211>  35
<212>  DNA
<213>  Artificial Sequence

<220>
<223>  Synthetic (ETAGEN Serial Number 100190)


<220>
<221>  misc_feature
<223>  N-term Phosphate

<400>  124
tcatcatagg aaacaccaaa gatgatattt tcttt                                35


<210>  125
<211>  35
<212>  DNA
<213>  Artificial Sequence

<220>
<223>  Synthetic (ETAGEN Serial Number 100191)


<220>
<221>  misc_feature
<223>  N-term Phosphate

<400>  125
tcatcatagg aaacaccaat aatattttct ttgat                                35


<210>  126
<211>  35
<212>  DNA
<213>  Artificial Sequence

<220>
<223>  Synthetic (ETAGEN Serial Number 100242)


<220>
<221>  misc_feature
<223>  N-term Phosphate

<400>  126
tcatcatagg aaacaccaaa gatgatattt tcttt                                35

<210>  127
<211>  35
<212>  DNA
<213>  Artificial Sequence

<220>
<223>  Synthetic (ETAGEN Serial Number 100243)


<220>
<221>  misc_feature
<223>  N-term Phosphate

<400>  127
tcatcatagg aaacaccaaa gatgatattt tcttt                                35


<210>  128
<211>  35
<212>  DNA
<213>  Artificial Sequence

<220>
<223>  Synthetic (ETAGEN Serial Number 100244)


<220>
<221>  misc_feature
<223>  N-term Phosphate

<400>  128
tcatcatagg aaacaccaaa gatgatattt tcttt                                35


<210>  129
<211>  35
<212>  DNA
<213>  Artificial Sequence

<220>
<223>  Synthetic (ETAGEN Serial Number 100245)


<220>
<221>  misc_feature
<223>  N-term Phosphate

<400>  129
tcatcatagg aaacaccaaa gatgatattt tcttt                                35


<210>  130
<211>  35
<212>  DNA
<213>  Artificial Sequence

<220>
<223>  Synthetic (ETAGEN Serial Number 100246)


<220>

<221>  misc_feature
<223>  N-term Phosphate

<400>  130
tcatcatagg aaacaccaaa gatgatattt tcttt                                35


<210>  131
<211>  35
<212>  DNA
<213>  Artificial Sequence

<220>
<223>  Synthetic (ETAGEN Serial Number 100247)

<400>  131
tcatcatagg aaacaccaaa gatgatattt tcttt                                35


<210>  132
<211>  35
<212>  DNA
<213>  Artificial Sequence

<220>
<223>  Synthetic (ETAGEN Serial Number 100248)


<220>
<221>  misc_feature
<223>  N-term Phosphate

<400>  132
tcatcatagg aaacaccaaa gatgatattt tcttt                                35


<210>  133
<211>  35
<212>  DNA
<213>  Artificial Sequence

<220>
<223>  Synthetic (ETAGEN Serial Number 100249)


<220>
<221>  misc_feature
<223>  N-term Phosphate

<400>  133
tcatcatagg aaacaccaaa gatgatattt tcttt                                35


<210>  134
<211>  35
<212>  DNA
<213>  Artificial Sequence

<220>
<223>  Synthetic (ETAGEN Serial Number 100250)


<220>

<221>  misc_feature
<223>  N-term Phosphate

<400>  134
tcatcatagg aaacaccaaa gatgatattt tcttt                                35


<210>  135
<211>  35
<212>  DNA
<213>  Artificial Sequence

<220>
<223>  Synthetic (ETAGEN Serial Number 100251)


<220>
<221>  misc_feature
<223>  N-term Phosphate

<400>  135
tcatcatagg aaacaccaaa gatgatattt tcttt                                35


<210>  136
<211>  35
<212>  DNA
<213>  Artificial Sequence

<220>
<223>  Synthetic (ETAGEN Serial Number 100252)


<220>
<221>  misc_feature
<223>  N-term Phosphate

<400>  136
tcatcatagg aaacaccaaa gatgatattt tcttt                                35


<210>  137
<211>  25
<212>  DNA
<213>  Artificial Sequence

<220>
<223>  Synthetic


<220>
<221>  misc_feature
<223>  N terminus and C terminus has KKK

<220>
<221>  misc_feature
<222>  (1)..(1)
<223>  n is pseudoisocytosine

<220>
<221>  misc_feature
<222>  (3)..(3)
<223>  n is pseudoisocytosine

<220>
<221>  misc_feature
<222>  (6)..(6)
<223>  n is pseudoisocytosine

<220>
<221>  misc_feature
<222>  (9)..(9)
<223>  n is pseudoisocytosine

<220>
<221>  misc_feature
<222>  (10)..(11)
<223>  site of 3 Os (8-amino-2,6-dioxaoctanoic 5 acid linker)

<400>  137
ntnttnttnt tcttcttctc atttc                                          25


<210>  138
<211>  18
<212>  DNA
<213>  Artificial Sequence

<220>
<223>  Synthetic


<220>
<221>  misc_feature
<223>  N terminus has KKKKKK

<220>
<221>  misc_feature
<222>  (1)..(2)
<223>  n is pseudoisocytosine

<220>
<221>  misc_feature
<222>  (4)..(4)
<223>  n is pseudoisocytosine

<220>
<221>  misc_feature
<222>  (7)..(7)
<223>  n is pseudoisocytosine

<220>
<221>  misc_feature
<222>  (9)..(10)
<223>  site of 3 Os (8-amino-2,6-dioxaoctanoic 5 acid linker)

<400>  138
nntnttnttt tcttctcc                                                  18


<210>  139
<211>  28
<212>  DNA
<213>  Artificial Sequence

<220>

<223>  Synthetic

<220>
<221>  misc_feature
<223>  N terminus and C terminus has KKK

<220>
<221>  misc_feature
<222>  (1)..(3)
<223>  n is pseudoisocytosine

<220>
<221>  misc_feature
<222>  (5)..(6)
<223>  n is pseudoisocytosine

<220>
<221>  misc_feature
<222>  (9)..(9)
<223>  n is pseudoisocytosine

<220>
<221>  misc_feature
<222>  (10)..(11)
<223>  site of 3 Os (8-amino-2,6-dioxaoctanoic 5 acid linker)

<400>  139
nnntnnttnt tcttcctccc acagctcc                                          28